# EUROPEAN PATENT APPLICATION

(11) **EP 4 741 406 A1**
(43) Date of publication of application: **13.05.2026**
(21) Application number: 24858403.9
(22) Date of filing: 21.08.2024
(51) Int. Cl.: C07F 9/6561, C07D 471/10, A61K 31/438, A61P 1/08, A61P 11/06, A61P 11/14, A61P 25/06, A61P 25/22, A61P 25/24

(54) **NEUROKININ-1 RECEPTOR ANTAGONIST COMPOUND**

(30) Priority: 28.08.2023 CN 202311086740
(71) Applicant: Creadev (Nanjing) Pharmaceutical Technology Co., Ltd., Nanjing, Jiangsu 210018 (CN)
(72) Inventor: GE, Hui, Nanjing, Jiangsu 210018 (CN); CHEN, Zhengbang, Nanjing, Jiangsu 210018 (CN); WANG, Shan, Nanjing, Jiangsu 210018 (CN); LIU, Chunbo, Nanjing, Jiangsu 210018 (CN)
(74) Representative: Metida
(86) International application number: PCT/CN2024/113639
(87) International publication number: WO 2025/044860

(57) **Abstract**

Disclosed in the present invention is a neurokinin-1 receptor antagonist compound, specifically, a compound having a structure represented by the following formula (II), formula (III) or formula (IV), an isomer, or a pharmaceutically acceptable salt or acid thereof or a deuterated compound thereof. The compound of the present invention has suitable solubility, and can be rapidly converted into an active metabolite rolapitant in blood plasma and liver, such that the problem that rolapitant is difficult to develop into a conventional injection formulation due to poor solubility is solved; in addition, hemolysis can be avoided, and the injection risk can be reduced. Compared with an existing prodrug molecule (a compound of formula (I)), the compound of the present invention is converted in vivo to release the active metabolite rolapitant at a much faster rate, has higher in-vivo exposure of the active metabolite rolapitant, and exhibits superior pharmacokinetic properties.

## Description

### CROSS-REFERENCE TO RELATED APPLICATIONS

This application claims priority to Chinese Patent Application No. 202311086740.7, filed on August 28, 2023.

### BACKGROUND OF THE INVENTION

### Field of the Invention

The present invention relates to the field of medicinal chemistry, and in particular to a neurokinin-1 (NK1 or NK-1) receptor antagonist.

### Description of Related Art

Neurokinins are central neurotransmitters that participate in a variety of physiological activities in the human body. Neurokinins comprise multiple substances, such as substance P, neurokinin A, and neurokinin B. Neurokinin receptors are classified into three types, namely neurokinin receptor 1 (NK1R), neurokinin receptor 2 (NK2R), and neurokinin receptor 3 (NK3R). Among them, NK1R is the most important and the most widely distributed, being present in neurons, the brainstem, vascular endothelium, the gastrointestinal tract, and the urogenital system, and is most abundantly expressed in the vomiting center of the brain. Substance P is the most important neurotransmitter, is widely distributed in the central nervous system and gastrointestinal tract, and has the strongest binding affinity for NK1R. After binding to NK1R, substance P acts on calcium ion channels on the cell membrane via inositol triphosphate, leading to membrane depolarization and changes in protein kinase activity, thereby participating in pain and stress signaling and triggering physiological responses such as vomiting, anxiety, and pain.

Neurokinin receptor antagonists and their uses are disclosed in, for example, US5760018 (1998) (pain, inflammation, migraine, and vomiting), US5620989 (1997) (pain, inflammation, and nociception), WO95/19344 (1995), WO94/13639 (1994), and WO94/10165 (1994). Other classes of NK1 receptor antagonists are also reported in, for example, Wu et al., Tetrahedron 56, 3043-3051 (2000); Rombouts et al., Tetrahedron Letters 42, 7397-7399 (2001); and Rogiers et al., Tetrahedron 57, 8971-8981 (2001).

US7049320 discloses an effective and selective NK1 antagonist having beneficial therapeutic and pharmacological properties and good metabolic stability, namely (5S,8S)-8-(((1R)-1-(3,5-bis(trifluoromethyl)phenyl)ethoxy)methyl)-8-phenyl-1,7-diazaspiro[4.5]decan-2-one (rolapitant). This compound, in the free base form or in the form of a pharmaceutically acceptable salt, is suitable for formulations for parenteral administration.

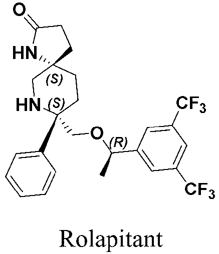

Rolapitant has very poor solubility at physiological pH and is difficult to develop into conventional injectable formulations. To address this issue, researchers have employed cosolvent-based formulations containing Captisol, propylene glycol, and ethanol to significantly improve the solubility of rolapitant; however, such formulations exhibit significant hemolytic effects after intravenous administration. Drug-induced hemolysis refers to the extensive destruction of red blood cells caused by immune factors after a drug enters the human body, and clinically manifests as anemia, jaundice, and dark-colored urine. CN102573475 discloses a series of rolapitant prodrugs and an improved formulation containing polyethylene glycol 15-hydroxystearate and medium-chain triglycerides; however, the hemolytic effect of the pharmaceutical composition has not been completely resolved. WO2020259675A1 discloses a series of rolapitant prodrug compounds. The prodrug compounds of this invention (formula (I)) exhibit significantly improved solubility and are largely converted to rolapitant in vivo, thereby avoiding hemolysis of the pharmaceutical composition and reducing side effects during administration. A prodrug refers to a compound obtained by chemical modification of a drug (parent drug) that is inactive or has low activity in vitro, and that releases the active drug through enzymatic or non-enzymatic conversion in vivo to exert pharmacological effects. The prodrug design of rolapitant is mainly intended to improve its water solubility to facilitate the development of injectable formulations. Such prodrugs are rapidly converted in vivo to release the active metabolite rolapitant, and when the in vivo exposure of the active metabolite rolapitant is high, they are considered to possess desirable pharmacokinetic properties and to be ideal rolapitant prodrugs.

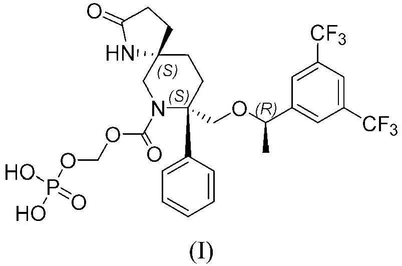

In view of the above, there is significant clinical value in providing additional prodrug designs of rolapitant to identify prodrug molecules that are converted more rapidly in vivo to release the active metabolite rolapitant, achieve higher in vivo exposure of the active metabolite rolapitant, and are safer and more effective.

### BRIEF SUMMARY OF THE INVENTION

One of the objectives of the present invention is to provide a neurokinin-1 (NK1 or NK-1) receptor antagonist, comprising a compound having a structure represented by formula (II), formula (III), or formula (IV), or an isomer thereof, or a pharmaceutically acceptable salt or acid thereof, or a deuterated derivative thereof: 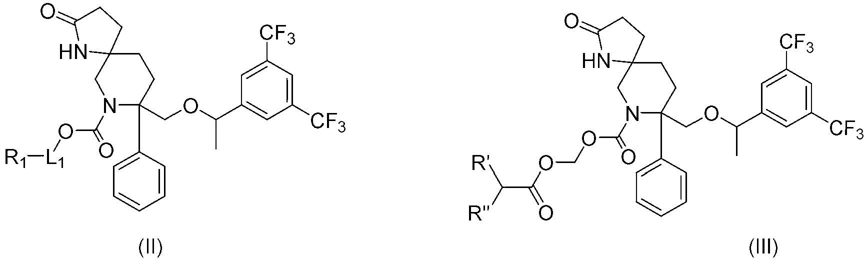 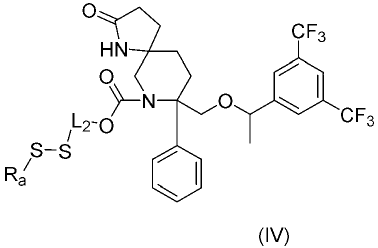 wherein:
in formula (II),
L₁ is selected from -[C(R₄)(R₅)]ₘ-(A)_{q}-, -[C(R₄)(R₅)]ₘ-(A)ₚ-C(O)-[C(R₄)(R₅)]ₙ-(A)_{q}-, -[C(R₄)(R₅)]ₘ-C(O)-(A)ₚ-[C(R₄)(R₅)]ₙ-(A)_{q}-, -[C(R₄)(R₅)]ₘ-(A)ₚ-B-(A)_{q}-, - [C(R₄)(R₅)]ₘ-C(O)-(A)ₚ-B-(A)_{q}-, -[C(R₄)(R₅)]ₘ-(A)ₚ-C(O)-B-(A)_{q}-, -[C(R₄)(R₅)]ₘ-(A)ₚ-C(S)-B-(A)_{q}-, -[C(R₄)(R₅)],C(S)-(A)ₚ-B-(A)_{q}-, -[C(R₄)(R₅)]ₘ-B-(A)ₚ-, - [C(R₄)(R₅)]ₘ-B-C(O)-(A)ₚ-, -[C(R₄)(R₅)]ₘ-B-(A)ₚ-C(O)-(A)_{q}-, -[C(R₄)(R₅)]ₘ-B-(A)ₚ-C(S)-(A)_{q}-, -[C(R₄)(R₅)]ₘ-B-C(S)-(A)ₚ-, -[C(R₄)(R₅)]ₘ- (A)ₚ-S(O)ₜ-B-, - [C(R₄)(R₅)]ₘ-S(O)₁-(A)ₚ-B-, -[C(R₄)(R₅)]ₘ-S(O)=N-[C(R₄)(R₅)]ₙ-, and -[C(R₄)(R₅)]ₘ-N=S(O)-[C(R₄)(R₅)]ₙ-;
A is selected from O, S, and N(R₆);
B is selected from the group consisting of cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, or alkynyl, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of alkyl, haloalkyl, cycloalkyl, alkoxy, hydroxyalkyl, alkenyl, alkynyl, nitro, cyano, hydroxy, SH, and halogen;
R₁ is selected from 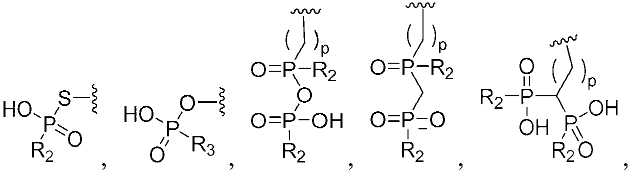 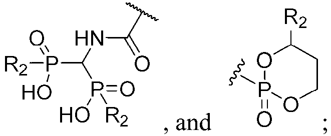
R₂ is selected from the group consisting of hydrogen, hydroxy, SH, alkyl, haloalkyl, alkoxy, -[C(R₄)(R₅)]ₘ-N(R₇)₃⁺, cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, alkynyl, or cyano, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₃ is selected from the group consisting of hydrogen, SH, alkyl, haloalkyl, alkoxy, -[C(R₄)(R₅)]ₘ-N(R₇)₃⁺, cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, or alkynyl, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
when L₁ is -[C(R₄)(R₅)]ₘ-(A)ₚ-B-(A)_{q}-, -[C(R₄)(R₅)]ₘ-S(O)=N-[C(R₄)(R₅)]ₙ-, or - [C(R₄)(R₅)]ₘ-N=S(O)-[C(R₄)(R₅)]ₙ-, R₁ can further be selected from 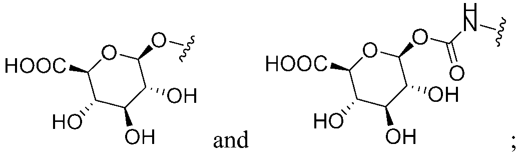
when L₁ is-[C(R₄)(R₅)]ₘ-(A)_{q}-, -[C(R₄)(R₅)]ₘ-S(O)=N-[C(R₄)(R₅)]ₙ-, or - [C(R₄)(R₅)]ₘ-N=S(O)-[C(R₄)(R₅)]ₙ-, R₁ can further be 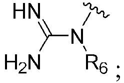
R₄ and R₅ are independently selected from the group consisting of hydrogen, hydroxy, SH, alkyl, haloalkyl, alkoxy, cycloalkyl, heterocyclyl, aryl, heteroaryl, alkenyl, alkynyl, cyano, or halogen, wherein the cycloalkyl, heterocyclyl, aryl, and heteroaryl are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₆ is selected from the group consisting of hydrogen, C₁-C₆ alkyl, haloalkyl, C₁-C₈ cycloalkyl, C₁-C₈ heterocyclyl, aryl, and heteroaryl, wherein the cycloalkyl, heterocyclyl, aryl, and heteroaryl are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₇ is selected from the group consisting of C₁-C₆ alkyl, haloalkyl, alkenyl, alkynyl, and cyano;
m is an integer from 0 to 6; n is an integer from 0 to 6; p is 0, 1, or 2; q is 0, 1, or 2; t is 1 or 2;
in formula (III),
R' is selected from the group consisting of H, 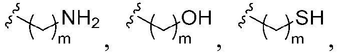 alkoxy, alkenyl, alkynyl, nitro, cyano, halogen, acetyl, sulfonyl, 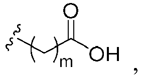 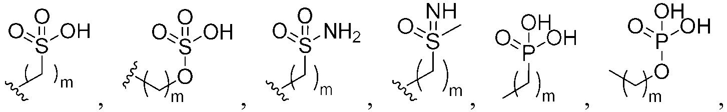 C₁-C₆ alkyl, haloalkyl, C₁-C₈ cycloalkyl, C₁-C₈ heterocyclyl, aryl, heteroaryl and 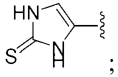
R" is selected from the group consisting of NH₂ and groups selected from the substituted or unsubstituted following: 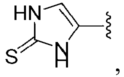 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, -[C(R₄)(R₅)]m-N(R'")₃⁺, C₂-C₇ cyclic quaternary ammonium salts (e.g., 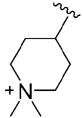 ), 5-12 membered heterospirocyclic quaternary ammonium salts containing N, O, or S (e.g., 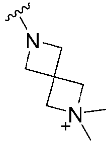 ), and 5-12 membered heterobridged quaternary ammonium salts containing N, O, or S (e.g., 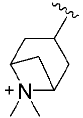 ), wherein substituents are selected from the group consisting of H, OH, SH, NH₂, C₁-C₆ alkyl, haloalkyl, alkenyl, alkynyl, nitro, cyano, and halogen; R₄ and R₅ are as previously defined;
m is an integer from 0 to 6;
or R' and R" together with the carbon atom to which they are attached form a substituted or unsubstituted group selected from the following: 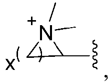 groups containing a 5-12 membered spirocyclic, 5-12 membered heterobridged cyclic, or 6-12 membered fused cyclic system containing N, O, or S, wherein substituents are selected from the group consisting of H, OH, SH, NH₂, C₁-C₆ alkyl, haloalkyl, alkenyl, alkynyl, nitro, cyano, and halogen; x is an integer from 1 to 6;
R‴ is selected from the group consisting of C₁-C₆ alkyl, haloalkyl, alkenyl, alkynyl, and cyano;
in formula (IV),
L₂ is selected from the group consisting of -[C(R_{b})(R_{c})]ₓ-, -[C(R_{b})(R_{c})]ₓ-Z-[C(R_{d})(Rₑ)]ₕ-, -[C(R_{b})(R_{c})]ₓ-A-C(O)-[C(R_{d})(Rₑ)]ₕ-, -[C(R_{b})(R_{c})]ₓ-A-C(S)-[C(R_{d})(Rₑ)]ₕ-, -[C(R_{b})(R_{c})]ₓ-A-S(O)₂-[C(R_{d})(Rₑ)]ₕ-, -[C(R_{b})(R_{c})]ₓ-C(O)-A-[C(R_{d})(Rₑ)]ₕ -, -[C(R_{b})(R_{c})]ₓ-C(S)-A-[C(R_{d})(Rₑ)]ₕ-, -[C(Rₚ)(Rₑ)]ₓ-S(O)₂-A-[C(R_{d})(Rₑ)]ₕ-, -[C(R_{b})(R_{c})]ₓ-ethenyl-[C(R_{d})(Rₑ)]ₕ-, -[C(R_{b})(R_{c})]ₓ-ethynyl-[C(R_{d})(Rₑ)]ₕ-, -[C(R_{b})(R_{c})]ₓ-S(O)(NRₖ)- [C(R_{d})(Rₑ)]ₕ-, -[C(R_{b})(Rₑ)]ₓ-S(O)=N-[C(R_{d})(Rₑ)]ₕ-, and -[C(R_{b})(Rₑ)]ₓ -S(O)ₜ-[C(R_{d})(Rₑ)]ₕ-;
Rₐ is selected from the group consisting of -[C(R_{f})(R_{g})]ₙ-Rⱼ, 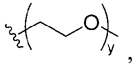 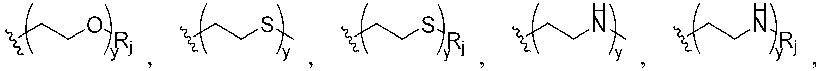 -Y-[C(R_{f})(R_{g})]ₙ-Rⱼ, -[C(Rr)(R_{g})]ₙ-N(Rₚ)₂⁺- [C(Rₕ)(Rᵢ)]ᵢ-Rⱼ, -[C(R_{f})(R_{g})]ₙ-A-C(O)-[C(Rₕ)(Rᵢ)]ᵢ-Rⱼ, -[C(R_{f})(R_{g})]ₙ-A-C(S)-[C(Rₕ)(Rᵢ)]ᵢ-Rⱼ, -[C(R_{f})(R_{g})]ₙ-ethenyl-[C(Rₕ)(Rᵢ)]ᵢ-Rⱼ, -[C(R_{f})(R_{g})]ₙ-ethynyl-[C(Rₕ)(Rᵢ)]ᵢ-Rⱼ, -[C(R_{f})(R_{g})]ₙ-S(O)(NRₖ)-[C(Rₕ)(Rᵢ)]ᵢ-Rⱼ, -[C(R_{f})(R_{g})]ₙ-S(O)=N-[C(Rₕ)(Rᵢ)]ᵢ-Rⱼ, -[C(R_{f})(R_{g})]ₙ-S(O)ₜ-[C(Rₕ)(Rᵢ)]ᵢ-Rⱼ, -[C(R_{f})(R_{g})]ₙ-C(O)-A-[C(Rₕ)(Rᵢ)]ᵢ -Rⱼ, -[C(R_{f})(R_{g})]ₙ-C(S)-A-[C(Rₕ)(Rᵢ)]ᵢ-Rⱼ, -[C(R_{f})(R_{g})]ₙ-ethenyl-[C(Rₕ)(Rᵢ)]ᵢ -Rⱼ, -[C(R_{f})(R_{g})]ₙ-ethynyl-[C(Rₕ)(Rᵢ)]ᵢ-Rⱼ, -[C(R_{f})(R_{g})]ₙ-S(O)(NRₖ)- [C(Rₕ)(Rᵢ)]ᵢ-Rⱼ, [C(R_{f})(R_{g})]ₙ-N=S(O)-[C(Rₕ)(Rᵢ)]ᵢ -Rⱼ, -[C(R_{f})(R_{g})]ₙ -S(O)ₜ -[C(Rₕ)(Rᵢ)]ᵢ -Rⱼ, and -[C(R_{f})(R_{g})]ₙ -N(Rₚ)₂⁺-[C(Rₕ)(Rᵢ)]ᵢ -Rⱼ;
R_{b}, R_{c}, R_{d}, Rₑ, R_{f}, R_{g}, Rₕ and Rᵢ are each independently selected from the group consisting of hydrogen, alkyl, haloalkyl, alkoxy, hydroxy, cycloalkyl, heterocyclyl, aryl, heteroaryl, alkenyl, alkynyl, cyano, or halogen, wherein the cycloalkyl, heterocyclyl, aryl, and heteroaryl are optionally substituted with one or more substituents selected from the group consisting of alkyl, haloalkyl, cycloalkyl, alkoxy, hydroxyalkyl, alkenyl, alkynyl, nitro, cyano, halogen, acetyl, sulfonyl, 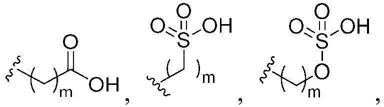 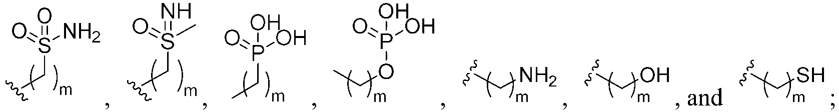
or R_{b} and R_{c}, R_{d} and Rₑ, R_{f} and R_{g}, Rₕ and Rᵢ together with the carbon atom(s) to which they are attached, respectively form a substituted or unsubstituted group selected from the following: cycloalkyl, heterocycloalkyl, 5-12 membered spirocyclic groups containing N, O, or S, 5-12 membered heterobridged cyclic groups containing N, O, or S, and 6-12 membered fused cyclic groups containing N, O, or S, wherein substituents are selected from the group consisting of H, OH, SH, NH₂, C₁-C₆ alkyl, haloalkyl, alkenyl, alkynyl, nitro, cyano, and halogen;
Rⱼ is selected from the group consisting of H, NH₂, OH, SH, alkoxy, hydroxyalkyl, haloalkyl, alkenyl, alkynyl, nitro, cyano, halogen, C₁-C₆ alkyl, C₁-C₈ cycloalkyl, C₁-C₈ heterocyclyl, aryl, heteroaryl, 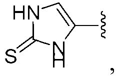 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, -N(Rₚ)₃⁺, C₂-C₇ cyclic quaternary ammonium salts (e.g., 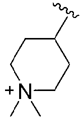 ), 5-12 membered heterospirocyclic quaternary ammonium salts containing N, O, or S (e.g., 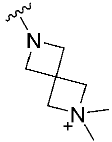 ), and 5-12 membered heterobridged quaternary ammonium salts containing N, O, or S (e.g., 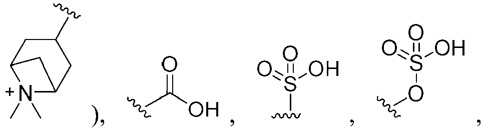 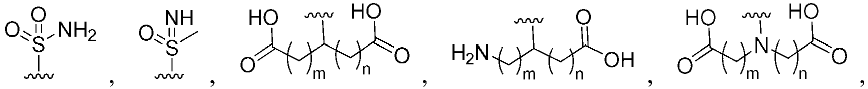 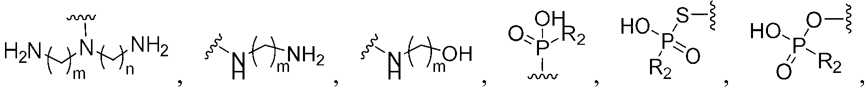 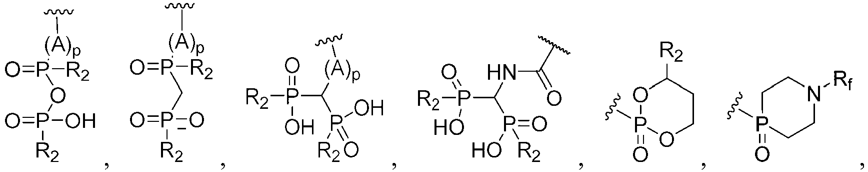 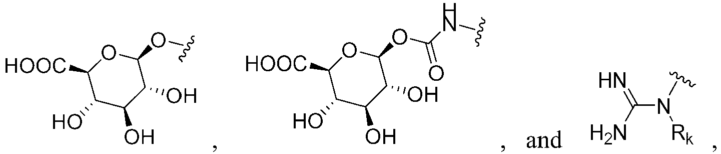 wherein the cycloalkyl, heterocyclyl, aryl, and heteroaryl are optionally substituted with one or more substituents selected from the group consisting of alkyl, cycloalkyl, alkoxy, hydroxyalkyl, alkenyl, alkynyl, nitro, cyano, hydroxy, amino, halogen, acetyl, sulfonyl, 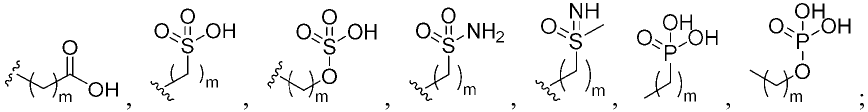 R₂ is as previously defined;
Rₖ is selected from the group consisting of hydrogen, C₁-C₆ alkyl, haloalkyl, C₁-C₈ cycloalkyl, C₁-C₈ heterocyclyl, aryl, and heteroaryl, wherein the C₁-C₈ cycloalkyl, C₁-C₈ heterocyclyl, aryl, and heteroaryl are optionally substituted with one or more substituents selected from the group consisting of alkyl, haloalkyl, cycloalkyl, alkoxy, hydroxyalkyl, alkenyl, alkynyl, nitro, cyano, hydroxy, amino, SH, and halogen;
Rₚ is selected from the group consisting of C₁-C₆ alkyl, haloalkyl, alkenyl, alkynyl, and cyano;
A is selected from O, S, or N(Rₖ);
Y is selected from substituted or unsubstituted C₁-C₈ cycloalkyl, C₁-C₈ heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S, wherein substituents are selected from H, NH₂, OH, SH, C₁-C₆ alkyl, haloalkyl, alkenyl, alkynyl, nitro, cyano, and halogen;
Z is selected from cycloalkyl, heterocyclyl, aryl, and heteroaryl, wherein the cycloalkyl, heterocyclyl, aryl, and heteroaryl are optionally substituted with one substituent selected from H, NH₂, OH, SH, alkyl, haloalkyl, cycloalkyl, alkoxy, hydroxyalkyl, alkenyl, alkynyl, nitro, cyano, and halogen;
h is an integer from 0 to 10; i is an integer from 0 to 10; m is an integer from 0 to 6; n is an integer from 0 to 6; x is an integer from 1 to 6; y is an integer from 0 to 10.

In some aspects, in formula (II) of the present invention:
In some embodiments, L₁ is selected from -[C(R₄)(R₅)]ₘ-(A)_{q}- or -[C(R₄)(R₅)]ₘ-(A)ₚ-C(O)-[C(R₄)(R₅)]ₙ-(A)_{q}-; A is selected from O or S;
R₁ is selected from 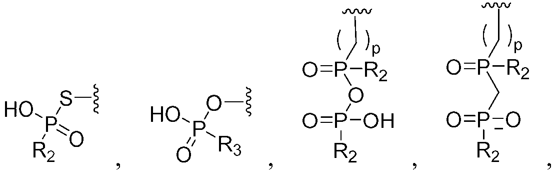 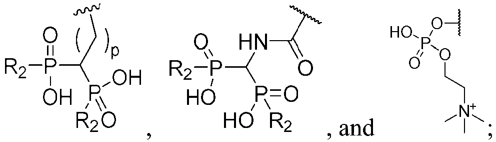
R₂ is selected from hydrogen, hydroxy, SH, alkyl, C₁-C₆ alkyl, and C₁-C₆ alkoxy;
R₃ is selected from hydrogen, SH, alkyl, C₁-C₆ alkyl, and C₁-C₆ alkoxy;
m is an integer from 0 to 6; n is an integer from 0 to 6; p is 0, 1, or 2; q is 0, 1, or 2; t is 1 or 2.

In some more specific examples, R₁ is selected from 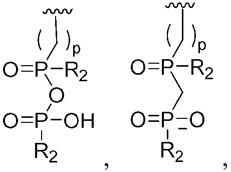 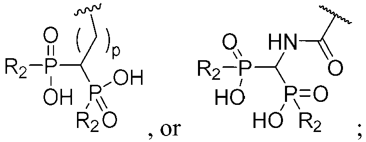 R₂ is selected from hydrogen, hydroxy, SH, alkyl, C₁-C₆ alkyl, and C₁-C₆ alkoxy; p is 0, 1, or 2.

In some aspects, in formula (III) of the present invention:
R' is selected from H, C₁-C₆ alkyl, 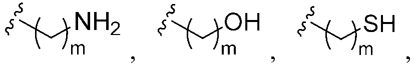 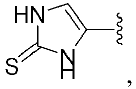 C₁-C₆ alkoxy, nitro, cyano, or halogen;
R" is selected from groups substituted or unsubstituted as follows: 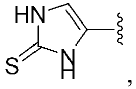 5-12 membered heterospirocyclic groups containing N, O, or S, -[C(R₄)(R₅)]ₘ-N(R‴)₃⁺; the substituents are selected from H, OH, SH, NH₂, C₁-C₄ alkyl, C₁-C₄ haloalkyl, nitro, cyano, or halogen; m is 0, 1, 2, 3, 4, 5, or 6; R₄ and R₅ are independently selected from hydrogen, hydroxy, SH, alkyl, or C₁-C₄ alkyl;
alternatively, R' and R" together with the carbon atom to which they are attached form a substituted or unsubstituted group selected from 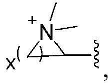 5-12 membered spirocyclic groups having N, O, or S, 5-12 membered heterobridged cyclic groups containing N, O, or S, or 6-12 membered fused cyclic groups containing N, O, or S; the substituents are selected from H, OH, SH, NH₂, C₁-C₄ alkyl, C₁-C₄ haloalkyl, nitro, cyano, or halogen; x is 1, 2, 3, 4, 5, or 6;
R‴ is selected from C₁-C₆ alkyl, C₁-C₆ haloalkyl, alkenyl, alkynyl, or cyano.

In some more specific examples,
R' is selected from H, C₁-C₆ alkyl, 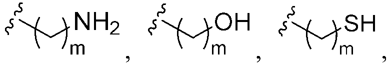 C₁-C₄ alkoxy, nitro, cyano, or halogen;
R" is selected from NH₂ and groups substituted or unsubstituted as follows: - [C(R₄)(R₅)]ₘ-N(R‴)₃⁺; the substituents are selected from H, OH, SH, NH₂, C₁-C₄ alkyl, C₁-C₄ haloalkyl, nitro, cyano, or halogen; m is 0, 1, 2, 3, 4, 5, or 6; R₄ and R₅ are independently selected from hydrogen, hydroxy, SH, alkyl, or C₁-C₄ alkyl;
R‴ is selected from C₁-C₄ alkyl, C₁-C₄ haloalkyl, alkenyl, alkynyl, or cyano; preferably methyl, ethyl, propyl, or isopropyl.

In some aspects, in formula (IV),

In some specific examples, L₂ is selected from-[C(R_{b})(R_{c})]ₓ-, -[C(R_{b})(R_{c})]ₓ-Z-, - [C(R_{b})(R_{c})]ₓ-O-C(O)-[C(R_{d})(Rₑ)]ₕ-, -[C(R_{b})(R_{c})]ₓ-S-C(O)-[C(R_{d})(Rₑ)]ₕ -, -[C(R_{b})(R_{c})] x - O -C(O)-[C(R_{d})(Rₑ)]ₕ -, -[C(R_{b})(R_{c})] ₓ - O -C(S)-[C(R_{d})(Rₑ)] ₕ-; R_{b} is H or methyl; R_{c} is H or methyl; Z is a C₅-C₁₀ aryl or a 5-6 membered heteroaryl, preferably phenyl, naphthyl, or thiophenyl; R_{d} is methyl or ethyl; Rₑ is H, methyl, or ethyl; x is 1, 2, 3, 4, 5, or 6; h is an integer from 0 to 10;

In some more specific examples, L₂ is selected from -CH₂-, -CH(CH₃)- or - C(CH₃)₂-; more preferably, L₂ is -CH₂-;

In other examples, L₂ is selected from -CH₂-O-C(O)-(CH₂)ₕ-, -CH₂-S-C(O)-(CH₂)ₕ-, -CH₂-O-C(O)-[C(R_{d})(Rₑ)] ₕ-, -CH₂-O-C(S)-[C(R₄)(Rₑ)]ₕ-; R_{d} is methyl; Rₑ is H or methyl; h is 0 or an integer up to 10; more preferably, h is an integer from 0 to 6; most preferably, h is 0, 1, 2, 3, or 4.

In some examples, Rₐ is selected from substituted or unsubstituted groups as follows: C₁-C₁₀ alkyl, 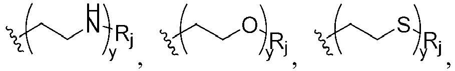 -(CH₂)ₕCHRₓR_{y}, - (CH₂) ₕNRₘRₙ, -(CH₂)ₕN(Rₚ)₂⁺-Rⱼ; the substituents are selected from NH₂, -OH, - COOH, 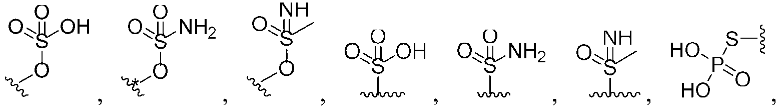 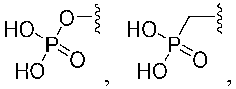 C₁-C₄ alkyl, C₁-C₄ alkoxy, or C₁-C₄ carboxyalkyl;
h or y is an integer from 0 to 10;
Rₓ is selected from H, -COOH, or C₁-C₄ alkyl;
R_{y} is selected from H, amino, or C₁-C₄ alkyl;
Rₘ and Rₙ are independently selected from H, amino, -COOH, or C₁-C₄ alkyl; or Rₘ and Rₙ together with the nitrogen atom to which they are attached form a 5-11 membered heterocyclic group containing 1-3 heteroatoms selected from N, O, or S;
Rⱼ is selected from H or C₁-C₆ alkyl;
Rₚ is selected from H or C₁-C₆ alkyl;

In some more specific examples, Rₐ is selected from substituted or unsubstituted groups as follows: C₁-C₆ alkyl, 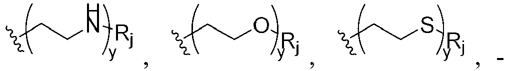 (CH₂)ₕCHRₓR_{y}, -(CH₂)ₕNRₘRₙ, -(CH₂)ₕN(Rₚ)₂⁺-Rⱼ; the substituents are selected from -NH₂, -OH, -COOH, 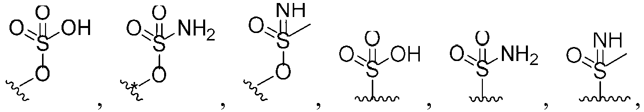 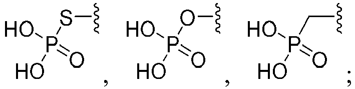
y is 1, 2, 3, 4, 5, or 6;
h is 0, 1, 2, 3, or 4;
Rₓ is selected from H, -COOH, methyl, ethyl, propyl, or isopropyl;
R_{y} is selected from H, amino, methyl, ethyl, propyl, or isopropyl;
Rₘ and Rₙ are independently selected from H, amino, -COOH, methyl, ethyl, propyl, or isopropyl; or Rm and Rn together with the nitrogen atom to which they are attached form a 5-11 membered heterocyclic group containing 1-3 heteroatoms selected from N, O, or S;
Rⱼ is selected from H, methyl, ethyl, propyl, or isopropyl;
Rₚ is selected from H, methyl, ethyl, propyl, or isopropyl.

In some specific examples, the present invention provides a compound having the following structure, or an isomer thereof, or a pharmaceutically acceptable salt or acid thereof, or a deuterated derivative thereof:

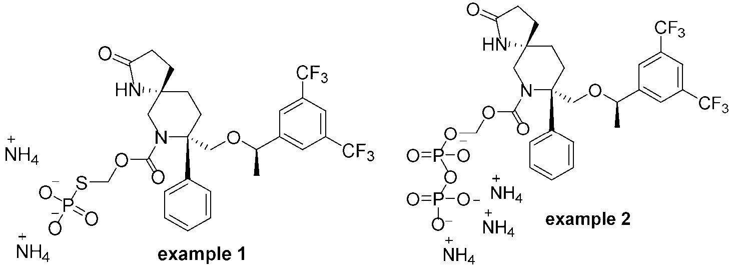

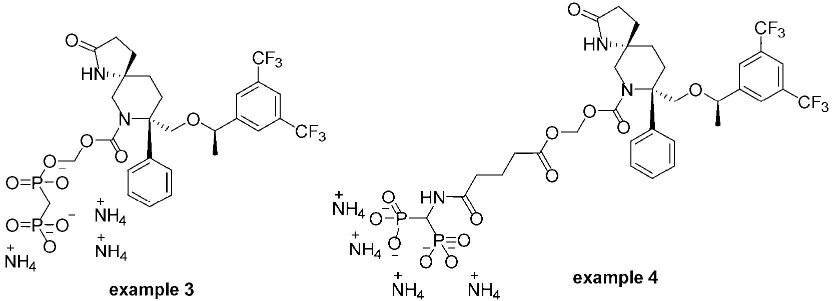

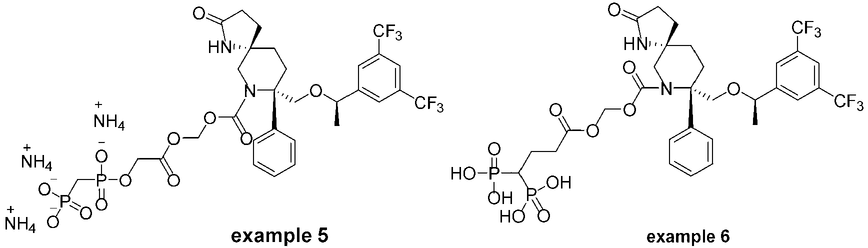

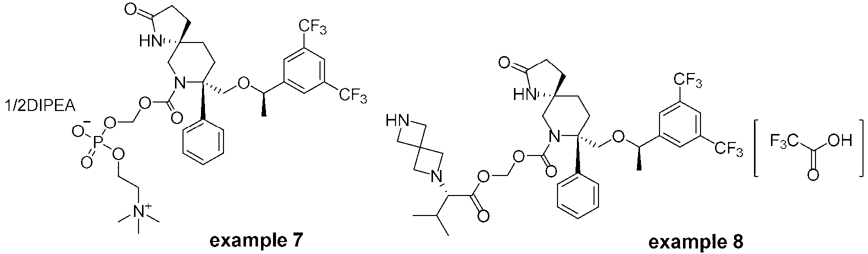

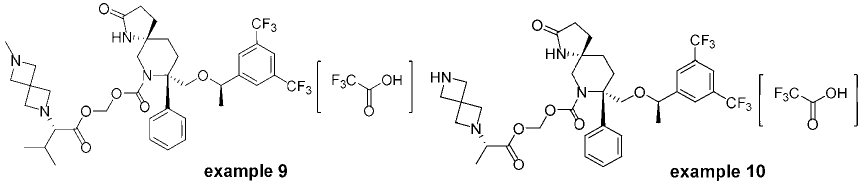

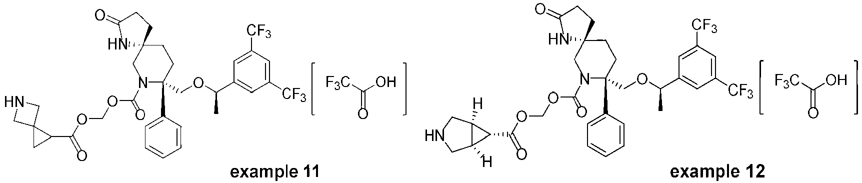

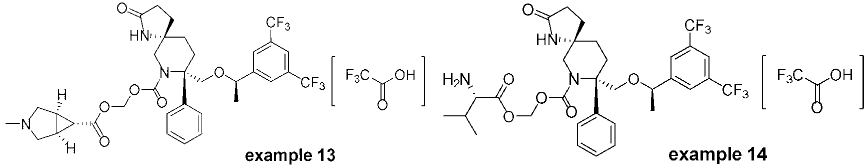

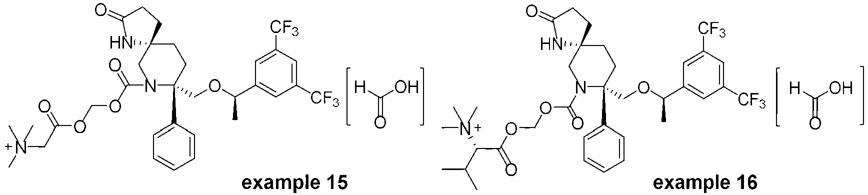

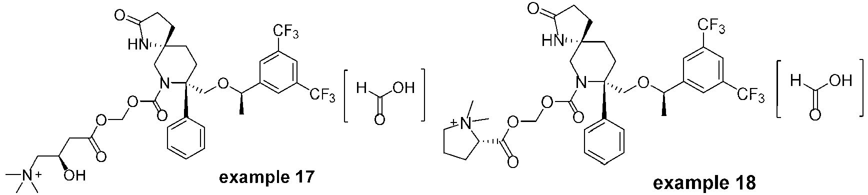

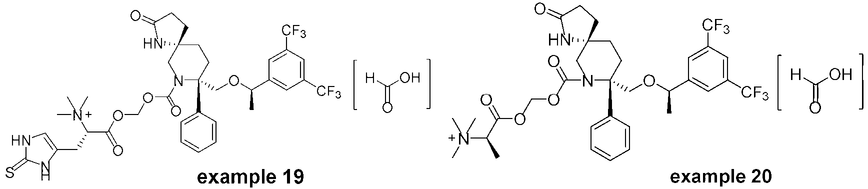

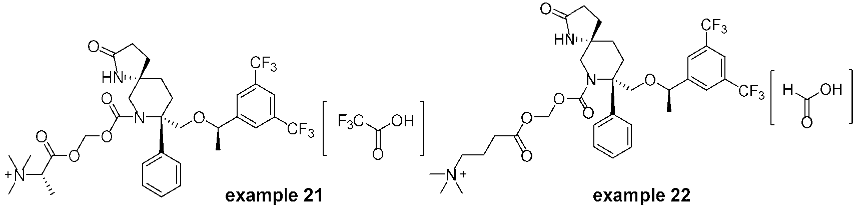

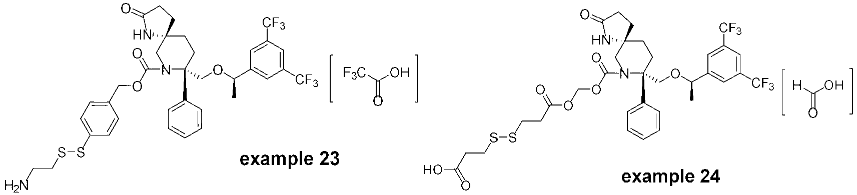

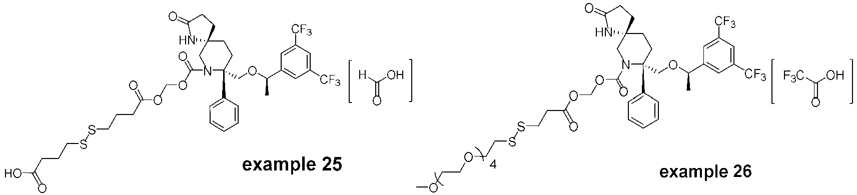

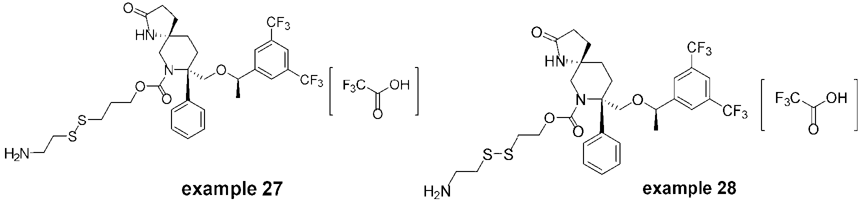

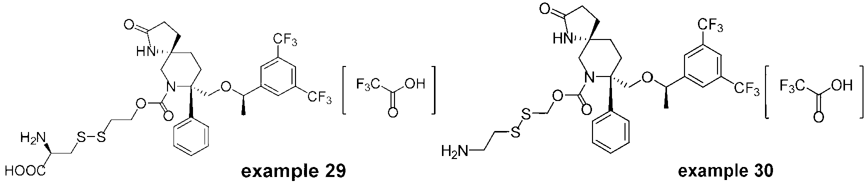

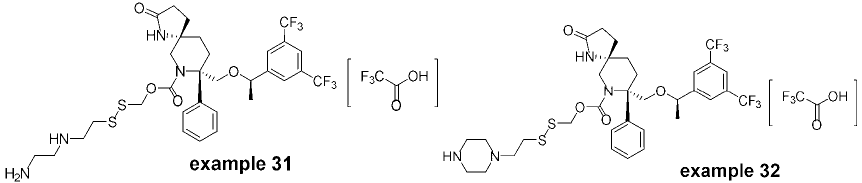

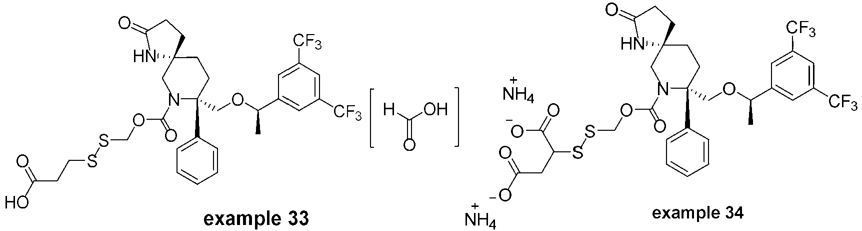

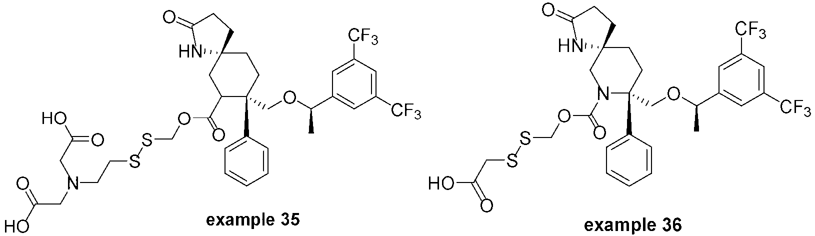

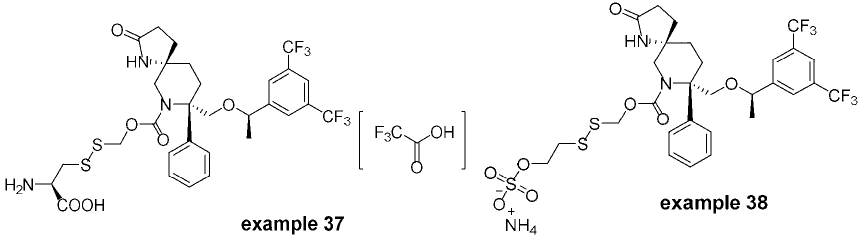

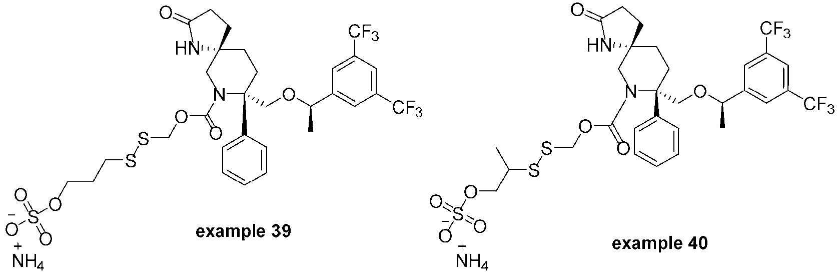

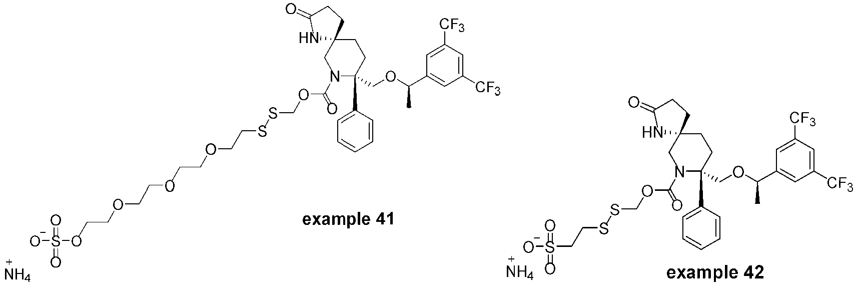

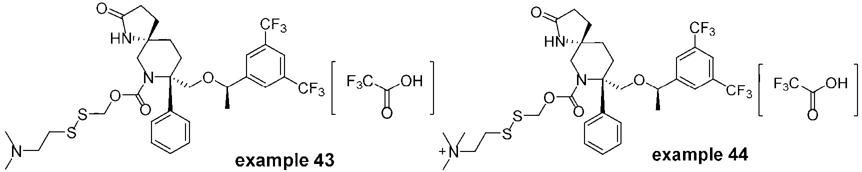

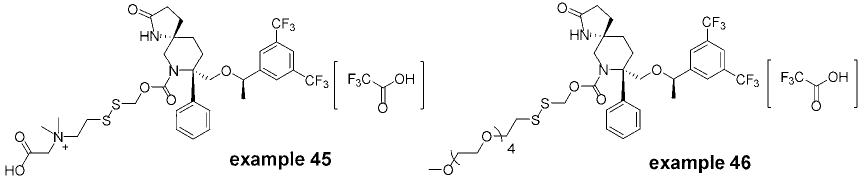

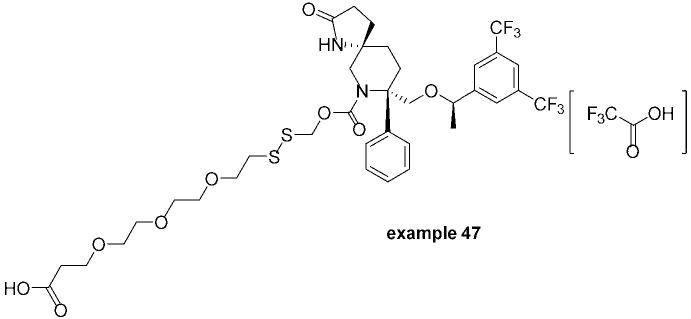

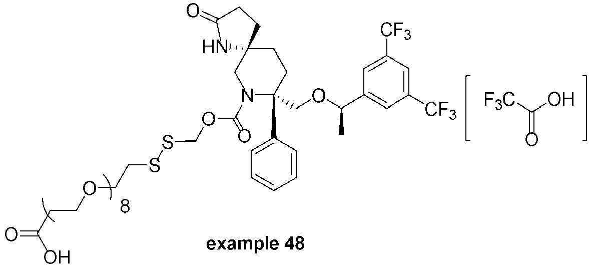

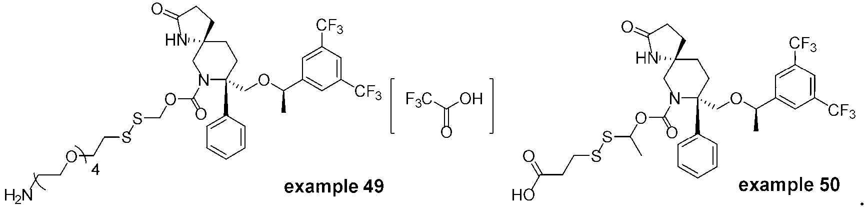

The present invention also provides a composition comprising the compound of the present invention and a pharmaceutically acceptable carrier.

The present invention further provides the use of the aforementioned compound in the preparation of a medicament for treating vomiting, nausea, asthma, anxiety, depression, cough, or migraine.

Unless otherwise specified, the terms used in the present invention are defined as follows:
"Spiro ring": a system in which two rings in a molecule share a single carbon atom.
"Bridged ring" refers to a polycyclic hydrocarbon in which two or more carbon atoms (bridgehead carbons) are shared, classified as bicyclic, tricyclic, tetracyclic, etc., depending on the number of rings.
"8-16-membered ring, saturated or unsaturated" refers to a ring composed of 8 to 16 atoms. An 8-16-membered ring containing unsaturation refers to a ring that contains at least one carbon-carbon double bond, carbon-carbon triple bond, or both.
"Alkyl" denotes a saturated hydrocarbon group containing 1-20 carbon atoms, which can be linear or branched. (The numerical ranges mentioned herein, e.g., "1-20", refer to the number of carbon atoms in the alkyl group, which can be 1, 2, 3, ... up to 20.) More preferably, the alkyl group contains 1-10 carbon atoms, e.g., methyl, ethyl, propyl, isopropyl, n-butyl, isobutyl, sec-butyl, tert-butyl, pentyl, etc. A C₁-C₈ alkyl refers to a linear or branched alkyl containing 1-8 carbon atoms, e.g., methyl, ethyl, propyl, isopropyl, n-butyl, isobutyl, sec-butyl, tert-butyl, pentyl, neopentyl, hexyl, heptyl, octyl. Alkyl groups containing 1-4 carbon atoms are referred to as lower alkyls. When a lower alkyl is unsubstituted, it is referred to as an unsubstituted lower alkyl. Preferably, the alkyl group is a lower alkyl containing 1-4 carbon atoms, such as methyl, ethyl, propyl, isopropyl, n-butyl, isobutyl, sec-butyl, or tert-butyl. Alkyl groups can be substituted or unsubstituted, and the substituents can be multiple.
"Haloalkyl" refers to an alkyl substituted with one or more identical or different halogen atoms, preferably a halogen-substituted lower alkyl as defined above, e.g., - CH₂Cl, -CF₃, -CH₂CF₃, -CH₂CCl₃.
"Alkoxy" denotes -O-(unsubstituted alkyl) or -O-(unsubstituted cycloalkyl). Representative examples include, but are not limited to, methoxy, ethoxy, propoxy, butoxy, cyclopropoxy, cyclobutoxy, cyclopentoxy, cyclohexoxy.
"C₁-C₆ alkoxy" refers to a linear or branched alkoxy group containing 1-6 carbon atoms, e.g., methoxy, ethoxy, propoxy, isopropoxy, butoxy, isobutoxy, tert-butoxy, sec-butoxy, pentoxy, neopentoxy, hexyloxy. Preferably, the alkoxy group contains 1-4 carbon atoms, especially methoxy or ethoxy.
"Cycloalkyl" refers to a group consisting entirely of carbon, which can be a single ring or fused rings ("fused" meaning that each ring shares an adjacent pair of carbon atoms with another ring in the system), in which one or more rings do not possess a fully conjugated π-electron system. Examples of cycloalkyl groups include, but are not limited to, cyclopropyl, cyclobutyl, cyclopentyl, cyclopentenyl, cyclohexyl, adamantyl, cyclohexadienyl, cycloheptyl, cycloheptatrienyl.
"Heterocycle" refers to a saturated ring group containing 3-8 ring atoms, in which one or two ring atoms are heteroatoms selected from N, O, or S(O)m (where m is an integer from 0 to 2), and the remaining ring atoms are carbon, with one or two carbon atoms optionally replaced by carbonyl. The rings of a heterocyclic group can optionally be independently substituted with one, two, or three substituents, selected from lower alkyl (optionally substituted with one or two substituents independently selected from carboxyl or ester), haloalkyl, cyanoalkyl, halogen, nitro, cyano, hydroxy, alkoxy, amino, monoalkylamino, dialkylamino, aralkyl, heteroaralkyl, and -COR (where R is alkyl). More specifically, heterocyclic groups include, but are not limited to, tetrahydropyranyl, 2,2-dimethyl-1,3-dioxolanyl, piperidinyl, N-methylpiperidin-3-yl, piperazinyl, N-methylpyrrolidin-3-yl, pyrrolidinyl, morpholinyl, thiamorpholinyl, thiamorpholinyl-1-oxide, thiamorpholinyl-1,1-dioxide, 4-ethoxycarbonylpiperazinyl, 3-oxopiperazinyl, 2-imidazolinonyl, 2-pyrrolidinonyl, 2-oxopiperazinyl, tetrahydropyrimidin-2-onyl and their derivatives. Preferably, the heterocyclic group is optionally substituted with one or two substituents, which are independently selected from halogen, lower alkyl, lower alkyl substituted with carboxyl or ester, hydroxy, and mono- or dialkylamino.

The term "heterocyclic group" refers to a 3-12-membered aromatic or nonaromatic heterocycle containing 1-4 heteroatoms selected from O, N, and S, and includes bicyclic groups. The term "heterocyclic group" therefore encompasses the aforementioned heteroaryl groups, as well as their dihydro and tetrahydro analogs. Further examples of heterocyclic groups include, but are not limited to, benzimidazolyl, benzofuranyl, benzopyranyl, benzopyrazolyl, benzotriazolyl, benzothienyl, benzooxazolyl, carbazolyl, carbolinyl, thiazolinyl, furanyl, imidazolyl, dihydroindolyl, indolyl, indazolyl, isobenzofuranyl, isoindolyl, isoquinolinyl, isothiazolyl, isoxazolyl, oxadiazolyl, oxazolyl, oxazoline, isoxazoline, oxetanyl, pyranyl, pyrazinyl, pyrazolyl, diazinyl, pyridopyridinyl, diazinyl, pyridyl, pyrimidinyl, pyrrolidinyl, quinolinyl, quinazolinyl, tetrahydropyranyl, tetrazolyl, tetrazolopyridinyl, thiazolidinyl, thiazolyl, thiophenyl, triazolyl, azetidinyl, 1,4-dioxanyl, hexahydroazepinyl, piperazinyl, piperidinyl, pyridin-2-onyl, pyrrolidinyl, morpholinyl, thiomorpholinyl, dihydrobenzimidazolyl, dihydrobenzofuranyl, dihydrobenzothienyl, dihydrobenzooxazolyl, dihydrofuranyl, dihydroimidazolyl, dihydroindolyl, dihydroisoxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolidinyl, dihydroquinolinyl, dihydrotetrazolyl, dihydrothiazolidinyl, dihydrothiazolyl, dihydrothiophenyl, dihydrotriazolyl, dihydroazetidinyl, methylenedioxybenzoyl, tetrahydrofuranyl, and tetrahydrothiophenyl, as well as their N-oxides. The heterocyclic substituent can be attached via a carbon atom or via a heteroatom.

"Aryl" refers to a fully carbon-containing monocyclic or fused polycyclic group with a fully conjugated π-electron system, containing 1-12 carbon atoms. Non-limiting examples of aryl include phenyl, naphthyl, and anthryl. An aryl group can be substituted or unsubstituted. When substituted, the substituents are preferably one or more, more preferably one, two, or three, and even more preferably one or two, independently selected from lower alkyl, trihaloalkyl, halogen, hydroxy, lower alkoxy, mercapto, (lower alkyl)thio, cyano, acyl, thioacyl, O-carbamoyl, N-carbamoyl, O-thiocarbamoyl, N-thiocarbamoyl, C-amido, N-amido, nitro, N-sulfonamido, S-sulfonamido, R¹⁰S(O)-, R¹⁰S(O)₂-, -C(O)OR¹⁰, R¹⁰C(O)O-, and -NR¹⁰R¹¹, wherein R¹⁰ and R¹¹ are as defined above. Preferably, the aryl group is optionally substituted with one or two substituents, independently selected from halogen, lower alkyl, trihaloalkyl, hydroxy, mercapto, cyano, N-amido, mono- or dialkylamino, carboxyl, or N-sulfonamido.

"Heteroaryl" refers to a stable monocyclic or bicyclic carbon-containing ring system with up to 3-10 atoms per ring, in which at least one ring is aromatic and contains 1-4 heteroatoms selected from O, N, and S. Non-limiting examples of unsubstituted heteroaryl include pyrrolyl, furanyl, thiophenyl, imidazolyl, oxazolyl, thiazolyl, pyrazolyl, pyrimidinyl, quinolinyl, isoquinolinyl, purinyl, acridinyl, carbazolyl, thiazolinyl, quinoxalinyl, indolyl, benzotriazolyl, benzothienyl, benzofuranyl, isoxazolyl, indolyl, pyrazinyl, diazinyl, pyridyl, tetrazolyl, triazinyl, and carbazolyl. Heteroaryl groups can be substituted or unsubstituted. When substituted, the substituents are preferably one or more, more preferably one, two, or three, and even more preferably one or two, independently selected from lower alkyl, trihaloalkyl, halogen, hydroxy, lower alkoxy, mercapto, (lower alkyl)thio, cyano, acyl, thioacyl, O-carbamoyl, N-carbamoyl, O-thiocarbamoyl, N-thiocarbamoyl, C-amido, N-amido, nitro, N-sulfonamido, S-sulfonamido, R¹⁰S(O)-, R¹⁰S(O)₂-, -C(O)OR¹⁰, R¹⁰C(O)O-, and -NR¹⁰R¹¹, wherein R¹⁰ and R¹¹ are as defined above. Preferably, the heteroaryl group is optionally substituted with one or two substituents, independently selected from halogen, lower alkyl, trihaloalkyl, hydroxy, mercapto, cyano, N-amido, mono- or dialkylamino, carboxyl, or N-sulfonamido.

"Acyl" refers to a -C(O)-R' group, wherein R' is selected from the group consisting of hydrogen, unsubstituted lower alkyl, trifluoromethyl, unsubstituted cycloalkyl, optionally substituted aryl with one or more, preferably 1, 2, or 3 substituents independently selected from unsubstituted lower alkyl, trifluoromethyl, unsubstituted lower alkoxy, halogen, and -NR10R11, wherein R10 and R11 are as defined above, optionally substituted heteroaryl with one or more, preferably 1, 2, or 3 substituents independently selected from unsubstituted lower alkyl, trifluoromethyl, unsubstituted lower alkoxy, halogen, and -NR10R11 (bonded via a ring carbon atom), and optionally substituted heterocyclic aliphatic group with one or more, preferably 1, 2, or 3 substituents independently selected from unsubstituted lower alkyl, trifluoromethyl, unsubstituted lower alkoxy, halogen, and -NR10R11 (bonded via a ring carbon atom). Representative acyl groups include, but are not limited to, acetyl, trifluoroacetyl, benzoyl.

"Sulfonyl" refers to a -S(O)₂-R' group, wherein R' is selected from the group consisting of unsubstituted lower alkyl, trifluoromethyl, unsubstituted cycloalkyl, optionally substituted aryl with one or more, preferably 1, 2, or 3 substituents independently selected from unsubstituted lower alkyl, trifluoromethyl, unsubstituted lower alkoxy, halogen, and -NR¹⁰R¹¹, wherein R¹⁰ and R¹¹ are as defined above, optionally substituted heteroaryl with one or more, preferably 1, 2, or 3 substituents independently selected from unsubstituted lower alkyl, trifluoromethyl, unsubstituted lower alkoxy, halogen, and -NR¹⁰R¹¹ (bonded via a ring carbon atom), and optionally substituted heterocyclic aliphatic group with one or more, preferably 1, 2, or 3 substituents independently selected from unsubstituted lower alkyl, trifluoromethyl, unsubstituted lower alkoxy, halogen, and-NR¹⁰R¹¹ (bonded via a ring carbon atom). Representative sulfonyl groups include, but are not limited to, methylsulfonyl, trifluoromethylsulfonyl, benzenesulfonyl.

"Carbonyl group" refers to an organic functional group consisting of a carbon atom double-bonded to an oxygen atom (C=O).

"Quaternary ammonium salt" refers to a compound in which all four hydrogen atoms of an ammonium ion are replaced by hydrocarbon groups.

The compounds of the present invention have suitable solubility and can rapidly convert into the active metabolite rolapitant in plasma and liver, addressing the poor solubility of rolapitant and the difficulty in developing conventional injectable formulations, while also avoiding hemolytic effects and reducing injection risk. Compared to existing prodrug molecules (compound of formula (I)), the compounds of the present invention convert to release active rolapitant faster in vivo and achieve higher in vivo exposure of the active metabolite rolapitant, exhibiting superior pharmacokinetic properties.

### DETAILED DESCRIPTION OF THE INVENTION

The following examples are provided to facilitate a better understanding of the present invention, but are not intended to limit the invention. Unless otherwise stated, the experimental methods used in the following examples are conventional methods. Unless otherwise specified, the reagents and materials used in the following examples were obtained from commercial biochemical suppliers.

### Example 1

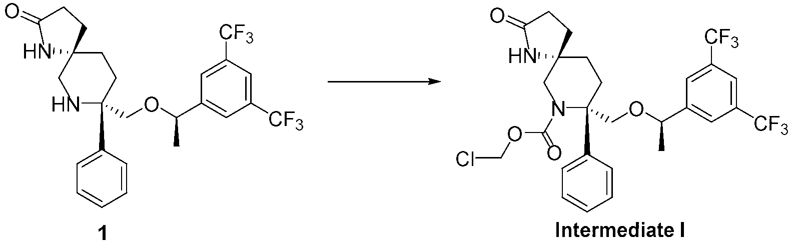

Under N₂ protection, Compound 1 (3.0 g, 6.0 mmol) was weighed into a 100 mL three-neck flask and dissolved in dichloromethane (45 mL). Diisopropylethylamine (6.2 g, 48 mmol) was added, and the mixture was cooled to -30°C. Trimethylsilyl chloride (1.69 g, 15.6 mmol) was added, and the reaction was stirred at room temperature for 2 h. The mixture was cooled to -25°C, and a dichloromethane solution of chloromethyl chloroformate (950 mg, 7.30 mmol) was added dropwise, maintaining the temperature between -25°C and -5°C until the reaction was complete. The reaction mixture was poured into ice water, and the layers were separated. The aqueous layer was extracted with dichloromethane, then water and 1 N dilute hydrochloric acid were added, and the layers were separated again. The organic phase was successively washed with water, saturated sodium bicarbonate solution, and brine, then dried over anhydrous sodium sulfate, filtered, and concentrated. The residue was purified by column chromatography (petroleum ether/ethyl acetate = 1/3) to afford 3.0 g of Intermediate I with a yield of 83.3%. LC-MS [M+H]⁺: 593.2.

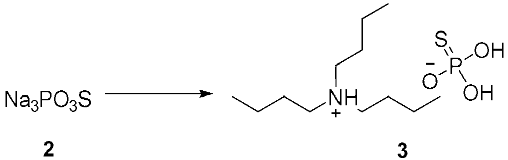

Compound 2 (1.439 g, 12.6 mmol) was dissolved in water (4 mL) and passed through an ion exchange resin (Dowex 50WX-8, H+), washing with purified water. The eluate was cooled in an ice bath to 0 °C, then tributyamine (2.34 g, 12.6 mmol) was added. The mixture was allowed to warm to room temperature and stirred for 2 h, then lyophilized to afford 3.0 g of Compound 3, with a yield of 80.1%, which was used directly in the next step.

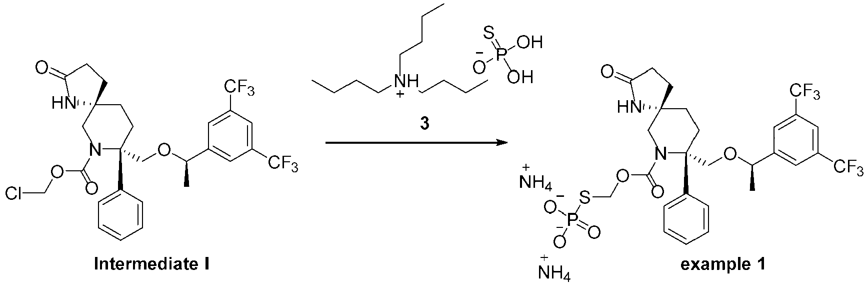

Under N₂ protection, Intermediate I (200 mg, 0.336 mmol) was weighed into a 100 mL three-neck flask and dissolved in acetonitrile (5 mL). Compound 3 (1.5 g, 5.04 mmol) was added, and the reaction mixture was stirred at room temperature for 2 h. The reaction mixture was purified by preparative separation (Solvent A: 0.05% ammonia, Solvent B: acetonitrile) to obtain 3.6 mg of the compound of example 1, with a yield of 1.49%. LC-MS [M+H]⁺: 670.1. ¹H NMR (400 MHz, DMSO-*d₆*) δ 8.16 (s, 1H), 8.00 (s, 1H), 7.95 (s, 2H), 7.39 - 6.98 (m, 9H), 5.25 - 5.17 (m, 1H), 5.11 - 5.02 (m, 1H), 4.69 - 4.61 (m, 1H), 4.09 - 3.97 (m, 2H), 3.53 (d, J = 13.6 Hz, 1H), 3.41 (d, J = 13.8 Hz, 1H), 2.45 - 2.30 (m, 3H), 2.12 - 2.02 (m, 2H), 1.96 - 1.88 (m, 1H), 1.64 - 1.55 (m, 2H), 1.36 (d, J = 6.4 Hz, 3H).

### Example 2

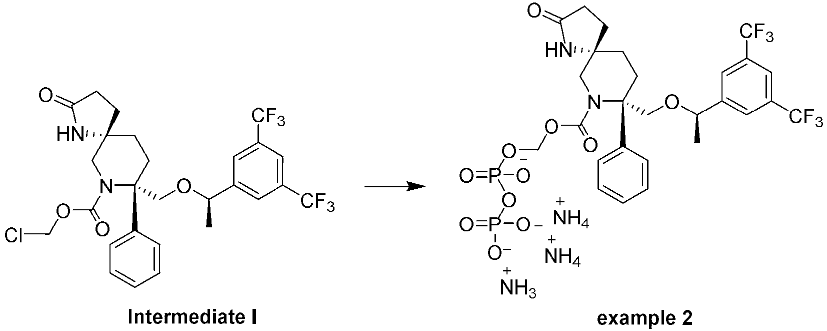

Under N₂ protection, tris(tetrabutylammonium) hydrogen pyrophosphate (567 mg, 1.36 mmol) was weighed into a 100 mL three-neck flask and dissolved in acetonitrile (5 mL). A solution of Intermediate I (400 mg, 0.68 mmol) in acetonitrile (5 mL) was added, and the reaction mixture was stirred at room temperature for 3 h. The crude product was directly purified by preparative HPLC (Solvent A: 0.1% HN₃·H₂O, Solvent B: acetonitrile) to afford 150 mg of the compound of example 2, with a yield of 29.56%. LC-MS: 734.6[M+H] ⁺. ¹H NMR (400 MHz, DMSO-*d₆*) δ 8.49 (s, 1H), 7.98 (s, 1H), 7.94 (s, 2H), 7.84 - 5.90 (m, 11H), 5.47 - 5.36 (m, 1H), 5.26 (d, *J =* 6.8 Hz, 1H), 4.62 (s, 1H), 4.17 (d, *J* = *7.7* Hz, 1H), 3.98 (d, *J =* 9.0 Hz, 1H), 3.70 (d, *J =* 13.7 Hz, 1H), 3.14 (d, *J =* 13.8 Hz, 1H), 2.33 (s, 2H), 2.19 - 2.05 (m, 2H), 1.94 (s, 1H), 1.65 - 1.55 (m, 2H), 1.42 (t, *J* = 11.9 Hz, 1H), 1.32 (d, *J =* 6.3 Hz, 3H).

### Example 3

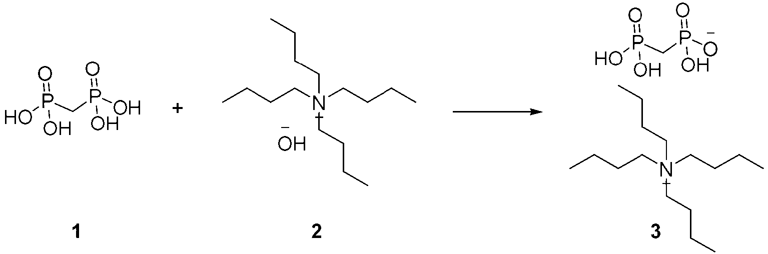

Compound 1 (200 mg, 1.36 mmol) was weighed into a 100 mL round-bottom flask and dissolved in 10 mL of water. Compound 2 (40% aqueous solution) was added slowly to adjust the solution pH to 8-9, and the mixture was lyophilized to afford crude Compound 3 (240 mg), which was used directly in the next step.

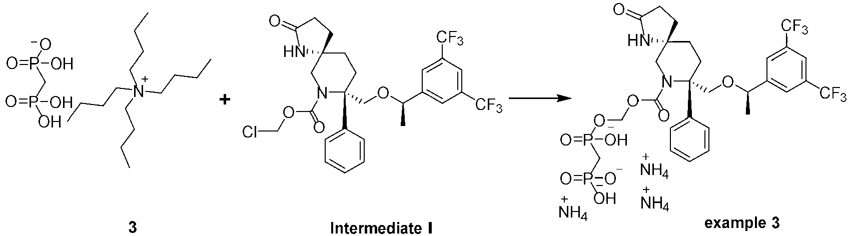

Compound 3 (240 mg) was added to a solution of Intermediate I (150 mg, 0.25 mmol) in anhydrous acetonitrile (3 mL). The resulting solution was stirred at room temperature for 1 h, then the solvent was removed under vacuum. The residue was purified by preparative HPLC (Gemini-C18 150 × 21.2 mm, 5 µm, ACN-H₂O, 0.1% NH₄HCO₃) to afford 6 mg of the compound of example 3, with a yield of 3.25%. LC-MS: 733.1 [M+H] ⁺. ¹H NMR (400 MHz, MeOD) δ 7.91 - 7.81 (m, 3H), 7.46 - 7.35 (m, 4H), 7.32 - 7.25 (m, 1H), 5.67 - 5.60 (m, 1H), 5.57 - 5.50 (m, 1H), 4.64 - 4.54 (m, 1H), 4.26 - 4.18 (m, 1H), 4.18 - 4.09 (m, 1H), 4.00 - 3.94 (m, 1H), 3.25 - 3.14 (m, 1H), 2.53 - 2.32 (m, 4H), 2.21 (t, J = 19.5 Hz, 2H), 2.09 - 1.98 (m, 1H), 1.88 - 1.76 (m, 2H), 1.74 - 1.65 (m, 1H), 1.38 (d, J = 6.4 Hz, 3H).

### Example 4

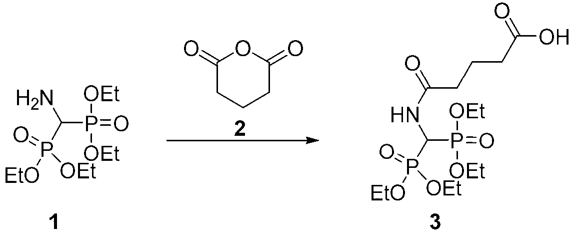

Compound 1 (200 mg, 0.66 mmol) was dissolved in 5 mL of anhydrous dichloromethane, and Compound 2 (75 mg, 0.67 mmol) and 4-dimethylaminopyridine (8.6 mg, 0.066 mmol) were added. The mixture was stirred at room temperature overnight. The solvent was then removed under vacuum. The crude product was purified by silica gel column chromatography (dichloromethane/methanol = 1/5) to afford 200 mg of Compound 3, with a yield of 72.6%. ¹H NMR (400 MHz, MeOD) δ 5.12 (t, J = 23.1 Hz, 1H), 4.30 - 4.12 (m, 8H), 2.44 - 2.34 (m, 4H), 2.00 - 1.85 (m, 2H), 1.43 - 1.33 (m, 12H).

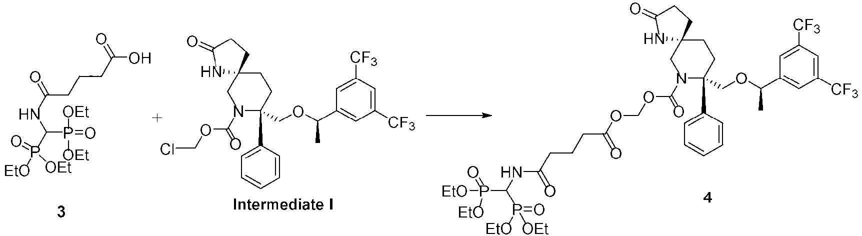

Compound 3 (100 mg, 0.168 mmol), Intermediate I (140 mg, 0.336 mmol), sodium iodide (50.43 mg, 0.336 mmol), and potassium bicarbonate (33.66 mg, 0.336 mmol) were dissolved in anhydrous dimethylformamide (3 mL). The solution was stirred under nitrogen at 25°C for 16 h. The reaction was quenched with water (50 mL) and extracted three times with ethyl acetate (50 mL). The combined organic layers were washed with water and brine, dried over sodium sulfate, filtered, concentrated, and purified by preparative HPLC (Gemini-C18 150 × 21.2 mm, 5 µm, ACN-H₂O, 0.1% NH₄HCO₃) to afford 65 mg of Compound 4, with a yield of 39.62%. LC-MS: 974.3 [M+H] ⁺.

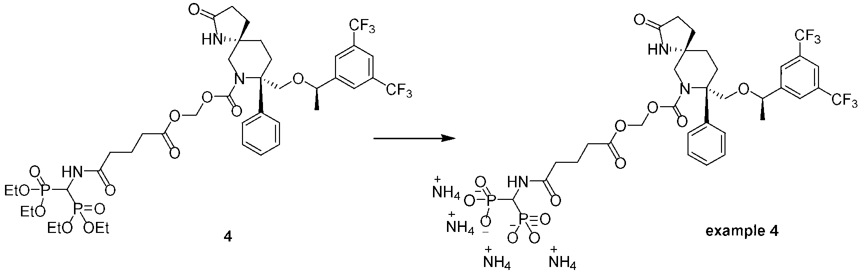

To a solution of Compound 4 (20 mg, 0.021 mmol) in anhydrous dichloromethane (2 mL) was added trimethylsilyl bromide (31 mg, 0.205 mmol). The mixture was stirred at -10°C for 16 h. The solvent was removed under vacuum, and the residue was purified by preparative HPLC (Gemini-C18 150 × 21.2 mm, 5 µm, ACN-H₂O, 0.1 % NH₄HCO₃) to afford 2 mg of the compound of example 4, with a yield of 11.22%. LC-MS: 861.6[M+H] ⁺. ¹H NMR (400 MHz, D₂O) δ 7.94 (s, 1H), 7.84 (s, 2H), 7.34 - 7.18 (m, 3H), 7.16 - 7.01 (m, 2H), 5.64 - 5.48 (m, 1H), 5.33 - 5.19 (m, 1H), 4.59 - 4.51 (m, 1H), 4.30 (t, J = 19.1 Hz, 1H), 4.07 - 3.96 (m, 2H), 3.74 - 3.58 (m, 2H), 2.54 - 2.13 (m, 8H), 1.88 - 1.58 (m, 6H), 1.50 - 1.40 (m, 3H).

### Example 5

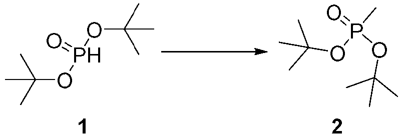

Compound 1 (5 g, 25.7 mmol) was dissolved in 40 mL of anhydrous acetonitrile. At 0°C, sodium hydride (1.54 g, 38.5 mmol) was added, and the reaction mixture was stirred under nitrogen at room temperature for 30 minutes. Methyl iodide (5.47 g, 38.5 mmol) was then added slowly, and the mixture was stirred overnight at ambient temperature. After completion, the reaction was quenched with 1 mL of water, concentrated to dryness, and the crude product was purified by silica gel column chromatography (petroleum ether/ethyl acetate = 1/1) to afford 4.74 g of Compound 2, with a yield of 88.7%. ¹H NMR (400 MHz, DMSO-*d₆*) δ 1.42 (s, 18H), 1.32 (d, J = 17.2 Hz, 3H).

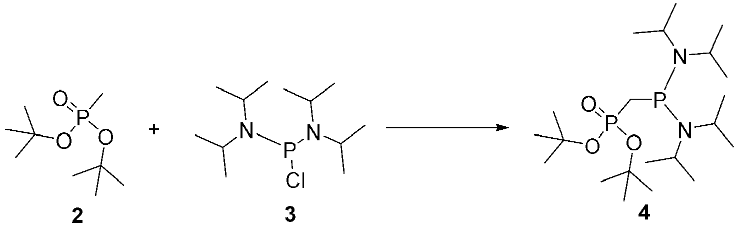

Compound 2 (3.6 g, 17.3 mmol) was dissolved in 60 mL of anhydrous tetrahydrofuran under nitrogen, and the solution was cooled to -78°C. Diisopropylamino lithium (17.3 mL, 34.6 mmol, 2.0 M) was added dropwise, and the reaction mixture was stirred at this temperature for 30 minutes. A solution of Compound 3 (4.62 g, 17.3 mmol) in tetrahydrofuran (35 mL) was then added. The mixture was stirred and allowed to warm to room temperature overnight. The reaction was quenched with saturated sodium bicarbonate solution and extracted three times with ethyl acetate (200 mL). The combined organic layers were washed with saturated brine, dried over anhydrous sodium sulfate, filtered, and concentrated. The residue was purified by silica gel column chromatography (petroleum ether/ethyl acetate = 20/1 + 1% triethylamine) to afford 1.9 g of Compound 4, with a yield of 24.28%. ¹HNMR (400 MHz, CDCl₃) δ 3.42 - 3.32 (m, 4H), 2.10 (dd, J = 20.7, 1.3 Hz, 2H), 1.50 (s, 18H), 1.19 - 1.14 (m, 24H).

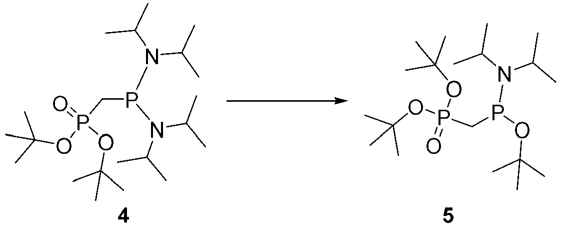

4,5-Dicyanoimidazole (300 mg, 2.6 mmol, 1 eq) was added slowly to a mixture of Compound 4 (1.9 g, 4.3 mmol) and tert-butanol (320 mg, 4.3 mmol) in dichloromethane (20 mL). The reaction mixture was stirred at room temperature for 4 hours and then concentrated. The crude product was purified by silica gel column chromatography (petroleum ether/ethyl acetate = 10/1 + 1% triethylamine) to afford 900 mg of Compound 5, with a yield of 51.16%. ¹HNMR (400 MHz, CDCl₃) δ 3.60 - 3.38 (m, 2H), 2.21 - 2.07 (m, 1H), 1.92 - 1.81 (m, 1H), 1.50 (s, 18H), 1.30 (s, 9H), 1.17 (d, J = 6.6 Hz, 6H), 1.10 (d, J = 6.7 Hz, 6H).

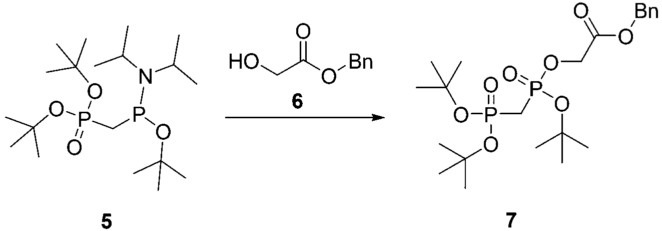

Compound 5 (300 mg, 0.73 mmol) and Compound 6 (122 mg, 0.73 mmol) were dissolved in 2 mL of dimethylformamide, and tetrazole (204 mg, 2.92 mmol) was added. The mixture was stirred at room temperature for 2 hours. Hydrogen peroxide solution (165 mg, 30%) was added dropwise at 0°C, and the mixture was stirred for 16 hours. The reaction was quenched with water (50 mL) and extracted three times with ethyl acetate (50 mL). The combined organic layers were washed with water and saturated brine, dried over sodium sulfate, filtered, concentrated, and the residue was purified by silica gel column chromatography (dichloromethane/methanol = 100/0-92/8) to afford 150 mg of Compound 7, with a yield of 41.70%. ¹H NMR (400 MHz, MeOD) δ 7.44 - 7.33 (m, 5H), 5.27 - 5.18 (m, 2H), 4.79 - 4.65 (m, 2H), 2.67 - 2.49 (m, J = 22.2, 20.8 Hz, 2H), 1.57 - 1.51 (m, 27H).

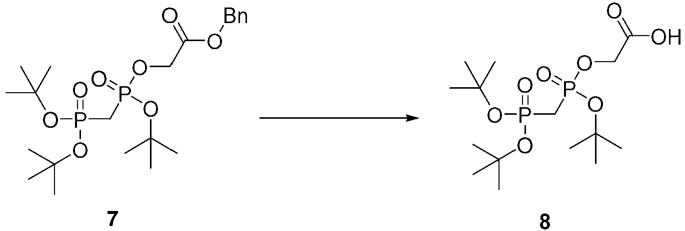

Compound 7 (100 mg, 0.203 mmol) was dissolved in ethyl acetate (3 mL), and Pd/C (100 mg) was added. The mixture was stirred under hydrogen at room temperature for 4 hours. The solid was filtered off, and the filtrate was concentrated under vacuum to afford 70 mg of Compound 8, with a yield of 85.88%. ¹H NMR (400 MHz, CD₃OD) δ 4.66 - 4.49 (m, 2H), 2.63 - 2.49 (m, 2H), 1.55 - 1.50 (m, 27H).

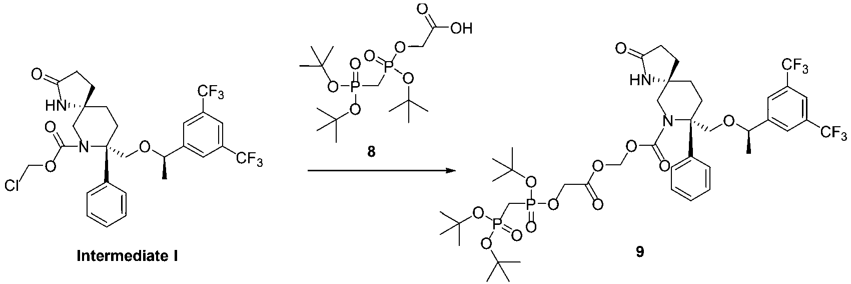

Compound 8 (176 mg, 0.437 mmol), Intermediate I (130 mg, 0.219 mmol), sodium iodide (65.55 mg, 0.437 mmol), and potassium bicarbonate (43.75 mg, 0.437 mmol) were dissolved in anhydrous dimethylformamide (5 mL) and stirred under nitrogen at 25°C for 16 hours. The reaction was quenched with water (50 mL) and extracted three times with ethyl acetate (50 mL). The combined organic layers were washed with water and brine, dried over sodium sulfate, filtered, concentrated, and the residue was purified by preparative HPLC (A: 0.1% NH₄HCO₃, B: acetonitrile) to afford 70 mg of Compound 9, with a yield of 33.36%. LC-MS: 981.2[M+Na] ⁺.

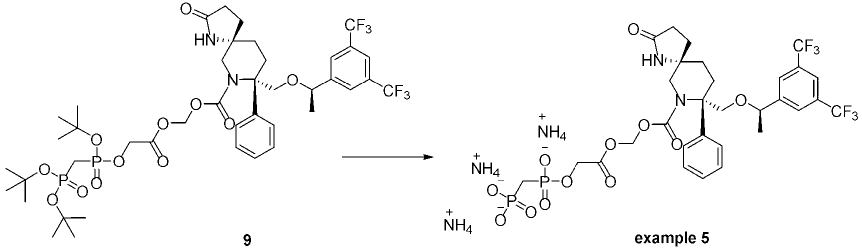

Compound 9 (25 mg, 0.026 mmol) was dissolved in anhydrous dichloromethane (2 mL), and phosphoric acid (0.1 mL) was added at -10°C. The solution was stirred at -10°C for 1 hour, then the solvent was removed under vacuum, and the residue was purified by preparative HPLC (Gemini-C18 150 × 21.2 mm, 5 µm, ACN-H₂O, 0.1% NH₄HCO₃) to afford 1 mg of the compound of example 5, with a yield of 4.85%. LC-MS: 790.6[M+H] ⁺.

### Example 6

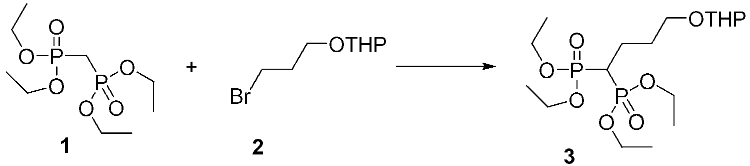

Under ice-bath conditions, a suspension of sodium hydride (5.33 g, 0.1332 mol) in N,N-dimethylformamide (30 mL) was prepared, and Compound 1 (12.8 g, 0.0444 mol) was added dropwise. The reaction mixture was stirred under nitrogen at 25°C for 1 hour. A solution of Compound 2 (12.88 g, 0.1332 mol) in N,N-dimethylformamide (10 mL) was added slowly to the reaction mixture, and the mixture was stirred at 65°C for 16 hours. Saturated ammonium chloride solution (500 mL) was added to the reaction system, and the aqueous phase was extracted three times with ethyl acetate (100 mL each). The combined organic layers were washed with saturated brine (50 mL), dried over anhydrous sodium sulfate, filtered, concentrated, and the residue was purified by silica gel column chromatography (dichloromethane/methanol = 97/3) to give 13.8 g of crude Compound 3, yield 35.98%, which was used directly in the next step. LC-MS: 882.6.[2M+Na]⁺.

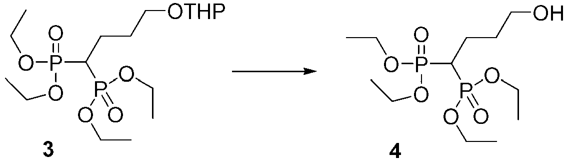

At room temperature, Compound 3 (2.4 g, 5.6 mmol) was dissolved in methanol (10 mL), and p-toluenesulfonic acid (0.19 g, 1.1 mmol) was added. The reaction mixture was stirred under nitrogen at 25°C for 16 hours. Saturated sodium bicarbonate solution was added to adjust the pH to 8-9, and the resulting solution was concentrated to dryness. The residue was purified by silica gel column chromatography (dichloromethane/methanol = 96/4) to give 0.8 g of Compound 4, yield 37.50%. LC-MS: 346.9[M+H]⁺.

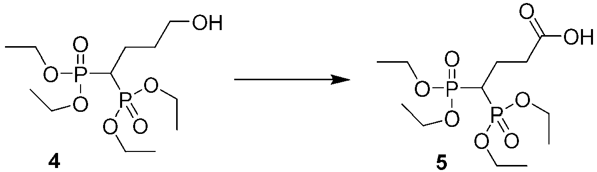

At room temperature, Compound 4 (1.2 g, 3.5 mmol) was dissolved in DMF (20 mL), and PDC (13.17 g, 35 mmol) was added. The reaction mixture was stirred under nitrogen at 25°C for 16 hours. The reaction was diluted with water (300 mL), and the solution was adjusted to pH 8.0 with 1 M sodium hydroxide. The solution was cooled to 0°C, and a 6.1 wt% Na₂SO₃ solution (30 mL) was added slowly. The mixture was stirred at 0°C for 30 minutes and washed with ether (30 mL). The aqueous layer was acidified to pH 3-4 with concentrated HCl and extracted three times with chloroform/isopropanol (4:1, 30 mL each). The combined organic layers were dried over anhydrous sodium sulfate, filtered, concentrated, and the residue was purified by silica gel column chromatography (dichloromethane/methanol = 90/10-85/15) to give 0.35 g of Compound 5, yield 22.86%. LC-MS: 361.1 [M+H]⁺.

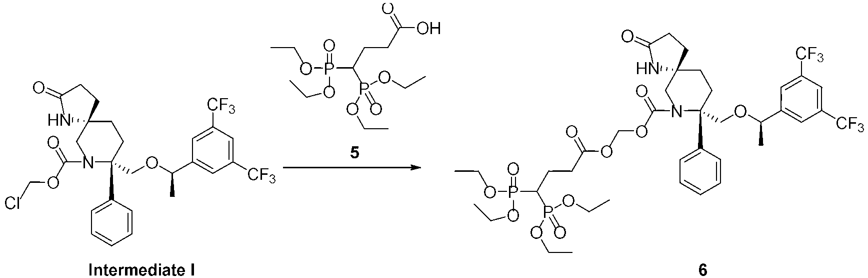

Compound 5 (227 mg, 0.63 mmol), intermediate I (150 mg, 0.25 mmol), sodium iodide (76 mg, 0.63 mmol), and potassium bicarbonate (50 mg, 0.50 mmol) were dissolved in anhydrous N,N-dimethylformamide (3 mL) and stirred under nitrogen at 30°C for 16 hours. The reaction was quenched with water (50 mL) and extracted three times with ethyl acetate (50 mL each). The combined organic layers were washed sequentially with water and saturated brine, dried over anhydrous sodium sulfate, filtered, concentrated, and the residue was purified by silica gel column chromatography (dichloromethane/methanol = 96/4) to give 160 mg of Compound 6, yield 62.16%. LC-MS: 917.2[M+H]⁺.

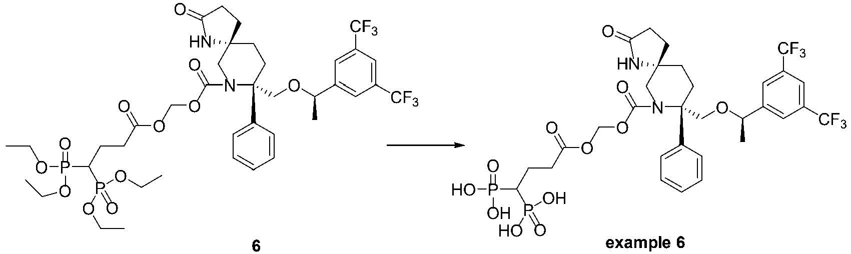

Compound 6 (30 mg, 0.032 mmol) was dissolved in anhydrous dichloromethane (2 mL), and trimethylbromosilane (175 mg, 1.144 mmol) was added. The solution was stirred at -10°C for 16 hours. The solvent was removed under vacuum, and the residue was purified by preparative HPLC (Gemini-C18 150 × 21.2 mm, 5 µm, ACN-H₂O, 0.1% TFA) to give 2 mg of the compound of example 6, yield 7.8%. LC-MS: 804.6[M+H]⁺.

### Example 7

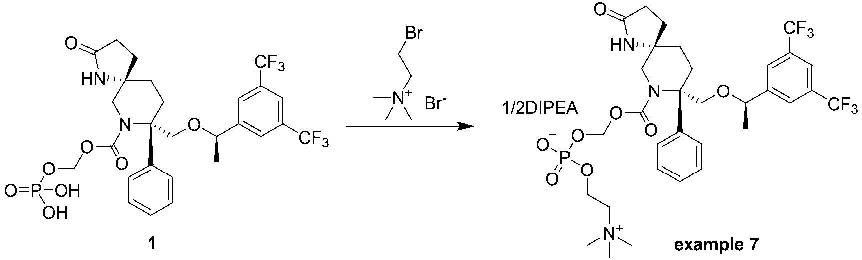

Under nitrogen protection, Compound 1 (20 mg, 0.031 mmol) was dissolved in N,N-dimethylformamide (1 mL) in a 25 mL three-necked flask. A solution of (2-bromoethyl)trimethylammonium bromide (38 mg, 0.155 mmol) and 4 Å molecular sieves (20 mg) in N,N-dimethylformamide (1 mL) was added, followed by a solution of diisopropylethylamine (80 mg, 0.62 mmol) in N,N-dimethylformamide (1 mL). The mixture was stirred at 55°C for 3 hours. After filtration, the crude reaction mixture was purified directly by HPLC (A: 0.1% NH₄HCO₃, B: acetonitrile) to give 5 mg of the compound of example 7, yield 20.35%. LC-MS: 740.2[M+H] ⁺. ¹H NMR (400 MHz, DMSO-*d₆*) δ 7.90 (s, 1H), 7.80 (s, 2H), 7.28 (t, *J =* 7.5 Hz, 2H), 7.23 - 7.15 (m, 3H), 5.17 (dd, *J =* 9.7, 5.3 Hz, 1H), 4.01 (s, 2H), 3.88 (s, 1H), 3.78 - 3.53 (m, 3H), 3.42 - 3.27 (m, 3H), 3.12 - 3.01 (m, 3H), 2.95 (s, 9H), 2.40 - 2.26 (m, 3H), 2.02 - 1.93 (m, 2H), 1.79 - 1.69 (m, 2H), 1.55 (t, *J* = 11.4 Hz, 1H), 1.38 (d, *J* = 6.4 Hz, 3H), 1.20 (d, *J* = 6.6 Hz, 8H).

### Example 8

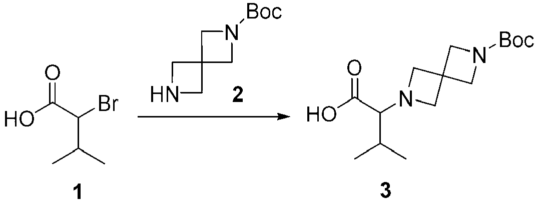

Compound 1 (300 mg, 1.66 mmol), Compound 2 (396 mg, 1.99 mmol, 1.2 eq), and potassium carbonate (458 mg, 3.31 mmol, 2 eq) were dissolved in anhydrous tetrahydrofuran (10 mL) and stirred at 25°C under nitrogen protection for 16 hours. The reaction was then quenched with water (50 mL) and extracted with ethyl acetate (50 mL) three times. The combined organic layers were washed successively with water and saturated brine, dried over sodium sulfate, filtered, and concentrated to give 260 mg of Compound 3, which was used directly in the next step without further purification. LC-MS: 299.2[M+H]⁺.

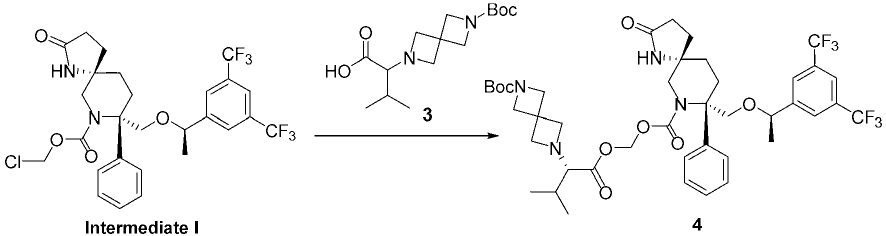

Compound 3 (190 mg) was dissolved in dimethylformamide (5 mL), and Intermediate I (113 mg, 0.19 mmol) and sodium iodide (57 mg, 0.38 mmol) were added. The solution was stirred at room temperature under nitrogen protection for 16 hours. The mixture was concentrated, and the residue was purified by preparative HPLC (A: 0.1% NH₄HCO₃, B: acetonitrile) to give 75 mg of Compound 4, with a yield of 15.62%. LC-MS: 855.4 [M+H]⁺.

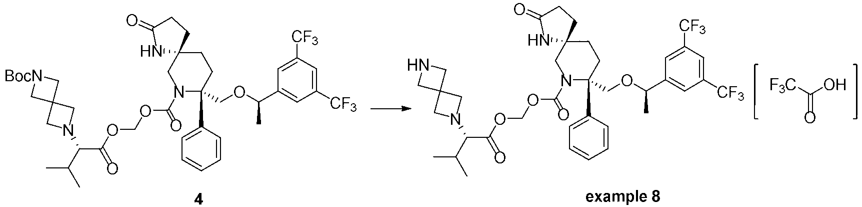

Compound 4 (80 mg, 0.09 mmol) was dissolved in anhydrous dichloromethane (4 mL), and trifluoroacetic acid (1 mL) was added at 0°C. The reaction mixture was stirred at room temperature for 2 hours, after which the solvent was removed under reduced pressure. The residue was purified by preparative HPLC (A: 0.1% TFA, B: acetonitrile) and lyophilized to give 15 mg of the compound of example 8, with a yield of 21.20%. LC-MS: 755.0[M+H]⁺. ¹H NMR (400 MHz, D₂O) δ 7.93 (s, 1H), 7.82 (s, 2H), 7.35 - 7.19 (m, 3H), 7.18 - 7.07 (m, 2H), 5.83 - 5.72 (m, 1H), 5.61 - 5.51 (m, 1H), 4.60 - 4.53 (m, 1H), 4.50 - 4.34 (m, 3H), 4.30 (s, 4H), 4.16 - 4.02 (m, 2H), 3.91 - 3.83 (m, 1H), 3.72 - 3.62 (m, 1H), 3.39 - 3.22 (m, 2H), 2.45 - 2.26 (m, 3H), 2.13 - 1.77 (m, 5H), 1.63-1.59 (m, 1H), 1.41 (d, J = 6.4 Hz, 3H), 1.01 - 0.65 (m, 6H).

### Example 9

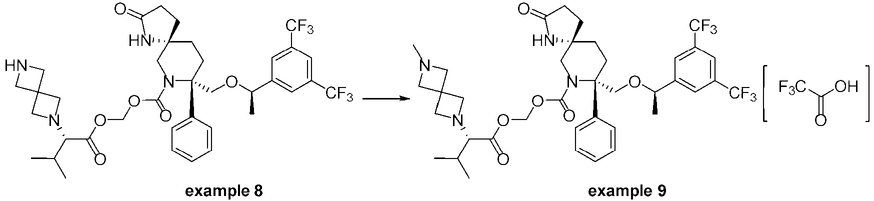

The compound of example 8 (50 mg, 0.066 mmol) was dissolved in methanol (3 mL), and sodium cyanoborohydride (8.31 mg, 0.132 mmol) and paraformaldehyde (9.92 mg, 0.331 mmol) were added at 0°C. The reaction mixture was stirred at room temperature for 16 hours, then filtered and concentrated. The residue was purified by preparative HPLC (A: 0.1% TFA, B: acetonitrile) and lyophilized to give 8 mg of the compound of example 9, with a yield of 15.73%. LC-MS: 768.8[M+H]⁺. ¹H NMR (400 MHz, DMSO-*d₆*) δ 10.85 - 10.52 (m, 1H), 9.95 - 9.78 (m, 1H), 8.24 - 8.13 (m, 1H), 8.09 - 7.92 (m, 3H), 7.37 - 7.14 (m, 5H), 5.95 - 5.77 (m, 1H), 5.68 - 5.54 (m, 1H), 4.72 - 4.64 (m, 1H), 4.52 - 4.26 (m, 5H), 4.16 - 3.92 (m, 6H), 3.60 - 3.37 (m, 3H), 2.85 - 2.75 (m, 3H), 2.28 - 2.13 (m, 2H), 2.10 - 1.83 (m, 3H), 1.80 - 1.61 (m, 2H), 1.51 - 1.33 (m, 4H), 1.07 - 0.91 (m, 3H), 0.91 - 0.72 (m, 3H).

### Example 10

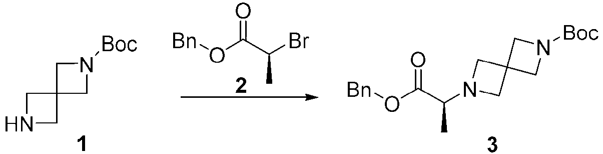

Compound 1 (300 mg, 1.51 mmol), compound 2 (441 mg, 1.81 mmol), and potassium carbonate (416 mg, 3.01 mmol) were dissolved in anhydrous acetonitrile (10 mL), and the mixture was stirred at 60°C under nitrogen protection for 16 hours. The reaction was then quenched with water (50 mL) and extracted three times with ethyl acetate (50 mL). The combined organic phases were washed successively with water and brine, dried over sodium sulfate, filtered, and concentrated to give 500 mg of Compound 3, which was used directly in the next step without purification. LC-MS: 361.2 [M+H]⁺.

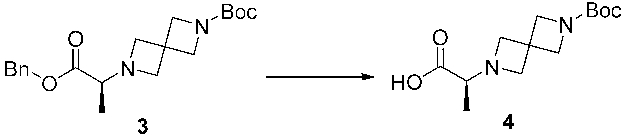

Compound 3 (500 mg) was dissolved in ethyl acetate (10 mL), and Pd/C (500 mg) was added. The mixture was stirred at room temperature under a hydrogen atmosphere for 6 hours. After filtration, the filtrate was concentrated to dryness to give 400 mg of Compound 4, which was used directly in the next step without purification. LC-MS: 271.1 [M+H]⁺.

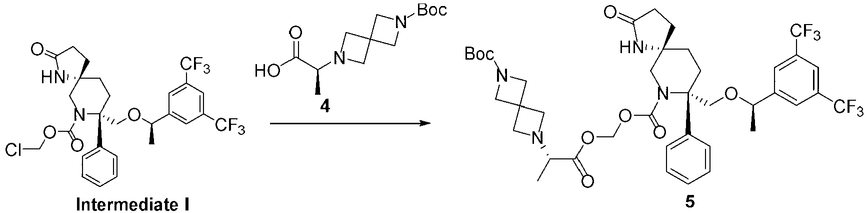

Intermediate I (300 mg, 0.50 mmol) was dissolved in anhydrous N,N-dimethylformamide (5 mL), and sodium iodide (151 mg, 1.0 mmol), potassium bicarbonate (101 mg, 1.0 mmol), and Compound 4 (273.6 mg, 1.008 mmol) were added. The reaction mixture was stirred at room temperature under nitrogen protection for 2 hours, then quenched with water (50 mL) and extracted three times with ethyl acetate (50 mL). The combined organic phases were washed successively with water and brine, dried over sodium sulfate, filtered, concentrated, and purified by silica gel column chromatography (petroleum ether/ethyl acetate = 1/1) to give 150 mg of Compound 5, with a yield of 35.86%. LC-MS: 827.3 [M+H]⁺.

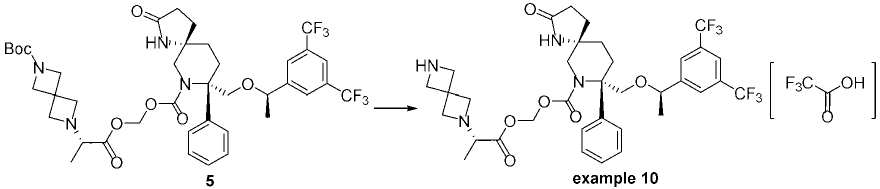

Compound 5 (50 mg, 0.0603 mmol) was dissolved in dichloromethane (3 mL), and trifluoroacetic acid (1 mL) was added at 0°C under nitrogen protection. The reaction mixture was stirred at room temperature for 1 hour, then concentrated and purified by preparative HPLC (A: 0.1% TFA, B: acetonitrile). After lyophilization, 10 mg of the compound of example 10 was obtained, with a yield of 22.72%. LC-MS: 727.2[M+H]⁺. ¹H NMR (400 MHz, MeOD) δ 7.89 (s, 3H), 7.44 - 7.36 (m, 2H), 7.36 - 7.28 (m, 3H), 5.99 - 5.69 (m, 2H), 4.58 - 4.25 (m, 11H), 4.22 - 4.14 (m, 1H), 4.10 - 4.05 (m, 1H), 3.95 - 3.82 (m, 1H), 2.49 - 2.34 (m, 4H), 2.02 - 1.92 (m, 1H), 1.91 - 1.82 (m, 2H), 1.80 - 1.72 (m, 1H), 1.52 - 1.46 (m, 3H), 1.44 - 1.39 (m, 3H).

### Example 11

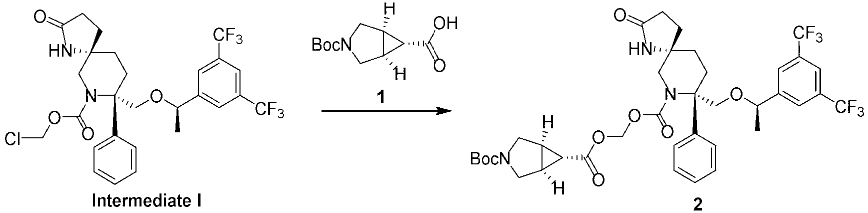

Compound 1 (115 mg, 0.50 mmol) and sodium iodide (101 mg, 0.67 mmol) were added to DMF (2 mL), followed by potassium bicarbonate (67.3 mg, 0.67 mmol). The reaction mixture was stirred at room temperature for 30 minutes, after which a solution of Intermediate I (200 mg, 0.34 mmol) in DMF (10 mL) was added dropwise. The reaction was allowed to proceed overnight. Water (50 mL) was added to quench the reaction, and the mixture was extracted twice with ethyl acetate (50 mL). The combined organic layers were dried over anhydrous sodium sulfate, filtered, and concentrated. The residue was purified by silica gel column chromatography (petroleum ether/ethyl acetate = 1/1) to give 200 mg of Compound 2 in a yield of 75.6%. LC-MS [M+H]⁺: 784.3.

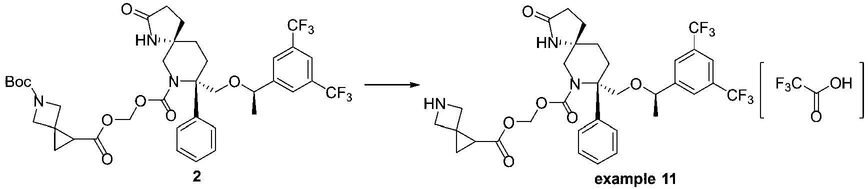

Compound 2 (200 mg, 0.25 mmol) was dissolved in dichloromethane (2 mL), and trifluoroacetic acid (0.5 mL) was added dropwise under an ice bath. The reaction mixture was warmed to room temperature and stirred for 1 hour, then concentrated. The crude product was purified by preparative HPLC (solution A: 0.1% TFA, solution B: acetonitrile) to afford 47.4 mg of the compound of example 11 in a yield of 26.6%. LC-MS [M+H]⁺: 684.3. ¹H NMR (400 MHz, D₂O) δ 7.62 - 7.38 (m, 3H), 7.16 - 6.78 (m, 5H), 5.85 - 5.13 (m, 2H), 4.36 - 3.99 (m, 5H), 3.94 - 3.50 (m, 3H), 3.11 - 2.49 (m, 1H), 2.42 - 1.98 (m, 3H), 1.95 - 1.71 (m, 2H), 1.65 - 1.41 (m, 2H), 1.40 - 1.25 (m, 2H), 1.23 - 0.62 (m, 5H).

### Example 12

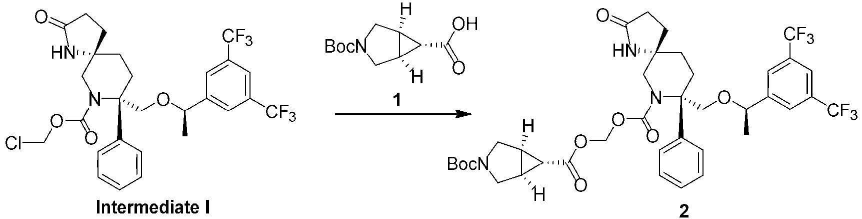

Under an N₂ atmosphere, Intermediate I (150 mg, 0.25 mmol) and Compound 1 (115 mg, 0.5 mmol) were weighed into a 25 mL three-necked flask and dissolved in DMF (3 mL). Sodium iodide (76 mg, 0.5 mmol) and potassium bicarbonate (76 mg, 0.75 mmol) were added, and the reaction mixture was stirred at room temperature for 5 hours. Water (10 mL) was added, and the mixture was extracted three times with ethyl acetate (5 mL). The combined organic layers were washed with saturated brine, dried over anhydrous sodium sulfate, and concentrated under reduced pressure. The residue was purified by silica gel column chromatography (dichloromethane/methanol = 3/1) to afford 160 mg of Compound 2 in a yield of 70.96%. LC/MS: 806.2 [M+Na]⁺.

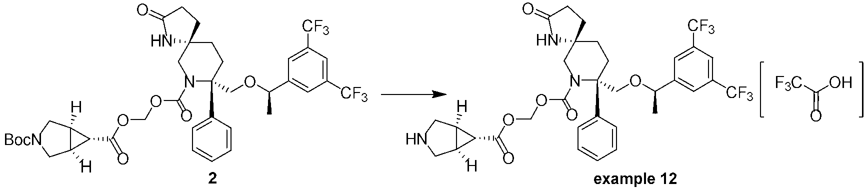

Compound 2 (160 mg, 0.2 mmol) was weighed into a 25 mL three-necked flask and dissolved in dichloromethane (3 mL). Trifluoroacetic acid (1 mL) was added, and the reaction mixture was stirred at room temperature for 2 hours. After concentration under reduced pressure, the residue was purified by C18 column chromatography (solution A: 0.1 % TFA, solution B: acetonitrile) to give 60 mg of the compound of example 12 in a yield of 42.99%. LC-MS: 684.2[M+H]⁺. ¹H NMR (400 MHz, MeOD) δ 7.88 (s, 2H), 7.85 (s, 1H), 7.32 (d, *J =* 6.6 Hz, 2H), 7.25 (s, 3H), 5.69 (s, 2H), 4.57 (s, 1H), 4.13 (s, 1H), 4.00 (d, *J* = 7.8 Hz, 1H), 3.77 (s, 1H), 3.50 (s, 4H), 2.65 - 2.06 (m, 7H), 2.00 (s, 1H), 1.81 (d, *J =* 11.6 Hz, 2H), 1.69 (d, *J* = 12.7 Hz, 2H), 1.41 (s, 3H).

### Example 13

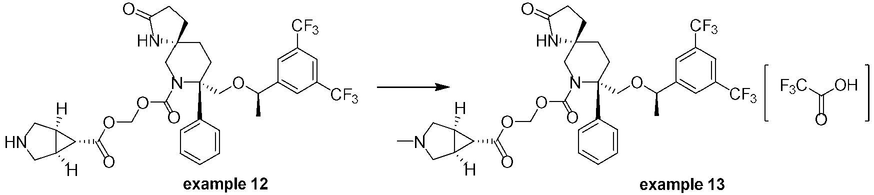

the compound of example 12 (20 mg, 0.03 mmol) was weighed into a 25 mL three-necked flask and dissolved in methanol (5 mL). Paraformaldehyde (3.5 mg, 0.12 mmol) was added, and the mixture was stirred at room temperature for 1 hour. Sodium cyanoborohydride (3.7 mg, 0.06 mmol) was then added, and stirring was continued at room temperature for 2 hours. The reaction mixture was filtered, and the filtrate was concentrated under reduced pressure. The residue was purified by preparative HPLC (solution A: 0.1% TFA, solution B: acetonitrile) to afford 2.5 mg of the compound of example 13, with a yield of 12.24%. LC-MS: 698.2[M+H] ⁺. ¹H NMR (400 MHz, MeOD) δ 7.87 (s, 2H), 7.85 (s, 1H), 7.33 (t, *J =* 7.3 Hz, 2H), 7.25 (t, *J =* 7.1 Hz, 3H), 5.68 (s, 2H), 4.57 (s, 1H), 4.13 (s, 1H), 3.99 (d, *J* = 8.7 Hz, 1H), 3.79 (dd, *J* = 11.4, 6.5 Hz, 3H), 3.44 (d, *J* = 11.4 Hz, 2H), 2.92 (s, 3H), 2.58 - 2.11 (m, 7H), 1.96 (s, 1H), 1.81 (dd, *J* = 10.0, 4.1 Hz, 3H), 1.73 - 1.61 (m, 1H), 1.41 (d, *J* = 5.2 Hz, 3H).

### Example 14

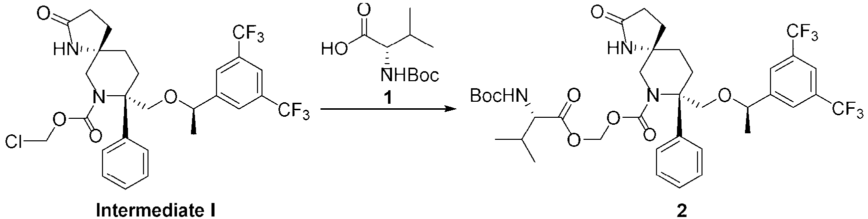

Compound 1 (109.53 mg, 0.504 mmol), sodium iodide (100.83 mg, 0.672 mmol), and potassium bicarbonate (67.3 mg, 0.672 mmol) were weighed into a 50 mL three-necked flask and dissolved in N,N-dimethylformamide (2 mL). The reaction mixture was stirred at 25 °C for 0.5 hours, after which a solution of Intermediate I (200 mg, 0.361 mmol) in N,N-dimethylformamide (5 mL) was added. The reaction mixture was stirred at room temperature for 18 hours. Water (20 mL) was added to quench the reaction, and the resulting solution was extracted with ethyl acetate (20 mL) three times. The combined organic phases were washed with saturated brine, dried over sodium sulfate, filtered, and concentrated. The residue was purified by silica gel column chromatography (petroleum ether/ethyl acetate = 80:20) to afford 200 mg of Compound 2, with a yield of 76.63%. LC-MS: 796.4 [M+Na]⁺.

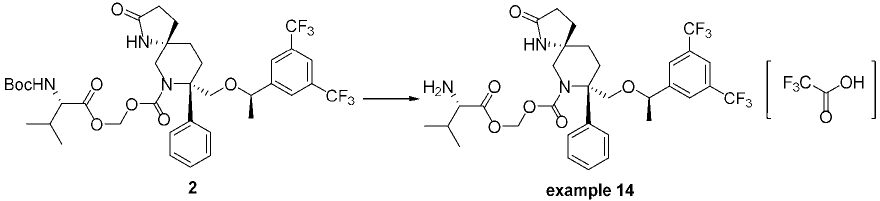

Compound 2 (200 mg, 0.258 mmol) was weighed into a 50 mL single-neck flask and dissolved in anhydrous dichloromethane (6 mL). Trifluoroacetic acid (2 mL) was added dropwise under an ice bath, and the reaction mixture was stirred at room temperature for 2 hours. The solvent was then removed under reduced pressure, and the residue was purified by preparative HPLC (Gemini-C18 150 × 21.2 mm, 5 µm, ACN-H₂O, 0.1% TFA) to afford 23.10 mg of the compound of example 14, with a yield of 13.27%. LC-MS: 674.3[M+H]⁺. ¹H NMR (400 MHz, CDCl₃) δ 8.29 (s, 1H), 7.71 (s, 1H), 7.60 (s, 2H), 7.37 (t, J = 7.3 Hz, 2H), 7.33 - 7.26 (m, 3H), 6.20 (s, 1H), 5.62 (d, J = 5.5 Hz, 1H), 4.26 (q, J = 6.2 Hz, 1H), 4.16 (d, J = 9.1 Hz, 1H), 4.03 (d, J = 9.4 Hz, 1H), 3.96 (d, J = 13.9 Hz, 1H), 3.82 (d, J = 4.6 Hz, 1H), 2.73 (d, J = 14.0 Hz, 1H), 2.53 (d, J = 14.9 Hz, 1H), 2.46 - 2.28 (m, 4H), 1.86 (d, J = 14.0 Hz, 1H), 1.74-1.70 (m, 3H), 1.24 (d, J = 6.3 Hz, 3H), 1.06 (dd, J = 11.5, 6.9 Hz, 6H).

### Example 15

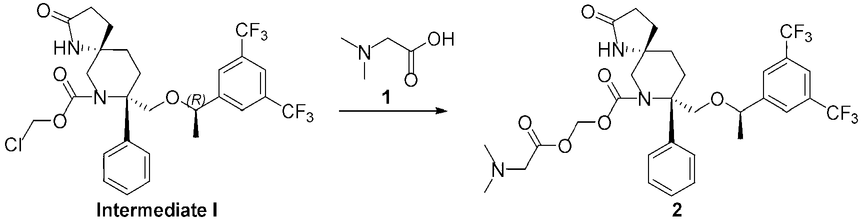

Intermediate I (200 mg, 0.336 mmol), Compound 1 (69 mg, 0.672 mmol), sodium iodide (101 mg, 0.672 mmol), and potassium bicarbonate (67 mg, 0.672 mmol) were dissolved in anhydrous N,N-dimethylformamide (6 mL), and the solution was stirred at 25 °C under nitrogen protection for 16 hours. The reaction was then quenched with water (50 mL) and extracted with ethyl acetate (50 mL) three times. The combined organic phases were washed successively with water and saturated brine, dried over sodium sulfate, filtered, and concentrated. The residue was purified by reversed-phase chromatography (ACN-H₂O, 0.1% NH₄HCO₃) to afford 150 mg of Compound 2, with a yield of 67.45%. LC-MS: 659.8 [M+H]⁺.

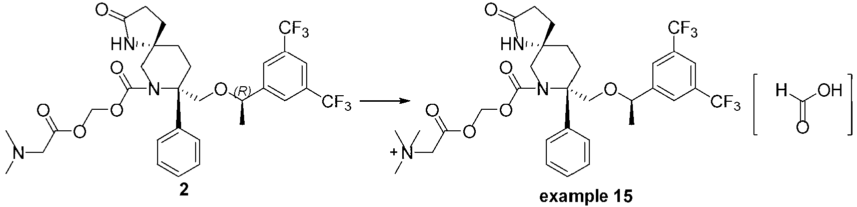

Compound 2 (150 mg, 0.227 mmol) was dissolved in anhydrous toluene (5 mL), and iodomethane (644 mg, 4.53 mmol) was added. The solution was stirred at 50 °C under nitrogen protection for 16 hours. The solvent was then removed under vacuum, and the residue was purified by preparative HPLC (Gemini-C18 150 × 21.2 mm, 5 µm, ACN-H₂O, 0.1% FA) to afford 8.46 mg of the compound of example 15, with a yield of 5.51%. LC-MS: 674.3[M+H] ⁺. ¹H NMR (400 MHz, D₂O) δ 8.07 - 7.72 (m, 3H), 7.44 - 7.14 (m, 5H), 5.49 - 5.14 (m, 1H), 4.61 - 4.45 (m, 1H), 4.38 - 4.00 (m, 2H), 3.92 - 3.49 (m, 6H), 3.30 - 3.00 (m, 4H), 2.79 - 2.14 (m, 8H), 2.04 - 1.64 (m, 4H), 1.54 - 1.32 (m, 3H).

### Example 16

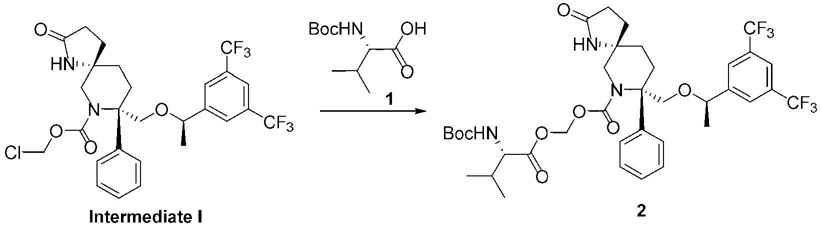

Compound 2 (109 mg, 0.504 mmol) and sodium iodide (101 mg, 0.672 mmol) were added to DMF (2 mL), followed by potassium bicarbonate (67.3 mg, 0.672 mmol). The mixture was stirred at room temperature for 0.5 hours, after which a solution of Intermediate I (200 mg, 0.336 mmol) in N,N-dimethylformamide (10 mL) was added dropwise to the reaction system. The reaction was stirred overnight, then quenched with water (50 mL) and extracted with ethyl acetate (50 mL) three times. The combined organic phases were washed successively with water and saturated brine, dried over sodium sulfate, filtered, and purified by silica gel column chromatography (petroleum ether/ethyl acetate = 1/1) to afford 210 mg of Compound 2, with a yield of 80.4%. LC-MS [M+H]⁺: 774.4.

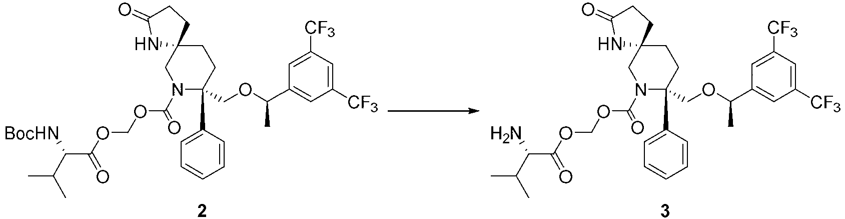

Compound 2 (210 mg, 0.271 mmol) was dissolved in dichloromethane (6 mL), and 1.5 mL of trifluoroacetic acid was added dropwise under ice-bath cooling. After addition, the reaction mixture was allowed to warm to room temperature and stirred for 1 hour, then concentrated. The residue was purified by silica gel column chromatography (dichloromethane/methanol = 10/1) to afford 150 mg of Compound 3, with a yield of 82%. LC-MS [M+H]⁺: 674.4.

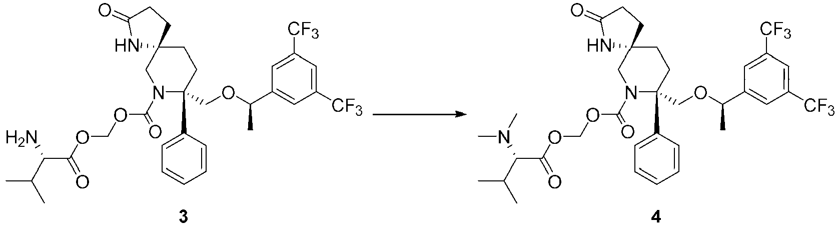

Compound 3 (50 mg, 0.074 mmol) and ethyl acetate (5 mL) were added to a 100 mL three-necked flask. Palladium on carbon (50 mg) and paraformaldehyde (88.9 mg, 2.96 mmol) were added portionwise at room temperature. The system was purged with hydrogen three times and then stirred at room temperature for 24 hours. After filtration, the filtrate was concentrated to afford 50 mg of Compound 4. LC-MS [M+H]⁺: 702.2.

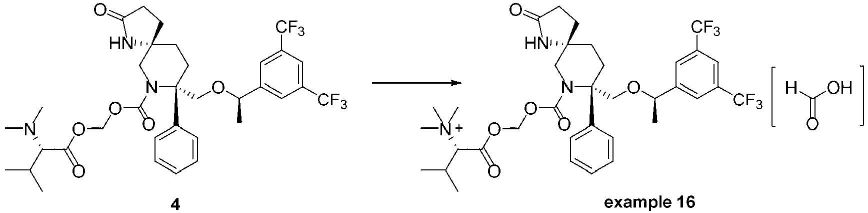

Compound 4 (50 mg, 0.0711 mmol, 1 eq) was dissolved in anhydrous toluene (5 mL) in a 100 mL single-necked flask, and iodomethane (404 mg, 2.844 mmol) was added. The mixture was stirred at 50 °C for 24 hours. The reaction mixture was concentrated, and the residue was purified by preparative HPLC (solution A: 0.1 % FA; solution B: acetonitrile) to afford 5.16 mg of the compound of example 16, with a yield of 10.1%. LC-MS [M+H]⁺: 716.0. ¹HNMR (400 MHz, D₂O) δ 7.94 - 7.60 (m, 3H), 7.30 - 7.00 (m, 5H), 5.91 - 5.50 (m, 2H), 4.62 - 4.28 (m, 1H), 4.09 - 3.81 (m, 2H), 3.70 - 3.58 (m, 1H), 3.50 - 3.24 (m, 1H), 3.12 (s, 10H), 2.55 - 2.20 (m, 4H), 2.08 - 1.82 (m, 2H), 1.81 - 1.72 (m, 1H), 1.65 - 1.51 (m, 1H), 1.38 (d, J = 5.0 Hz, 3H), 1.25 - 1.12 (m, 1H), 1.10 - 0.92 (m, 3H), 0.87 - 0.61 (m, 3H).

### Example 17

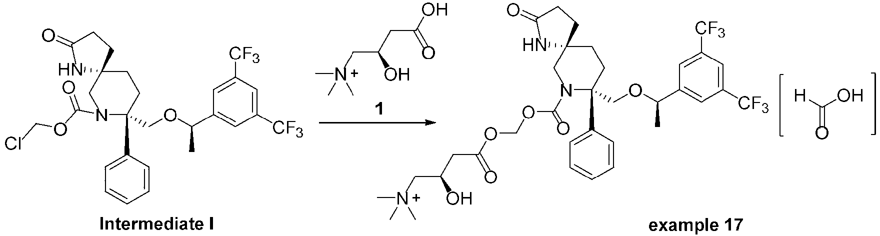

Intermediate I (100 mg, 0.168 mmol) was dissolved in anhydrous N,N-dimethylformamide (3 mL), and sodium iodide (50.42 mg, 0.336 mmol), potassium bicarbonate (33.65 mg, 0.336 mmol), and Compound 1 (54.37 mg, 0.336 mmol) were added. The reaction mixture was stirred at room temperature under nitrogen protection for 18 hours, then quenched with water (3 mL) and extracted with ethyl acetate (3 mL) three times. The aqueous phase was collected and purified by preparative HPLC (Gemini-C18 150 × 21.2 mm, 5 µm, ACN-H₂O, 0.1% FA), followed by lyophilization to afford 3.3 mg of the compound of example 17, with a yield of 2.72%. LC-MS: 718.4[M+H] ⁺. ¹H NMR (400 MHz, D₂O) δ 8.33 (s, 2H), 7.92 (s, 1H), 7.81 (s, 2H), 7.36-7.06 (m, 5H), 5.60-5.52 (m, 2H), 4.59-4.50 (m, 3H), 4.12-3.87 (m, 4H), 3.82-3.42 (m, 3H), 3.40-3.19 (m, 3H), 3.10-3.04 (m, 6H), 2.64-2.51 (m, 1H), 2.46-2.29 (m, 3H), 2.15-2.08 (m, 1H), 1.86-1.71 (m, 2H), 1.66-1.54 (m, 1H), 1.49-1.36 (m, 3H).

### Example 18

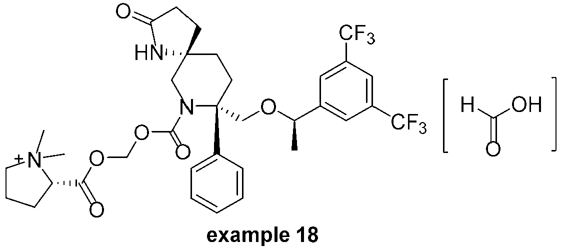

The compound of example 18 was prepared following the method of example 17, by reacting intermediate I with betaine hydrochloride. LC-MS [M+H]⁺: 700.4.

### Example 19

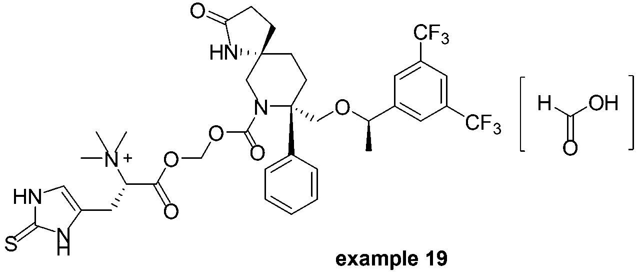

The compound of example 19 was prepared following the method of example 17, by reacting intermediate I with ergothioneine. LC-MS [M+H]⁺: 786.3. ¹H NMR (400 MHz, D₂O) δ 8.09 - 7.73 (m, 3H), 7.41 - 7.07 (m, 6H), 5.47 - 5.28 (m, 3H), 4.01 - 3.96 (m, 1H), 3.65- 3.59 (m, 1H), 3.35 - 2.92 (m, 12H), 2.49 - 2.21 (m, 2H), 1.95 - 1.73 (m, 3H), 1.58 - 1.36 (m, 1H), 1.36 - 1.26 (m, 3H), 1.24 - 1.01 (m, 3H), 0.79 - 0.61 (m, 1H).

### Example 20

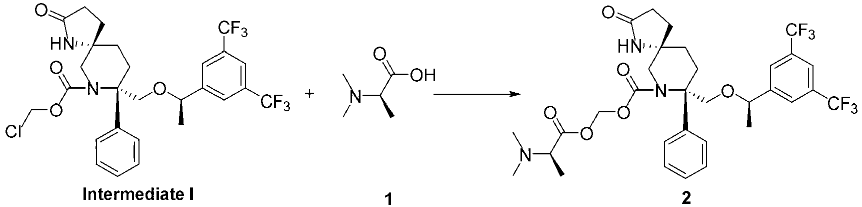

Intermediate I (200 mg, 0.336 mmol) was dissolved in anhydrous DMF (5 mL), then NaI (100.84 mg, 0.672 mmol), KHCO₃ (67.3 mg, 0.672 mmol), and Compound 1 (79.03 mg, 0.672 mmol) were added. The reaction mixture was stirred at room temperature under nitrogen for 18 hours, then quenched with water (30 mL) and extracted three times with ethyl acetate (30 mL). The combined organic phases were washed sequentially with water and brine, dried over Na₂SO₄, filtered, concentrated, and the residue was purified by silica gel column chromatography (petroleum ether/ethyl acetate = 1/1) to give 150 mg of Compound 2, yield 66.02%. LC-MS: 674.2 [M+H]⁺.

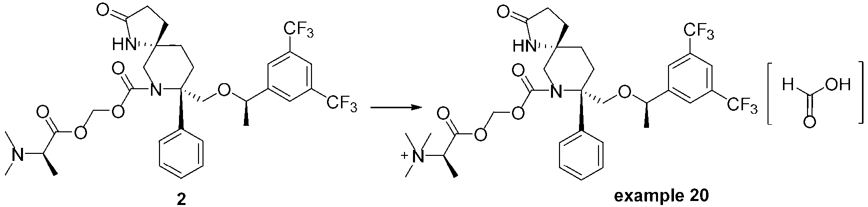

Compound 2 (150 mg, 0.223 mmol) was dissolved in anhydrous toluene (5 mL), and methyl iodide (630.21 mg, 4.44 mmol) was added. The reaction mixture was stirred at 50°C under nitrogen for 16 hours. The solvent was removed under reduced pressure, and the residue was purified by preparative HPLC (Gemini-C18 150 x 21.2 mm, 5 µm, ACN-H₂O, 0.1% FA) to afford 30 mg of the compound of example 20, yield 19.56%. LC-MS: 688.3[M+H]⁺. ¹H NMR (400 MHz, D₂O) δ 7.67-7.44 (m, 3H), 7.24-6.80 (m, 5H), 5.72-5.45 (m, 1H), 5.20-4.83 (m, 1H), 4.38-4.05 (m, 2H), 4.00-3.60 (m, 3H), 3.56-3.38 (m, 2H), 3.23-2.88 (m, 6H), 2.82-2.43 (m, 2H), 2.40-1.84 (m, 4H), 1.75 - 1.14 (m, 7H), 1.12-0.61 (m, 3H).

### Example 21

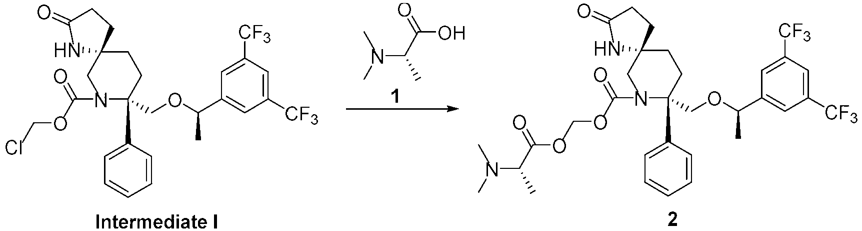

Intermediate I (150 mg, 0.25 mmol), compound 1 (59 mg, 0.50 mmol), and NaI (75.6 mg, 0.50 mmol) were dissolved in DMF (2 mL), then KHCO₃ (50.5 mg, 0.50 mmol) was added. The reaction mixture was stirred at room temperature for 16 hours. Upon completion, the reaction mixture was poured into water, separated, and extracted with ethyl acetate. The combined organic phases were dried over Na₂SO₄, filtered, concentrated, and the residue was purified by column chromatography (petroleum ether/ethyl acetate = 1/3) to give 80 mg of Compound 2, yield 46.9%. LC-MS [M+H]⁺: 674.3.

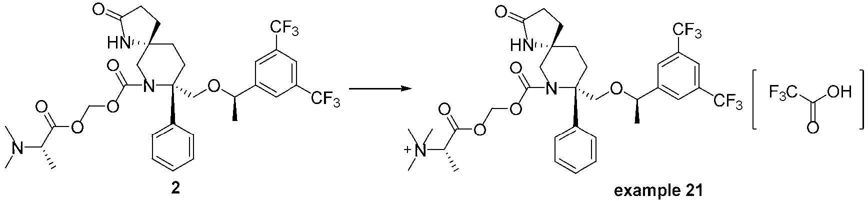

Compound 2 (80 mg, 0.118 mmol, 1 eq) was dissolved in toluene (2 mL), and methyl iodide (84 mg, 0.592 mmol, 5 eq) was added. The reaction mixture was heated to 50°C and stirred for 16 hours. After completion, the reaction mixture was concentrated to dryness, and the residue was purified by preparative HPLC to afford 11 mg of the compound of example 21, yield 13.4%. LC-MS [M+H]⁺: 688.3. ¹H NMR (400 MHz, D₂O) δ 8.01 - 7.73 (m, 3H), 7.41 - 7.07 (m, 5H), 5.38 - 5.11 (m, 2H), 4.57 - 4.41 (m, 1H), 4.25 - 4.04 (m, 2H), 4.01 - 3.85 (m, 1H), 3.77 - 3.58 (m, 3H), 3.54 - 3.34 (m, 2H), 3.25 - 2.91 (m, 4H), 2.71 - 2.63 (m, 1H), 2.55 - 2.48 (m, 1H), 2.45 - 2.15 (m, 4H), 1.99 - 1.64 (m, 4H), 1.61 - 1.52 (m, 1H), 1.49 - 1.32 (m, 3H), 1.24 - 0.98 (m, 2H).

### Example 22

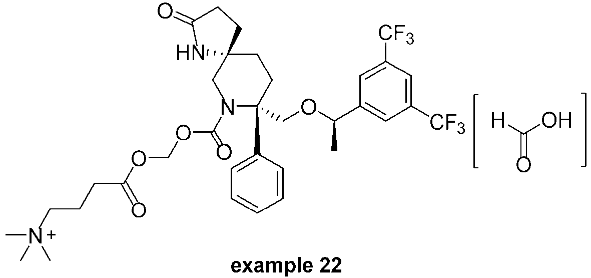

Following the procedure of example 20, 4-(dimethylamino)butyric acid was used instead of N,N-dimethyl-D-alanine to afford the compound of example 22. LC-MS [M+H]⁺: 701.7. ¹H NMR (400 MHz, D₂O) δ 8.38 (s, 1H), 8.00 - 7.87 (m, 3H), 7.37 - 7.26 (m, 5H), 5.21 - 5.10 (m, 2H), 4.58 - 4.52 (m, 1H), 4.26 - 4.11 (m, 4H), 3.63 (s, 3H), 3.33 - 3.22 (m, 2H), 3.08 - 2.99 (m, 3H), 2.59 - 1.71 (m, 15H), 1.47 (s, 3H).

### Example 23

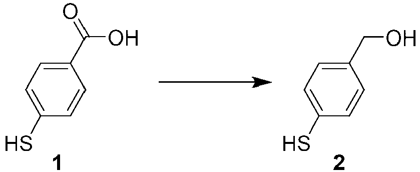

Under nitrogen protection, a solution of 4-mercaptobenzoic acid (2.5 g, 16.2 mmol) in tetrahydrofuran (30 mL) was slowly added to a suspension of lithium aluminum hydride (1.84 g, 48.6 mmol) in tetrahydrofuran (30 mL), and the mixture was stirred for 3 hours after the addition. To the reaction mixture was added 1 N hydrochloric acid (500 mL), and the resulting solution was extracted with ethyl acetate (50 mL) three times. The combined organic phases were washed with saturated brine (50 mL), dried over anhydrous sodium sulfate, filtered, concentrated, and the residue was purified by silica gel column chromatography (petroleum ether/ethyl acetate = 4/1) to give 2.2 g of Compound2, yield 97.04%. LC-MS [M+H]⁺: 141.2.

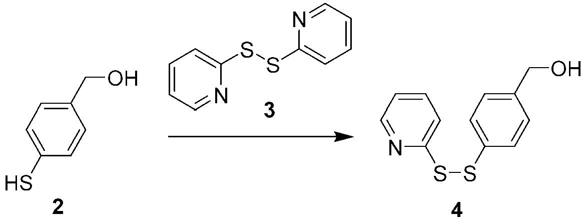

At room temperature, Compound 2 (2.2 g, 15.7 mmol) in dichloromethane (50 mL) was combined with Compound 3 (6.92 g, 31.4 mmol), and the mixture was stirred under nitrogen at 25°C for 16 hours. After completion, the solvent was removed under reduced pressure, and the residue was purified by silica gel column chromatography (petroleum ether/ethyl acetate = 1/1) to give 2.0 g of Compound 4, yield 45.86%. LC-MS [M+H]⁺: 250.1.

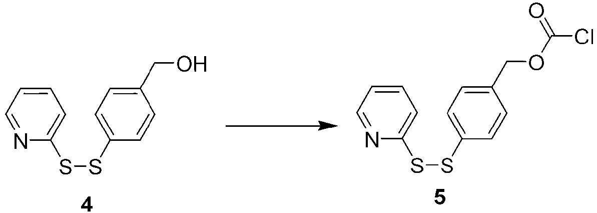

Under ice-bath conditions, Compound 4 (400 mg, 1.60 mmol) and N,N-diisopropylethylamine (207 mg, 1.60 mmol) were dissolved in dichloromethane (10 mL) under nitrogen, and triphosgene (476 mg, 1.60 mmol) was added slowly. The reaction mixture was stirred at 0°C for 0.5 hours, then warmed to 25°C and stirred for 2 hours. The reaction was quenched with ice water (10 mL), and the aqueous phase was extracted with ethyl acetate (20 mL) three times. The combined organic layers were washed with saturated brine (20 mL), dried over anhydrous sodium sulfate, filtered, concentrated, and the crude Compound 5 was used directly in the next step. LC-MS [M+H]⁺: 312.0.

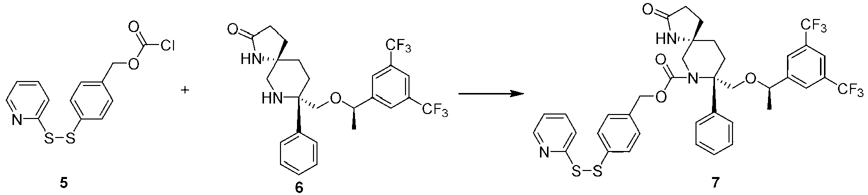

Under ice-bath conditions, Compound 6 (200 mg, 0.39 mmol) and anhydrous potassium carbonate (440 mg, 3.18 mmol) were dissolved in anhydrous acetonitrile (10 mL) under nitrogen. A solution of Compound 5 (372 mg, 1.19 mmol) in ethyl acetate (5 mL) was added slowly, and the mixture was stirred at 50°C for 16 hours. Ice water (10 mL) was added, and the solution was extracted with ethyl acetate (20 mL) three times. The combined organic layers were washed with saturated brine (20 mL), dried over anhydrous sodium sulfate, filtered, concentrated, and the residue was purified by silica gel column chromatography (dichloromethane/methanol = 100/2) to give 50 mg of Compound 7, yield 12.9%. LC-MS [M+H]⁺: 776.1.

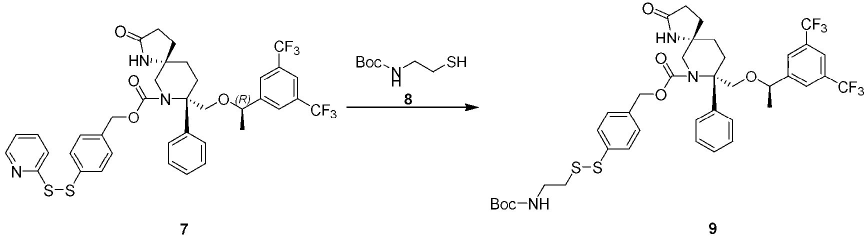

At room temperature, Compound 7 (30 mg, 0.04 mmol) and Compound 8 (13 mg, 0.08 mmol) were dissolved in anhydrous dichloromethane (2 mL) under nitrogen and stirred at 25°C for 16 hours. The solvent was removed under reduced pressure, and the crude Compound 9 was used directly in the next step. LC-MS [M+H]⁺: 842.2.

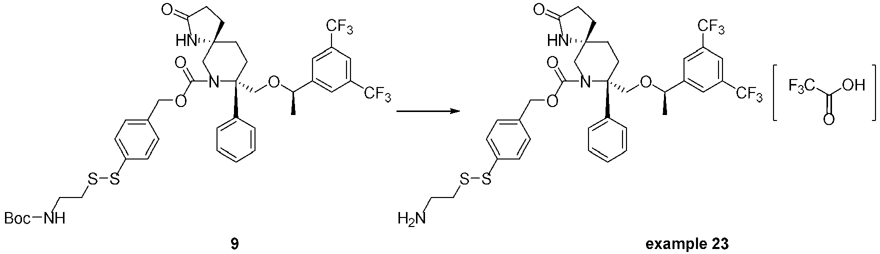

Under ice-bath conditions, Compound 9 (30 mg, 0.03 mmol) was dissolved in anhydrous dichloromethane (2 mL), and trifluoroacetic acid (0.5 mL) was added slowly under nitrogen. The reaction mixture was stirred at 25°C for 30 minutes. The solvent was removed under reduced pressure, and the residue was purified by preparative HPLC (A solution: 0.1 % TFA, B solution: acetonitrile) to give 30 mg of the compound of example 23, yield 14.65%. LC-MS [M+H]⁺: 742.1. ¹H NMR (400 MHz, DMSO-*d₆*) δ 8.14 (s, 1H), 8.00 (s, 1H), 7.94 (s, 2H), 7.84 (s, 2H), 7.42 (s, 2H), 7.32 - 7.28 (m, 2H), 7.24 - 7.19 (m, 3H), 4.89-4.83 (m, 2H), 4.64 (q, J = 6.3 Hz, 1H), 4.10 (d, J = 8.9 Hz, 1H), 4.00 - 3.98 (m, 1H), 3.54 (s, 2H), 3.10 - 3.07 (m, 2H), 2.95 - 2.91 (m, 2H), 2.27 - 2.07 (m, 2H), 2.08 - 1.94 (m, 2H), 1.92 - 1.82 (m, 1H), 1.78 - 1.59 (m, 2H), 1.47 - 1.39 (m, 1H), 1.40 (d, J = 6.4 Hz, 3H), 1.23 (s, 2H).

### Example 24

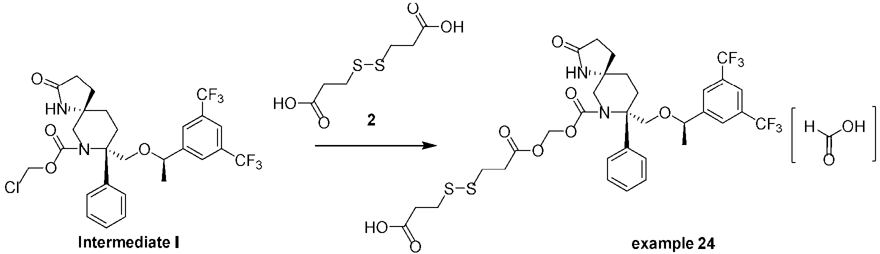

Under nitrogen protection, Intermediate I (50.0 mg, 0.08 mmol) was dissolved in N,N-dimethylformamide (1 mL) in a 50 mL three-necked flask, followed by the addition of Compound 2 (53.0 mg, 0.30 mmol), sodium iodide (25.2 mg, 0.17 mmol) and potassium bicarbonate (16.8 mg, 0.17 mmol), and the reaction mixture was stirred at room temperature for 16 hours. The reaction mixture was purified by preparative HPLC (solution A: 0.1% formic acid, solution B: acetonitrile) to afford 1.99 mg the compound of example 24, yield 2.98%. LC-MS [M+H]⁺: 766.5. ¹H NMR (400 MHz, DMSO-*d₆*) δ 8.11 (s, 1H), 8.01 (s, 1H), 7.96 (s, 2H), 7.32 (m, 2H), 7.25-7.20 (m, 3H), 5.55 (s, 2H), 4.66 (d, *J* = 5.7 Hz, 1H), 4.07 (d, *J* = 9.1 Hz, 1H), 4.01-3.99 (m, 1H), 3.51 (d, *J =* 12.6 Hz, 1H), 3.34 (s, 1H), 2.86-2.84 (m, 4H), 2.59 (t, *J =* 6.9 Hz, 2H), 2.52-2.50 (m, 2H), 2.23 - 2.11 (m, 2H), 2.02-1.95 (m, 2H), 1.70 - 1.60 (m, 2H), 1.42 (s, 1H), 1.40-1.38 (m, 4H).

### Example 25

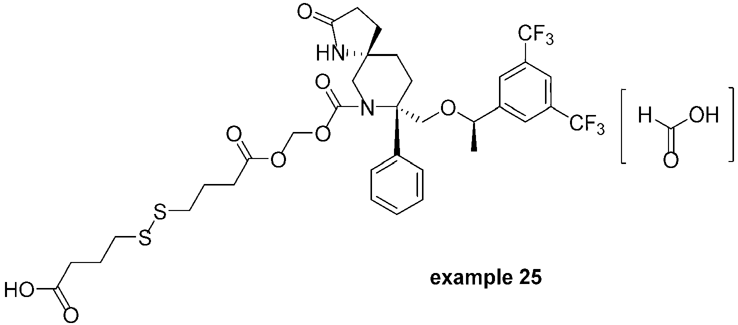

According to the method of example 24, using 4,4'-dithiodibutyric acid instead of 3,3'-dithiodipropionic acid, the compound of example was obtained. LC-MS [M+H]⁺: 794.5. ¹H NMR (400 MHz, DMSO-*d₆*) δ 12.09 (s, 1H), 8.12 (s, 1H), 8.02 (s, 1H), 7.96 (s, 2H), 7.34-7.30 (m, 2H), 7.25-7.18 (m, 3H), 5.52 (s, 2H), 4.59-4.66 (m, 1H), 4.08 (d, J = 8.9 Hz, 1H), 4.01 - 3.95 (m, 1H), 3.50-3.45 (m, 2H), 2.71 - 2.66 (m, 4H), 2.47 - 2.42 (m, 2H), 2.33 - 2.29 (m, 4H), 2.19 - 2.15 (m, 2H), 2.04 - 1.95 (m, 2H), 1.87 - 1.80 (m, 4H), 1.68 - 1.63 (m, 2H), 1.41 - 1.40 (d, J = 6.3 Hz, 3H).

### Example 26

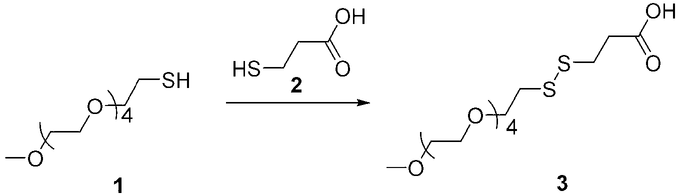

Compound 1 (100 mg, 0.37 mmol), Compound 2 (40 mg, 0.37 mmol), Copper chloride (5 mg, 0.037 mmol) and N,N,N',N'-Tetramethylethylenediamine (173 mg, 1.49 mmol) were dissolved in Methanol (3 mL) and stirred at room temperature under nitrogen for 3 hours. The solid was filtered off. The filtrate was quenched with Water (50 mL) and extracted with ethyl acetate (50 mL) three times. The combined organic phase was washed sequentially with water and saturated sodium chloride solution, dried over anhydrous Sodium sulfate, filtered, and concentrated to give 60 mg Compound 3, yield 43.24%. LC-MS: 373.1 [M+H]⁺.

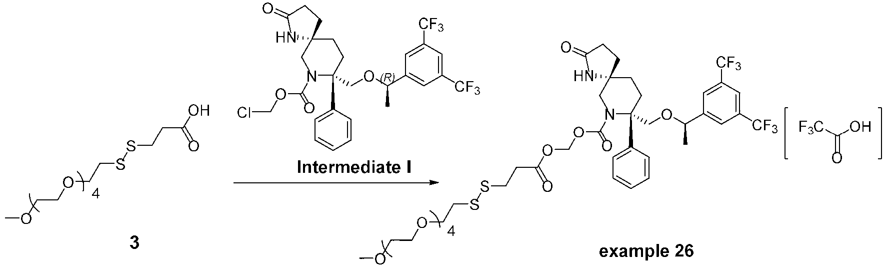

Intermediate I (60 mg, 0.10 mmol), Compound 3 (75 mg, 0.20 mmol), sodium iodide (30 mg, 0.20 mmol) and potassium bicarbonate (20 mg, 0.20 mmol) were dissolved in N,N-Dimethylformamide (3 mL) and stirred at room temperature under nitrogen for 16 hours. The reaction was quenched with water (50 mL) and extracted with ethyl acetate (50 mL) three times. The combined organic phase was washed sequentially with water and saturated sodium chloride solution, dried over anhydrous sodium sulfate, filtered, concentrated, and the residue was purified by preparative HPLC (Gemini-C18 150 x 21.2 mm, 5 µm, ACN-H₂O, 0.1% TFA) to give 10 mg the compound of example 26, yield 10.62%. LC-MS: 928.9 [M+H]⁺.

### Example 27

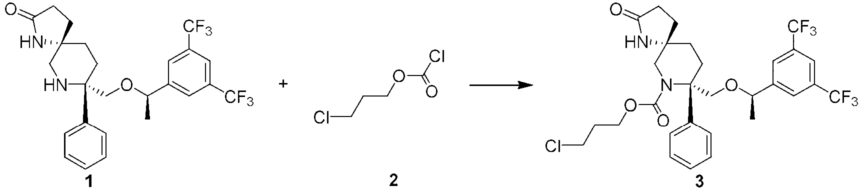

Under nitrogen protection, Compound 1 (500 mg, 1.0 mmol) was placed in a 25 mL three-necked flask, and 10 mL of anhydrous dichloromethane was added. The system was purged with N2 three times, and diisopropylethylamine (1032 mg, 8 mmol) was added. The mixture was cooled to -30°C, followed by addition of trimethylsilyl chloride (283 mg, 2.6 mmol), and stirred at room temperature for 2 hours. The mixture was then cooled again to -30°C, and a solution of Compound 2 (175 mg, 1.23 mmol) in 2 mL of anhydrous dichloromethane was added dropwise. The reaction temperature was maintained between -20°C and -5°C until completion. The reaction mixture was poured into 10 mL of ice water and extracted with dichloromethane (10 mL) three times. The combined organic phases were dried over anhydrous sodium sulfate, filtered, concentrated, and the residue was purified by silica gel column chromatography (dichloromethane/methanol = 20/1) to afford 300 mg of Compound 3, with a yield of 62%. LC-MS: 620.6 [M+H]⁺.

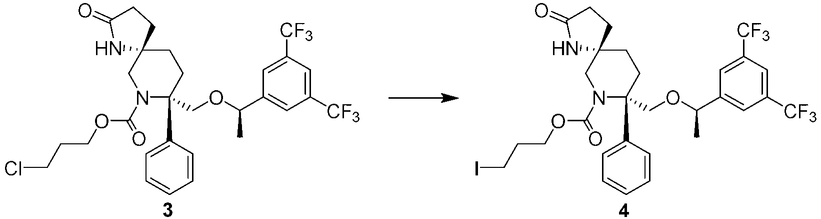

Compound 3 (200 mg, 0.322 mmol) and sodium iodide (484 mg, 3.22 mmol) were placed in a 25 mL three-necked flask, 5 mL of anhydrous acetonitrile was added, and the system was purged with N2 three times. The mixture was stirred at 80°C for 2 days. The reaction mixture was concentrated to dryness, and the residue was purified by silica gel column chromatography (dichloromethane/methanol = 20/1) to afford 120 mg of Compound 4, with a yield of 52%. LC-MS: 712.5 [M+H]⁺.

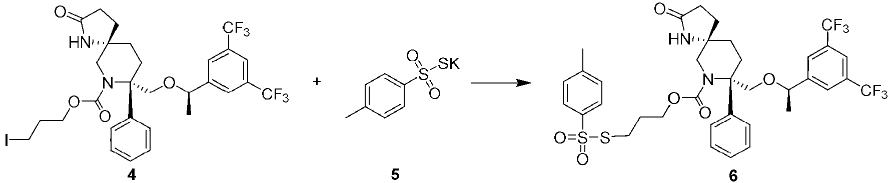

Compound 4 (120 mg, 0.1685 mmol) and Compound 5 (76 mg, 0.3371 mmol) were placed in a 25 mL three-necked flask, and 5 mL of anhydrous acetonitrile was added. The system was purged with N2 three times, and the mixture was stirred at 100°C for 3 hours, then at room temperature overnight. The reaction mixture was concentrated to dryness, diluted with 10 mL of water, and extracted with dichloromethane (10 mL) three times. The combined organic phases were dried over anhydrous sodium sulfate, filtered, concentrated, affording 70 mg of Compound 6, with a yield of 52%. LC-MS: 772.5 [M+H]⁺.

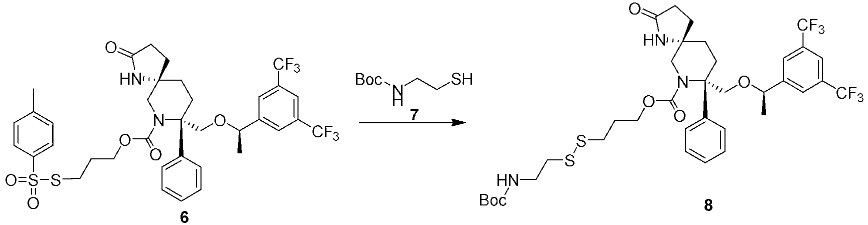

Compound 6 (70 mg, 0.09 mmol) and triethylamine (18 mg, 0.18 mmol) were placed in a 25 mL three-necked flask, and 4 mL of anhydrous dichloromethane was added. The system was purged with N2 three times. The mixture was cooled to -40°C, and a solution of Compound 7 (15.8 mg, 0.09 mmol, 1 eq) in 1 mL of dichloromethane was added dropwise. After addition, the reaction was maintained at -40°C for 1 hour. The reaction mixture was poured into 10 mL of ice water and extracted with dichloromethane (10 mL) three times. The combined organic phases were dried over anhydrous sodium sulfate, filtered, concentrated, affording 10 mg of Compound 8, with a yield of 14%. LC-MS: 794.1 [M+H]⁺.

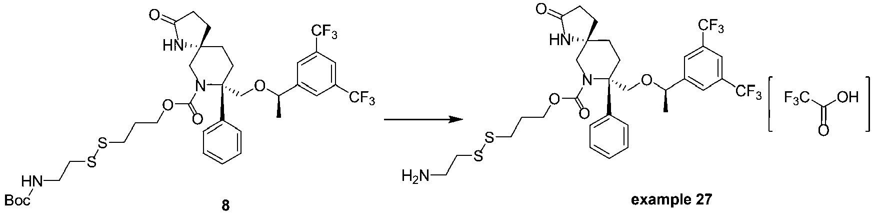

At 0°C, Compound 8 (10 mg, 0.0126 mmol) was dissolved in a mixture of 2 mL dichloromethane and 0.5 mL trifluoroacetic acid, and stirred for 0.5 hours. The reaction mixture was concentrated to dryness, and the crude product was purified by C18 column chromatography (ACN-H₂O, 0.1% TFA, gradient 30-60) to afford 2.32 mg of the compound of example 27, with a yield of 27%. LC-MS: 693.6[M+H] ⁺. ¹H NMR (400 MHz, DMSO-*d₆*): δ 8.12 (s, 1H), 8.03 (s, 1H), 7.96 (s, 2H), 7.75 (s, 3H), 7.35 - 7.28 (m, 2H), 7.21 (t, J = 7.6 Hz, 3H), 4.72-4.68 (m, 1H), 4.10 (d, J = 8.8 Hz, 2H), 3.98 (s, 2H), 3.83 (s, 2H), 3.44 (d, J = 12.7 Hz, 2H), 3.04-3.02 (m, 2H), 2.78-2.77 (m, 2H), 2.30 - 2.05 (m, 5H), 1.73-1.64 (m, 4H), 1.47-1.42 (m, 4H).

### Example 28

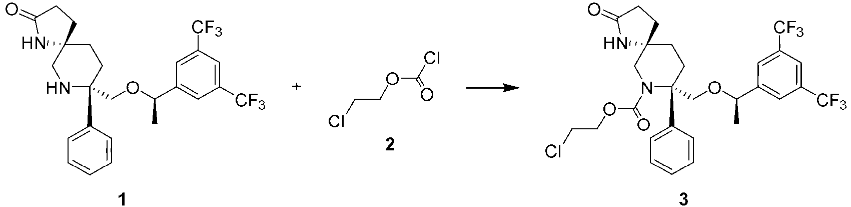

Under nitrogen protection, Compound 1 (200 mg, 0.4 mmol) was placed in a 25 mL three-necked flask, and 5 mL of anhydrous dichloromethane was added. The system was purged with N2 three times, and diisopropylethylamine (413 mg, 3.2 mmol) was added. The mixture was cooled to -30°C, followed by addition of trimethylsilyl chloride (113 mg, 1.04 mmol), and stirred at room temperature for 2 hours. The mixture was then cooled again to -30°C, and a solution of Compound 2 (70 mg, 0.492 mmol) in 2 mL of anhydrous dichloromethane was added dropwise. The reaction temperature was maintained between -20°C and -5°C until completion. The reaction mixture was poured into 10 mL of ice water and extracted with dichloromethane (10 mL) three times. The combined organic phases were dried over anhydrous sodium sulfate, filtered, concentrated, and the residue was purified by silica gel column chromatography (dichloromethane/methanol = 20/1) to afford 150 mg of Compound 3, with a yield of 62%. LC-MS: 606.7 [M+H]⁺.

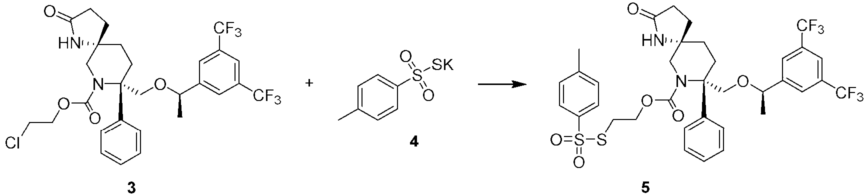

Compound 3 (120 mg, 0.198 mmol) and Compound 4 (90 mg, 0.396 mmol) were placed in a 5 mL microwave tube, and 3 mL of anhydrous acetonitrile was added. The system was purged with N₂ three times, and the mixture was stirred at 100°C for 6 hours. The reaction mixture was concentrated to dryness, diluted with 10 mL of water, and extracted with dichloromethane (10 mL) three times. The combined organic phases were dried over anhydrous sodium sulfate, filtered, concentrated, affording 80 mg of Compound 5, with a yield of 53%. LC-MS: 758.6 [M+H]⁺.

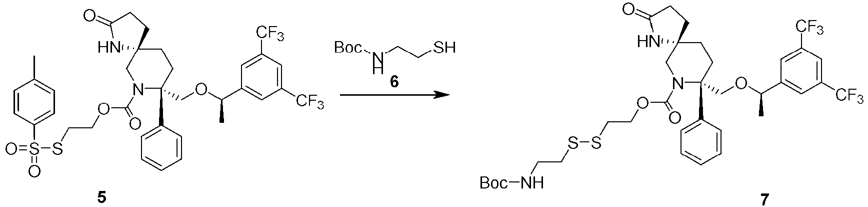

Compound 5 (40 mg, 0.0528 mmol) and triethylamine (10.5 mg, 0.1055 mmol) were placed in a 25 mL three-necked flask, and 4 mL of anhydrous dichloromethane was added. The system was purged with N₂ three times. The mixture was cooled to - 40°C, and a solution of Compound 6 (9.3 mg, 0.0528 mmol) in 1 mL of dichloromethane was added dropwise. After addition, the reaction was maintained at - 40°C for 1 hour. The reaction mixture was poured into 10 mL of ice water and extracted with dichloromethane (10 mL) three times. The combined organic phases were dried over anhydrous sodium sulfate, filtered, concentrated, affording 12 mg of Compound 7, with a yield of 29%. LC-MS: 779.6 [M+H]⁺.

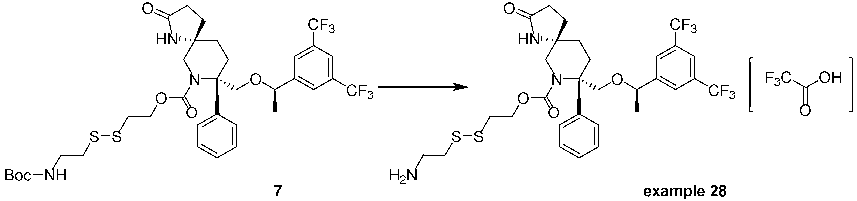

At 0°C, Compound 7 (12 mg, 0.0154 mmol) was dissolved in a mixture of 2 mL dichloromethane and 0.5 mL trifluoroacetic acid and stirred for 0.5 hours. The reaction mixture was concentrated to dryness, and the residue was purified by C18 column chromatography (ACN-H₂O, 0.1% TFA, gradient 30-70) to afford 5.3 mg of the compound of example 28, with a yield of 50%. LC-MS: 679.6 [M+H] ⁺. ¹H NMR (400 MHz, D₂O): δ 8.08 (s, 1H), 7.96 (s, 2H), 7.50 - 7.23 (m, 5H), 4.72-4.69 (m, 1H), 4.20-4.08 (m, 3H), 3.77 (s, 1H), 3.57 (s, 1H), 3.29 (s, 3H), 2.87 (s, 3H), 2.52-2.49 (m, 4H), 2.20 (s, 1H), 2.05-1.94 (m, 3H), 1.75-1.68 (m, 1H), 1.54 (s, 3H).

### Example 29

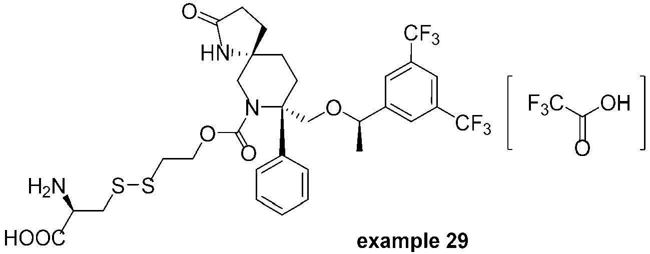

Following the procedure of the third and fourth steps in example 28, N-tert-butoxycarbonyl-L-cysteine was used instead of 2-tert-butoxycarbonylaminoethanethiol to synthesize and obtain example 29 compound. LC-MS [M+H]⁺: 723.8. ¹H NMR (400 MHz, DMSO-*d₆*): δ 8.13 (s, 1H), 8.02 (s, 1H), 7.96 (s, 2H), 7.35 - 7.28 (m, 2H), 7.25 - 7.19 (m, 3H), 4.66 (q, J = 6.2 Hz, 1H), 4.17 - 3.92 (m, 5H), 3.49 (s, 2H), 3.21 (d, J = 14.5 Hz, 2H), 3.05-2.99 (m, 1H), 2.80 (br, 1H), 2.45 (s, 1H), 2.30-2.23 (m, 1H), 2.17-2.11 (m, 1H), 2.08 - 2.03 (m, 1H), 1.87 (d, J = 14.0 Hz, 1H), 1.75 - 1.60 (m, 2H), 1.45-1.39 (m, 4H).

### Example 30

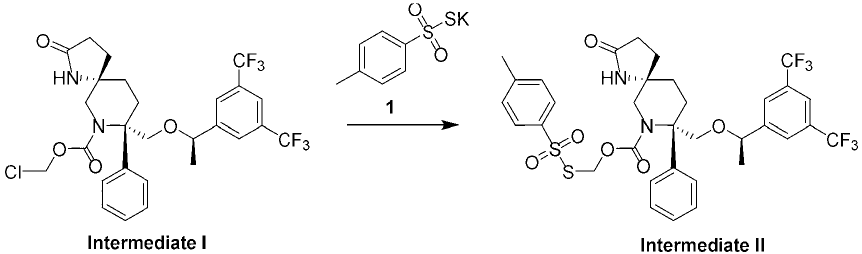

Intermediate I (100 mg, 0.1686 mmol) and compound 1 (76 mg, 0.3372 mmol) were placed in a 25 mL three-necked flask, to which anhydrous acetonitrile (5 mL) was added. The system was purged with N2 three times and stirred at 100°C for 3 h, followed by stirring at room temperature overnight. The reaction mixture was concentrated, and the residue was diluted with water (10 mL) and extracted with dichloromethane (10 mL) three times. The combined organic layers were dried over anhydrous sodium sulfate, filtered, and concentrated to give 40 mg of Intermediate II, yield 32%. LC-MS: 744.5 [M+H]⁺.

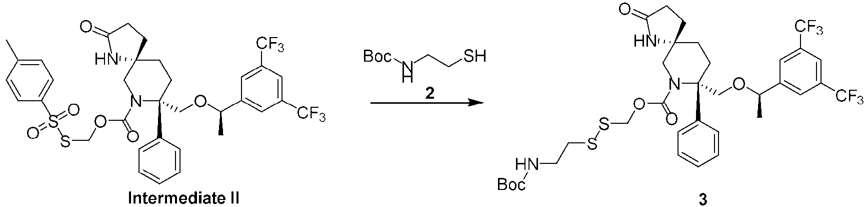

Intermediate II (40 mg, 0.05376 mmol) and triethylamine (10.75 mg, 0.1075 mmol) were placed in a 25 mL three-necked flask, and anhydrous dichloromethane (4 mL) was added. The system was purged with N₂ three times and cooled to -40°C. A solution of compound 2 (9.5 mg, 0.05376 mmol) in dichloromethane (1 mL) was added dropwise. After addition, the reaction was maintained at -40°C for 1 h. The reaction mixture was poured into ice water (10 mL) and extracted with dichloromethane (10 mL) three times. The combined organic layers were dried over anhydrous sodium sulfate, filtered, and concentrated to afford 26 mg of white solid Compound 3, yield 50.5%. LC-MS: 788.1 [M+Na]⁺.

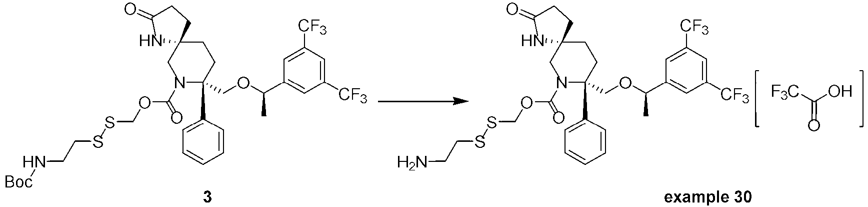

At 0°C, Compound 3 (26 mg, 0.02715 mmol) was dissolved in a mixture of dichloromethane (2 mL) and trifluoroacetic acid (0.5 mL) and stirred for 1 h. The reaction mixture was concentrated to dryness, and the residue was purified by C18 column chromatography (ACN-H₂O, 0.1% TFA, gradient: 25-60) to afford 10 mg of example 30 compound, yield 55%. LC-MS [M+H]⁺: 665.7. ¹H NMR (400 MHz, MeOD): δ 7.92 (s, 2H), 7.89 (s, 1H), 7.42 - 7.26 (m, 5H), 5.35-5.30 (m, 2H), 4.64-4.59 (m, 1H), 4.22 (d, J = 9.3 Hz, 1H), 4.04 (d, J = 9.0 Hz, 1H), 3.86 (d, J = 12.6 Hz, 1H), 3.25-3.21 (m, 3H), 2.97 (s, 2H), 2.55 - 2.36 (m, 3H), 2.31-2.25 (m, 1H), 2.12-2.05 (m, 1H), 1.91-1.85 (m, 2H), 1.75-1.67 (m, 1H), 1.46 (d, J = 6.4 Hz, 3H).

### Example 31

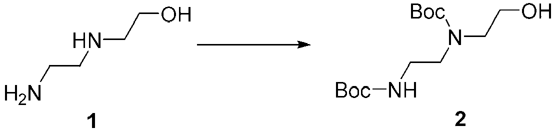

Compound 1 (2 g, 19.2 mmol) was dissolved in dichloromethane (30 mL), and ditert-butyl dicarbonate (10.48 g, 48 mmol) was added. The resulting solution was stirred at room temperature for 16 h. The reaction was quenched with water (100 mL) and extracted three times with ethyl acetate (100 mL). The combined organic layers were washed sequentially with water and saturated brine, dried over sodium sulfate, filtered, and concentrated to give 2.2 g of Compound 2, yield 37.50%. LC-MS: 305.3 [M+H]⁺.

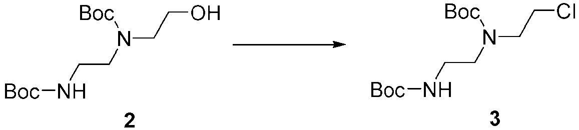

N-chlorosuccinimide (240 mg, 1.8 mmol) was dissolved in anhydrous tetrahydrofuran (5 mL), and triphenylphosphine (471 mg, 1.8 mmol) was added. The resulting solution was stirred at room temperature for 20 min. Under nitrogen protection, at 0°C, a solution of compound 2 (500 mg, 1.63 mmol) was added in portions, and the mixture was stirred for 11 h. The solvent was removed under reduced pressure, and the residue was purified by silica gel column chromatography (dichloromethane/methanol = 60:40) to afford 200 mg of compound 3, yield 37.73%. LC-MS: 323.1 [M+H]⁺.

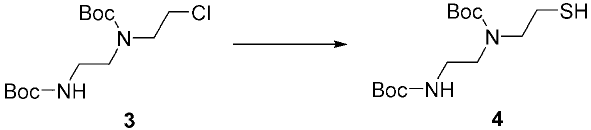

Compound 3 (100 mg, 0.31 mmol) was dissolved in anhydrous dimethylformamide (3 mL), and potassium thioacetate (47 mg, 0.42 mmol) was added under nitrogen. The mixture was heated to 90°C and stirred for 12 h. Water (20 mL) was added, and the solution was extracted with ethyl acetate (25 mL). The organic layer was washed with saturated brine (25 mL), dried over anhydrous sodium sulfate, filtered, and concentrated. The residue was dissolved in methanol (5 mL) under nitrogen at room temperature, potassium carbonate (128 mg, 0.92 mmol) was added, and the solution was stirred for 1 h. The pH was adjusted to 8-9 with 1 M dilute hydrochloric acid. Water (25 mL) was added, and the solution was extracted with dichloromethane (25 mL). The organic layer was washed twice with saturated brine (25 mL), dried over anhydrous sodium sulfate, filtered, and concentrated. The residue was purified by silica gel column chromatography (petroleum ether/ethyl acetate = 60:40) to give 60 mg of white solid Compound 4, yield 60.46%. LC-MS: 321.2 [M+H]⁺.

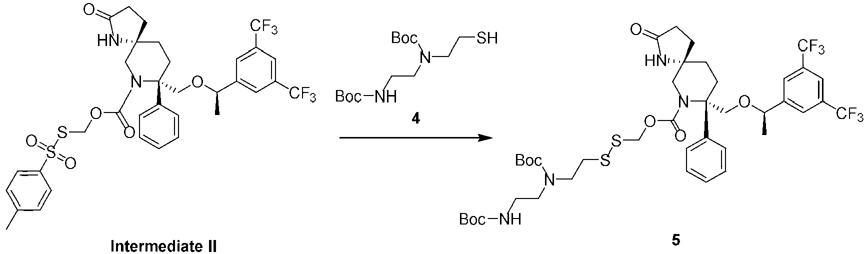

At -40°C, Intermediate II (60 mg, 0.08 mmol) was dissolved in anhydrous dichloromethane (3 mL), and triethylamine (16 mg, 0.16 mmol) and compound 4 (31 mg, 0.096 mmol) were added. The solution was stirred under nitrogen at -40°C for 30 min. The reaction was quenched with water (50 mL) and extracted three times with ethyl acetate (50 mL). The combined organic layers were washed sequentially with water and saturated brine, dried over anhydrous sodium sulfate, filtered, and concentrated to give 50 mg of Compound 5, yield 68.24%. LC-MS: 931.3 [M+Na]⁺.

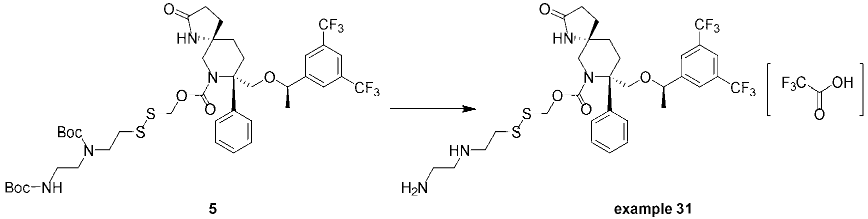

Compound 5 (50 mg, 0.055 mmol) was dissolved in anhydrous dichloromethane (5 mL) in a 50 mL single-necked flask, and trifluoroacetic acid (1 mL) was added. The mixture was stirred at room temperature for 2 h, then concentrated, and the residue was purified by preparative HPLC (Gemini-C18 150 x 21.2 mm, 5 µm, ACN-H₂O, 0.1% TFA) to afford 1.5 mg of example 31 compound, yield 3.85%. LC-MS:708.6[M+H]⁺ . ¹H NMR (400 MHz, DMSO-*d₆*) δ 8.87 (s, 3H), 8.18 (s, 1H), 8.02 (s, 1H), 7.96 (s, 2H), 7.36 - 7.29 (m, 2H), 7.28 - 7.19 (m, 3H), 5.25-5.15 (m, 2H), 4.65 (d, J = 6.1 Hz, 1H), 4.05 (dd, J = 24.7, 8.8 Hz, 2H), 3.56 (d, J = 13.0 Hz, 2H), 3.31 - 3.04 (m, 7H), 2.98 (s, 2H), 2.30 - 2.15 (m, 2H), ), 2.05-1.99 (m, 2H), 1.73-1.66 (m, 2H), 1.45-1.39 (m, 4H).

### Example 32

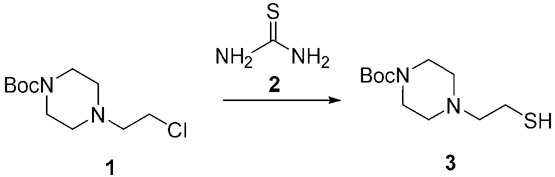

Compound 1 (2 g, 8.04 mmol) and Compound 2 (1.22 g, 16.08 mmol) were placed in a 50 mL three-necked flask and dissolved in methanol (20 mL). The reaction mixture was stirred at 50°C for 2 h, then an aqueous solution of sodium hydroxide (487 mg, 6.695 mmol in 5 mL water) was added, and the mixture was stirred at 50°C for 1 h. The reaction mixture was concentrated, and the residue was diluted with water (30 mL) and extracted three times with ethyl acetate (30 mL). The combined organic layers were washed with saturated brine, dried over anhydrous sodium sulfate, filtered, and concentrated to afford 1.5 g of Compound 3, yield 87.80%. LC-MS: 246.9 [M+H]⁺.

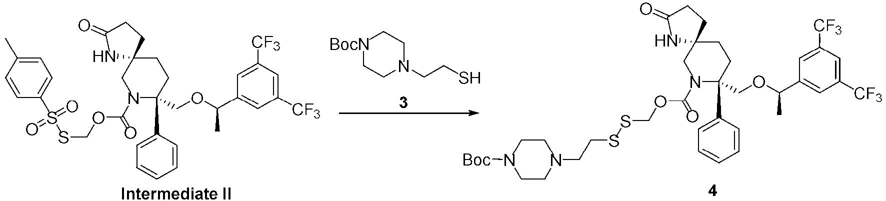

Intermediate II (200 mg, 0.269 mmol) was dissolved in anhydrous dichloromethane (8 mL) in a 50 mL three-necked flask. Triethylamine (52.24 mg, 0.538 mmol) and compound 3 (132.32 mg, 0.538 mmol) were added at -40°C under nitrogen. The mixture was stirred at this temperature for 0.5 h, then quenched with water (50 mL) and extracted three times with ethyl acetate (50 mL). The combined organic layers were washed sequentially with water and saturated brine, dried over anhydrous sodium sulfate, filtered, and concentrated to give 200 mg of Compound 4, yield 89.20%. LC-MS: 835.3 [M+H]⁺.

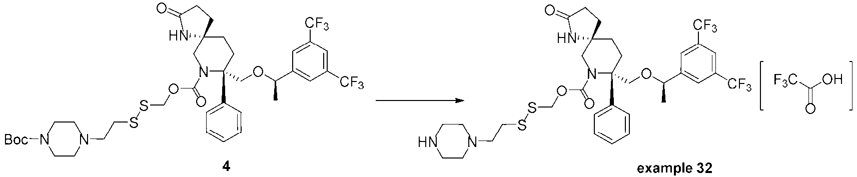

Compound 4 (200 mg, 0.269 mmol) was dissolved in dichloromethane (4 mL) in a 50 mL three-necked flask, and trifluoroacetic acid (1 mL) was added at room temperature. The mixture was stirred at room temperature for 1 h, then concentrated to dryness. The residue was purified by preparative HPLC (Gemini-C18 150 x 21.2 mm, 5 µm, ACN-H₂O, 0.1% TFA) to afford 29.12 mg of example 32 compound, yield 16.54%. LC-MS: 734.6[M+H]⁺. ¹H NMR (400 MHz, DMSO-*d₆*) δ 8.14 (s, 1H), 8.01 (s, 1H), 7.96 (s, 2H), 7.34-7.31 (t, 2H), 7.27 - 7.21 (m, 3H), 5.22-5.12 (m, 2H), 4.69-4.64 (m, 1H), 4.09-4.02 (m, 3H), 3.55-3.45 (m, 2H), 3.12 (s, 4H), 2.95 - 2.64 (m, 9H), 2.33 - 2.15 (m, 2H), 2.07 - 1.98 (m, 1H), 1.94-1.90 (m, 1H), 1.75-1.65 (m, 2H), 1.45-1.40 (m, 4H).

### Example 33

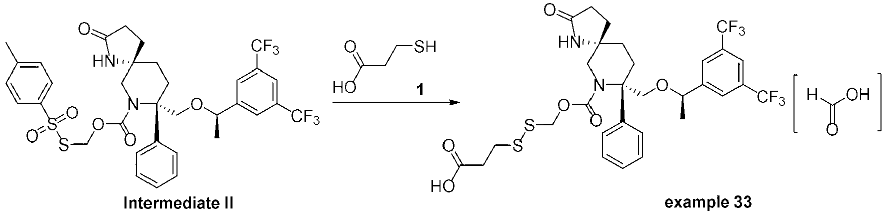

Intermediate II (80 mg, 0.107 mmol) was dissolved in anhydrous dichloromethane (5 mL) in a 50 mL three-necked flask. Triethylamine (21.67 mg, 0.214 mmol) and Compound 1 (56.84 mg, 0.536 mmol) were added at -40°C under a nitrogen atmosphere, and the reaction mixture was stirred at this temperature for 30 min. The reaction was then quenched with water (50 mL) and extracted three times with ethyl acetate (50 mL). The combined organic layers were washed sequentially with water and saturated brine, dried over anhydrous sodium sulfate, filtered, and concentrated. The residue was purified by preparative HPLC (Gemini-C18 150 × 21.2 mm, 5 µm, ACN-H₂O, 0.1% FA) to afford 25.73 mg of example 33 compound, with a yield of 34.48%. LC-MS: 694.6[M+H]⁺.¹H NMR (400 MHz, DMSO-*d₆*) δ 8.17 (s, 1H), 8.02 (s, 1H), 7.96 (s, 2H), 7.36 - 7.30 (m, 2H), 7.25-7.23 (m, 3H), 5.22-5.08 (m, 2H), 4.67-4.64 (m, 1H), 4.04 (dd, J = 21.3, 8.9 Hz, 2H), 3.57 (d, J = 13.3 Hz, 1H), 3.42-3.39 (m, 2H), 2.89-2.80 (m, 2H), 2.56-2.51 (m, 2H), 2.34 - 2.10 (m, 2H), 2.02-1.98 (m, 2H), 1.74 - 1.62 (m, 2H), 1.44-1.38 (m, 4H).

### Example 34

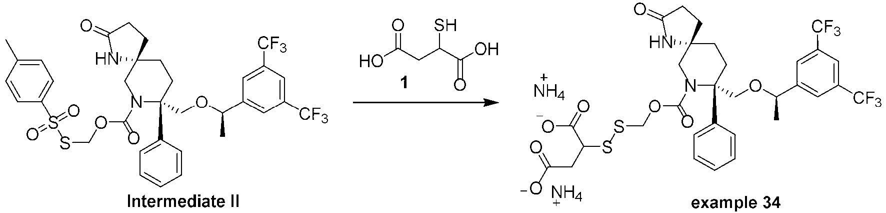

Under a nitrogen atmosphere, Intermediate II (50.0 mg, 0.07 mmol) was weighed into a 50 mL three-necked flask and dissolved in anhydrous tetrahydrofuran (1 mL). Compound 1 (10.1 mg, 0.07 mmol) and triethylamine (13.6 mg, 0.13 mmol) were added, and the reaction mixture was stirred at -40°C for 0.5 h. The reaction mixture was then poured into ice water (10 mL), and the resulting solution was extracted with dichloromethane (10 mL) three times. The combined organic layers were washed with water (10 mL), dried over anhydrous sodium sulfate, filtered, and concentrated. The residue was purified by preparative separation (solution A: 0.05% aqueous ammonia; solution B: acetonitrile) to afford 4.00 mg of the compound of example 34, with a yield of 7.61%. LC-MS [M+H]⁺: 738.5. ¹H NMR (400 MHz, D₂O) δ 7.93 (s, 1H), 7.83 (s, 2H), 7.34-7.30 (m, 2H), 7.25-7.19 (m, 3H), 4.99 (s, 1H), 4.58-4.50 (m, 3H), 4.04-3.98 (m, 3H), 3.72-3.60 (m, 2H), 3.40-3.35 (m, 1H), 2.50 - 2.43 (m, 1H), 2.35-2.30 (m, 2H), 2.05-1.98 (m, 2H), 1.78-1.74 (m, 2H), 1.60-1.55 (m, 1H), 1.42 (d, J = 6.5 Hz, 3H).

### Example 35

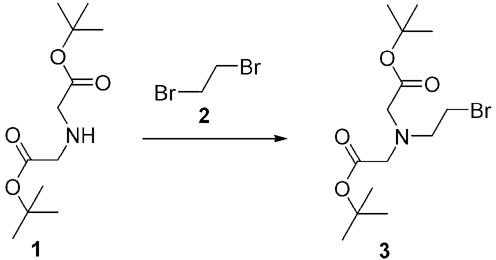

A solution of Compound 1 (1 g, 4.1 mmol) in 1,2-dibromoethane (10 mL) was treated with diisopropylethylamine (1.59 g, 12.3 mmol). The resulting solution was stirred at 80 °C for 50 h. The reaction was quenched with water (50 mL) and extracted with ethyl acetate (50 mL) three times. The combined organic layers were washed successively with water and saturated brine, dried over sodium sulfate, filtered, and concentrated. The residue was purified by silica gel column chromatography (petroleum ether/ethyl acetate = 7:3) to afford 600 mg of Compound 3, with a yield of 41.46%. LC-MS: 352.1[M+H]⁺.

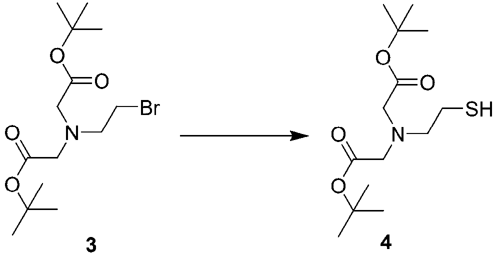

Thiourea (226.9 mg, 2.98 mmol) was added to a solution of Compound 3 (350 mg, 0.99 mmol) in ethanol (6 mL). The resulting solution was stirred at 80 °C for 16 h. Then, under a nitrogen atmosphere, ethylenediamine (1.492 g, 24.84 mmol) was added dropwise at 0 °C, and the mixture was stirred at 0 °C for 24 h. The solvent was removed under reduced pressure to give 250 mg of Compound 4, with a yield of 82.38%. LC-MS: 306.1 [M+H]⁺.

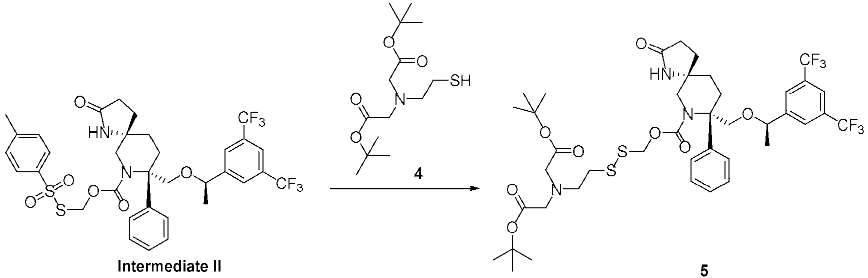

Intermediate II (200 mg, 0.268 mmol) was weighed into a 50 mL three-necked flask and dissolved in anhydrous dichloromethane (5 mL). Under a nitrogen atmosphere at -40 °C, triethylamine (81.3 mg, 0.804 mmol) and Compound 4 (164 mg, 0.536 mmol) were added. The reaction mixture was stirred at this temperature for 30 min, then quenched with water (50 mL) and extracted with ethyl acetate (50 mL) three times. The combined organic layers were washed successively with water and saturated brine, dried over sodium sulfate, filtered, and concentrated to afford 200 mg of Compound 5, with a yield of 83.31%. LC-MS: 893.6 [M+H]⁺.

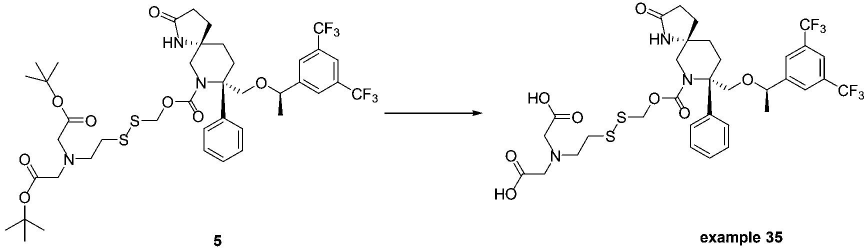

Compound 5 (200 mg, 0.224 mmol) was dissolved in anhydrous dichloromethane (5 mL) in a 50 mL single-necked flask, and trifluoroacetic acid (1 mL) was added. The reaction mixture was stirred at room temperature for 2 h, then concentrated to dryness. The residue was purified by preparative HPLC (Gemini-C18, 150 × 21.2 mm, 5 µm, ACN-H₂O, 0.1% TFA) to afford 15 mg of the compound of example 35, with a yield of 8.58%. LC-MS:781.5[M+H]⁺ . ¹H NMR (400 MHz, DMSO-*d₆*) δ 8.16 (s, 1H), 8.01 (s, 1H), 7.96 (s, 2H), 7.37 - 7.30 (m, 2H), 7.27 - 7.21 (m, 3H), 5.24 (s, 1H), 5.09 (d, J = 10.9 Hz, 1H), 4.66-4.59 (m, 2H), 4.08 (d, J = 8.8 Hz, 1H), 4.01 (d, J = 8.9 Hz, 1H), 3.70 (s, 4H), 3.55 (d, J = 13.7 Hz, 1H), 3.45 (d, J = 13.7 Hz, 1H), 3.11 (s, 2H), 2.93 (s, 2H), 2.29 - 2.13 (m, 2H), 2.06 - 1.91 (m, 2H), 1.69 (dd, J = 25.5, 12.6 Hz, 2H), 1.41 (t, J = 12.1 Hz, 4H).

### Example 36

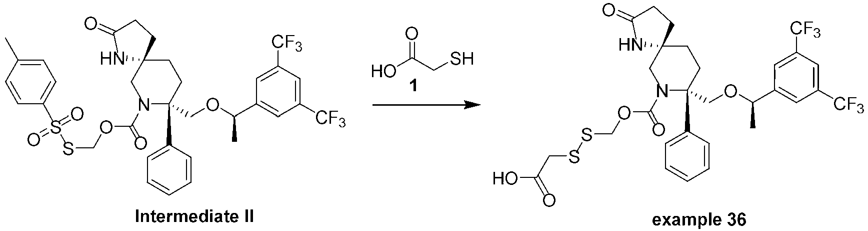

Under a nitrogen atmosphere, Intermediate II (130.0 mg, 0.17 mmol) was weighed into a 50 mL three-necked flask and dissolved in anhydrous dichloromethane (3 mL). Triethylamine (35.2 mg, 0.34 mmol) and Compound 1 (16.0 mg, 0.17 mmol) were added, and the reaction mixture was stirred at -40 °C for 1 h. The reaction mixture was then poured into ice water (10 mL), and the resulting solution was extracted with dichloromethane (10 mL) three times. The combined organic layers were washed with water (10 mL), dried over anhydrous sodium sulfate, filtered, and concentrated. The residue was purified by preparative separation (solvent A: 0.1% formic acid; solvent B: acetonitrile) to afford 30.00 mg of the compound of example 36, with a yield of 25.0%. LC-MS [M+H]⁺: 680.6. ¹H NMR (400 MHz, DMSO-*d₆*) δ 12.74 (s, 1H), 8.15 (s, 1H), 8.01 (s, 1H), 7.96 (s, 2H), 7.36 - 7.28 (m, 2H), 7.26 - 7.20 (m, 3H), 5.26 (s, 1H), 5.16 (d, *J=* 11.0 Hz, 1H), 4.67-4.64 (m, 1H), 4.09-4.00 (m, 2H), 3.65 - 3.50 (m, 3H), 3.48-3.43 (m, 1H), 2.36 - 2.21 (m, 2H), 2.20 - 2.10 (m, 1H), 2.04-2.01 (m, 1H), 1.95-1.91 (m, 1H), 1.70-1.63 (m, 2H), 1.44 (s, 1H), 1.39 (d, *J* = 6.4 Hz, 3H).

### Example 37

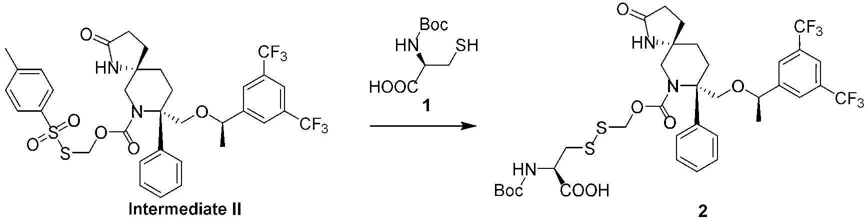

Intermediate II (60 mg, 0.0806 mmol) and triethylamine (16.1 mg, 0.1613 mmol) were placed in a 25 mL three-necked flask, anhydrous dichloromethane (4 mL) was added, and the system was purged with N₂ three times. The reaction mixture was cooled to -40 °C, and a solution of Compound 1 (17.8 mg, 0.0806 mmol) in dichloromethane (1 mL) was added dropwise. After the addition was complete, the reaction was allowed to proceed at -40 °C for 1 h. The reaction mixture was poured into ice water (10 mL), and the resulting solution was extracted with dichloromethane (10 mL) three times. The combined organic layers were dried over anhydrous sodium sulfate, filtered, and concentrated to give 30 mg of Compound 2, with a yield of 46%. LC-MS: 809.5 [M+H]⁺.

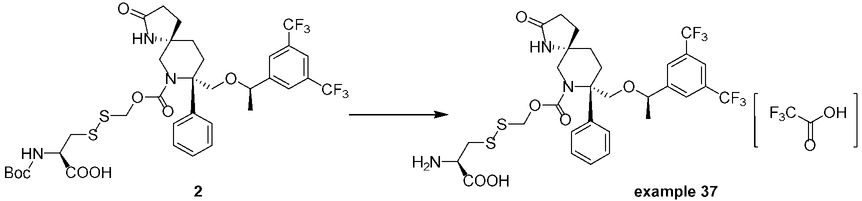

At 0 °C, Compound 2 (10 mg, 0.037 mmol) was dissolved in a mixture of dichloromethane (2 mL) and trifluoroacetic acid (0.5 mL) and stirred for 0.5 h. The reaction mixture was concentrated to dryness, and the residue was purified by reversed-phase preparative purification (ACN-H2O, 0.1% TFA) to afford 10 mg of the compound of example 37, with a yield of 38%. LC-MS: 709.6[M+H] ⁺. ¹H NMR (400 MHz, DMSO-*d₆*): δ 8.39 (s, 3H), 8.16 (s, 1H), 8.02 (s, 1H), 7.96 (s, 2H), 7.36 - 7.29 (m, 2H), 7.27 - 7.21 (m, 3H), 5.18 (d, J = 11.3 Hz, 1H), 4.65 (q, J = 6.1 Hz, 1H), 4.17 (s, 1H), 4.07 (d, J = 8.8 Hz, 1H), 4.00 (d, J = 8.9 Hz, 1H), 3.57 (d, J = 12.8 Hz, 2H), 3.30-3.20 (m, 3H), 2.31-2.13 (m, 2H), 2.03-1.94 (m, 2H), 1.74 - 1.65 (m, 2H), 1.45-1.38 (m, 4H).

### Example 38

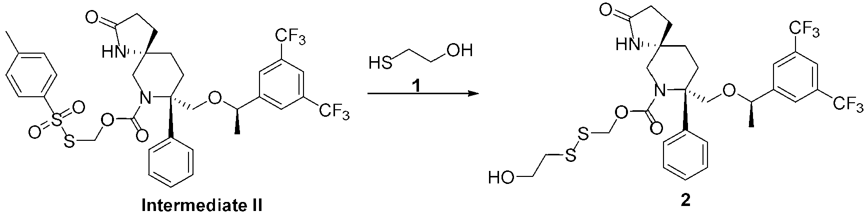

Under N₂ protection, Intermediate II (400.0 mg, 0.53 mmol) was dissolved in anhydrous dichloromethane (10 mL) in a 50 mL three-necked flask, followed by the addition of triethylamine (108.4 mg, 1.06 mmol) and Compound 1 (41.8 mg, 0.53 mmol). The reaction mixture was stirred at -40 °C for 10 min, then poured into ice water (20 mL). The resulting solution was extracted with dichloromethane (20 mL) three times. The combined organic layers were washed once with water (20 mL), dried over anhydrous sodium sulfate, filtered, and concentrated to give 320 mg of Compound 2, with a yield of 56.7%. The product was used directly in the next step without purification. LC-MS: 666.6 [M+H]⁺.

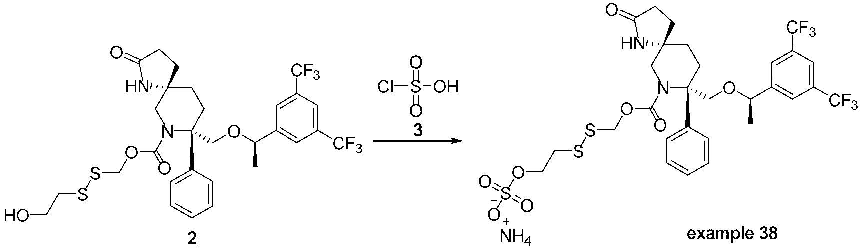

Compound 2 (100 mg, 0.150 mmol) was dissolved in anhydrous dichloromethane (5 mL) in a 50 mL three-necked flask, and chlorosulfonic acid (20.97 mg, 0.180 mmol) was added at 0 °C under N₂ protection. The reaction mixture was stirred at this temperature for 10 min, then concentrated to dryness. The residue was purified by preparative HPLC (Gemini-C18 150 × 21.2 mm, 5 µm, ACN-H₂O, 0.1% aqueous ammonia) to afford 70.12 mg of the compound of example 38, with a yield of 62.60%. LC-MS: 744.5[M-H]⁻. ¹H NMR (400 MHz, DMSO-*d₆*) δ 8.15 (s, 1H), 8.01 (s, 1H), 7.97 (s, 2H), 7.39 - 7.31 (m, 2H), 7.26-7.23(m, 3H), 7.20 (s, 1H), 7.07 (s, 1H), 6.95 (s, 1H), 5.25-5.09 (m, 2H), 4.66 (d, J = 6.4 Hz, 1H), 4.09 (d, J = 8.9 Hz, 1H), 4.01 (d, J = 9.1 Hz, 1H), 3.94-3.86 (m, 2H), 3.59 (d, J = 14.0 Hz, 1H), 3.38 (s, 1H), 2.90 (s, 2H), 2.34 - 2.24 (m, 1H), 2.18 - 2.09 (m, 1H), 2.07 - 1.93 (m, 2H), 1.71-1.62 (m, 2H), 1.44-1.38 (m , 4H).

### Example 39

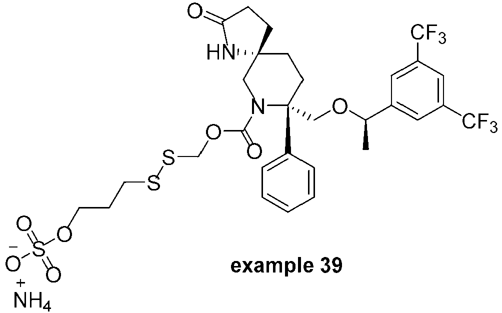

According to the method of example 38, the compound of example 39 was prepared via a two-step reaction using 3-mercapto-1-propanol in place of 2-mercaptoethanol. LC-MS [M-H]⁻: 758.6. ¹H NMR (400 MHz, DMSO-*d₆*) δ 8.13 (s, 1H), 8.01 (s, 1H), 7.97 (s, 2H), 7.36 - 7.31 (m, 2H), 7.27 - 7.22 (m, 3H), 7.20 (s, 1H), 7.07 (s, 1H), 6.95 (s, 1H), 5.21 (s, 1H), 5.08 (s, 2H), 4.69-4.65 (m, 1H), 4.10 (d, J = 9.3 Hz, 1H), 4.01 (d, J = 8.9 Hz, 2H), 3.75 (t, J = 6.1 Hz, 2H), 3.57 (d, J = 13.0 Hz, 2H), 3.44 (s, 2H), 2.25-2.20 (m, 2H), 2.18-2.15 (m, 1H), 2.01 -1.95(m, 1H), 1.81 (s, 2H), 1.69-1.63 (m, 2H), 1.39 (d, J = 6.3 Hz, 3H).

### Example 40

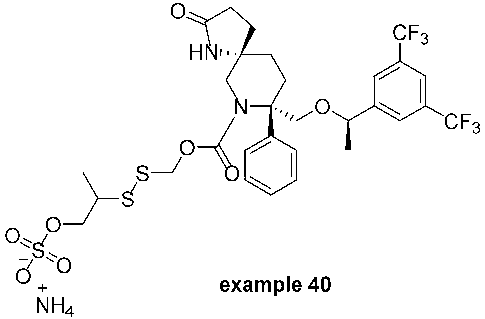

According to the method of example 38, the compound of example 40 was prepared via a two-step reaction using 2-mercaptopropanol in place of 2-mercaptoethanol. LC-MS: 758.7[M-H]⁻. ¹H NMR (400 MHz, D₂O) δ 7.54 (d, J = 0.5 Hz, 3H), 7.00 - 6.89 (m, 5H), 5.03 - 4.93 (m, 1H), 4.30 - 4.25 (m, 1H), 3.94 - 3.81 (m, 3H), 3.71 - 3.62 (m, 2H), 3.05 - 2.85 (m, 2H), 2.35 - 2.14 (m, 3H), 1.91 - 1.87 (m, 2H), 1.61 - 1.35 (m, 3H), 1.10 - 1.03 (m, 7H).

### Example 41

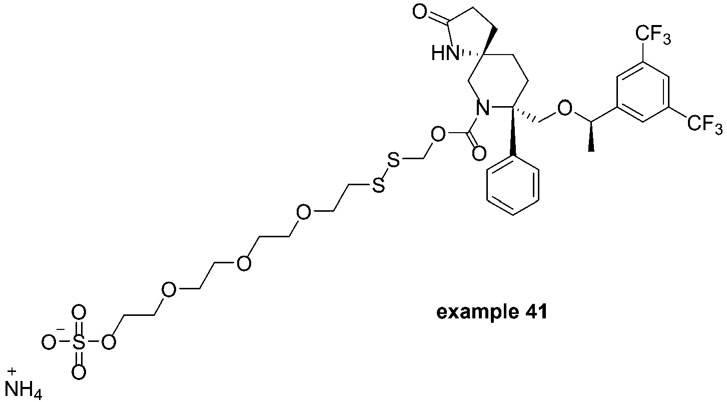

According to the method of example 38, the compound of example 41 was prepared via a two-step reaction using thiol-PEG4-alcohol in place of 2-mercaptoethanol. LC-MS: 876.7 [M-H]⁻. ¹H NMR (400 MHz, D₂O) δ 7.50 - 7.43 (m, 3H), 6.98 - 6.89(m, 5H), 5.03 (s, 1H), 4.23 (s, 1H), 4.00 (d, J = 2.9 Hz, 3H), 3.58 - 3.39 (m, 15H), 3.02 - 2.91 (m, 3H), 2.29 - 2.15 (m, 3H), 1.92 - 1.28 (m, 5H), 1.04 (s, 3H).

### Example 42

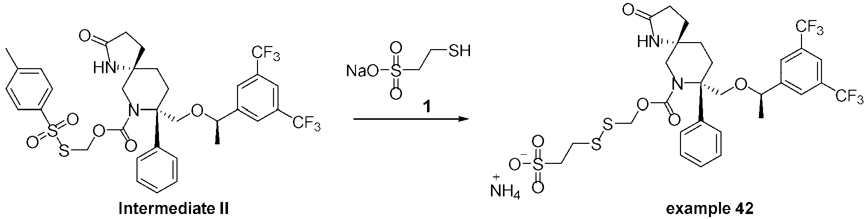

In a 50 mL three-necked flask, Intermediate II (100 mg, 0.150 mmol) was dissolved in anhydrous dichloromethane (5 mL). Under nitrogen protection at -40 °C, Compound 1 (26.45 mg, 0.180 mmol) and triethylamine (40.65 mg, 0.450 mmol) were added. The reaction mixture was stirred at this temperature for 30 minutes, then concentrated to dryness. The residue was purified by preparative HPLC (Gemini-C18 150 × 21.2 mm, 5 µm, ACN-H₂O, 0.1% ammonia) to afford 25 mg of the Compound of example 42, with a yield of 25.48%. LC-MS: 728.6[M-H]⁻. ¹H NMR (400 MHz, DMSO-*d₆*) δ 8.11 (s, 1H), 8.00 (s, 1H), 7.96 (s, 2H), 7.34 (t, J = 7.6 Hz, 2H), 7.27-7.22 (m, 3H), 7.07 (s, 3H), 5.25 (s, 1H), 5.03-5.00 (m, 1H), 4.67-4.64 (m, 1H), 4.11 (d, J = 8.9 Hz, 1H), 4.01 (d, J = 9.0 Hz, 1H), 3.59 (d, J = 13.7 Hz, 1H), 3.39 (d, J = 13.5 Hz, 1H), 2.93-2.89 (m, 2H), 2.71 - 2.65 (m, 2H), 2.29-2.30 (m, 2H), 2.20 - 2.08 (m, 1H), 2.02-1.95 (m, 2H), 1.72 - 1.59 (m, 2H), 1.42-4.38 (m, 4H).

### Example 43

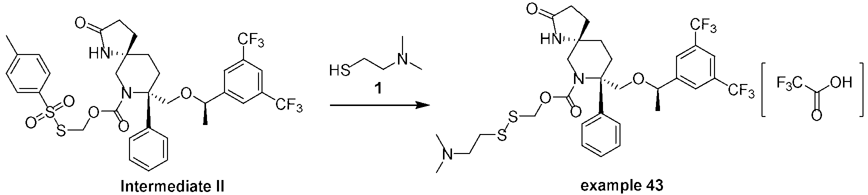

Intermediate II (200 mg, 0.2688 mmol) and triethylamine (53.7 mg, 0.5376 mmol) were placed in a 25 mL three-necked flask, and anhydrous dichloromethane (8 mL) was added. The system was purged with nitrogen three times. The reaction mixture was cooled to -40 °C, and a solution of Compound 1 (28.2 mg, 0.2688 mmol) in dichloromethane (2 mL) was added dropwise. After completion of the addition, the reaction was maintained at -40 °C for 0.5 hours. The reaction mixture was poured into ice water (10 mL), and the resulting mixture was extracted three times with dichloromethane (10 mL). The combined organic phases were dried over anhydrous sodium sulfate, filtered, and concentrated. The residue was purified by reversed-phase preparative chromatography (ACN-H₂O (0.1% TFA), gradient 30-60) to afford 20 mg of the compound of example 43, with a yield of 11%. LC-MS: 693.8[M+H] ⁺. ¹H NMR (400 MHz, DMSO-*d₆*): δ 9.43 (s, 1H), 8.16 (s, 1H), 8.02 (s, 1H), 7.96 (s, 2H), 7.38 - 7.29 (m, 2H), 7.25-7.23 (m, 3H), 5.25-5.15 (m, 2H), 4.66 (q, J = 6.4 Hz, 1H), 4.09-4.01 (m, 2H), 3.55 (d, J = 12.8 Hz, 2H), 3.33 (s, 3H), 3.06 (s, 2H), 2.79 (s, 6H), 2.35 - 2.12 (m, 2H), 2.09 - 1.82 (m, 2H), 1.75-1.65 (m, 2H), 1.45-1.39 (m, 4H).

### Example 44

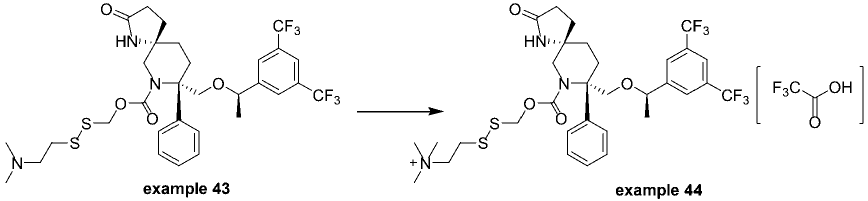

The compound of example 43 (20 mg, 0.02886 mmol, 1 eq) was placed in a 25 mL three-necked flask, and anhydrous dichloromethane (2 mL) was added. The system was purged with nitrogen three times. Iodomethane (0.1 mL, excess) was then added to the reaction mixture, which was stirred at room temperature overnight. The reaction mixture was directly concentrated to give a crude product, which was purified by reversed-phase preparative chromatography (ACN-H₂O (0.1% TFA), gradient 30-60) to afford the compound of example 44, with a yield of 21%. LC-MS: 707.6[M+H]⁺. ¹H NMR (400 MHz, DMSO-*d₆*): δ 8.15 (s, 1H), 8.02 (s, 1H), 7.96 (s, 2H), 7.36 - 7.29 (m, 2H), 7.25-7.22 (m, 3H), 5.31 - 5.07 (m, 2H), 4.66 (q, J = 6.3 Hz, 1H), 4.10-4.01 (m, 2H), 3.55 (d, J = 13.2 Hz, 5H), 3.18 (s, 2H), 3.06 (s, 9H), 2.29-2.15 (m, 2H), 2.08 - 1.98 (m, 1H), 1.94-1.86 (m, 1H), 1.78 - 1.63 (m, 2H), 1.45-1.40 (m, 4H).

### Example 45

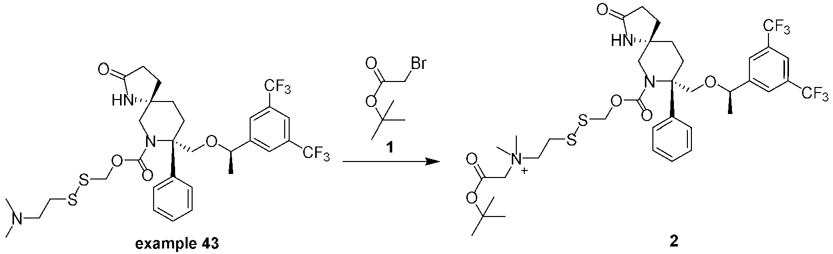

Under a nitrogen atmosphere, the compound of example 43 (20.0 mg, 0.03 mmol) was dissolved in anhydrous dichloromethane (4 mL) in a 50 mL three-necked flask. tert-Butyl bromoacetate (16.8 mg, 0.09 mmol) was added, and the reaction mixture was stirred at room temperature for 16 h. The reaction mixture was then poured into ice water (10 mL), and the resulting solution was extracted with dichloromethane (10 mL) three times. The combined organic phases were washed with water (10 mL), dried over anhydrous sodium sulfate, filtered, and concentrated to give 20.0 mg of crude Compound 2, with a yield of 67.9%. The crude product was used directly in the next step without further purification. LC-MS [M+H]⁺: 807.6.

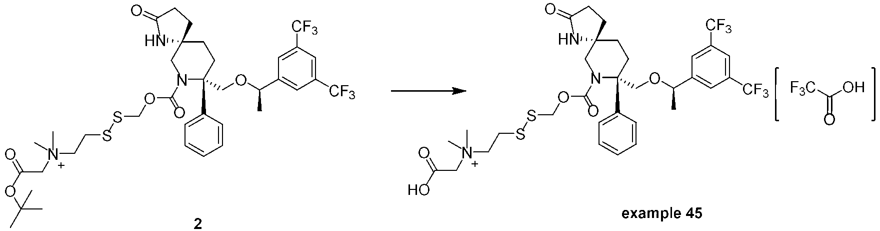

Under a nitrogen atmosphere, Compound 2 (20.0 mg, 0.01 mmol) was dissolved in a mixed solution of dichloromethane (4 mL) and trifluoroacetic acid (1 mL) in a 25 mL single-necked flask. The reaction mixture was stirred at room temperature for 16 h and then concentrated to dryness. The residue was purified by preparative HPLC (mobile phase A: 0.1% TFA; mobile phase B: acetonitrile) to afford 4.0 mg of the compound of example 45, with a yield of 41.5%. LC-MS [M+H]+: 751.9. ¹H NMR (400 MHz, DMSO-*d₆*) δ 8.14 (s, 1H), 8.02 (s, 1H), 7.97 (s, 2H), 7.39 - 7.30 (m, 2H), 7.28 - 7.21 (m, 3H), 5.34 - 5.19 (m, 1H), 5.15 (d, *J* = 11.0 Hz, 1H), 4.67 (q, *J* = 6.2 Hz, 1H), 4.33 (s, 2H), 4.05-4.01 (m, 2H), 3.78 (d, *J* = 7.1 Hz, 2H), 3.56-3.50 (m, 4H), 3.20 (s, 6H), 2.34 - 2.10 (m, 3H), 2.01-1.93 (m, 2H), 1.75 - 1.62 (m, 2H), 1.46 (s, 1H), 1.41 (d, *J* = 6.4 Hz, 3H).

### Example 46

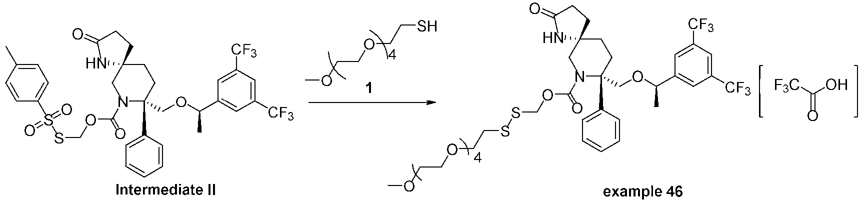

In a 50 mL three-necked flask, Intermediate II (30 mg, 0.042 mmol) was dissolved in water (2 mL). Under a nitrogen atmosphere at -40 °C, triethylamine (8.14 mg, 0.08 mmol) and Compound 1 (30 mg, 0.042 mmol) were added. The reaction mixture was stirred at this temperature for 30 min, after which the reaction was quenched with water (5 mL). The resulting solution was extracted with ethyl acetate (10 mL) three times. The combined organic phases were washed successively with water and saturated brine, dried over sodium sulfate, filtered, and concentrated. The residue was purified by preparative HPLC (mobile phase A: 0.1% TFA; mobile phase B: acetonitrile) to afford 8.66 mg of the compound of example 46, with a yield of 25.12%. LC-MS: 857.3[M+H]⁺. ¹H NMR (400 MHz, MeOD) δ 7.93 (s, 2H), 7.89 (s, 1H), 7.41 - 7.34 (m, 2H), 7.33 - 7.23 (m, 3H), 5.36 (s, 1H), 5.21 - 5.14 (m, 1H), 4.69 - 4.62 (m, 1H), 4.27 - 4.20 (m, 1H), 4.08 - 4.00 (m, 1H), 3.84 - 3.78 (m, 1H), 3.75 - 3.67 (m, 2H), 3.63 (s, 12H), 3.56 - 3.52 (m, 2H), 3.40 - 3.35 (m, 3H), 2.99 - 2.81 (m, 3H), 2.61 - 2.44 (m, 3H), 2.43 - 2.35 (m, 1H), 2.22 - 2.11 (m, 2H), 1.90 - 1.81 (m, 2H), 1.73 - 1.60 (m, 2H), 1.49 (d, J = 6.3 Hz, 3H).

### Example 47

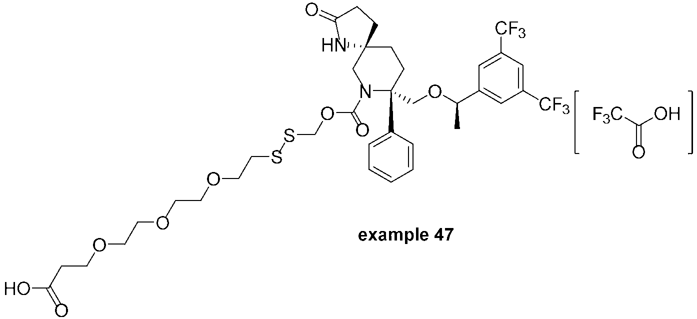

According to the method of example 46, mercapto-triethylene glycol-carboxylic acid was used in place of 3,6,9,12,15-pentaoxaoctadecane-1-thiol to react with Intermediate II, thereby affording the compound of example 47. LC-MS: 827.2[M+H]⁺. ¹H NMR (400 MHz, MeOD) δ 7.93 (s, 2H), 7.88 (s, 1H), 7.40 - 7.34 (m, 2H), 7.34 - 7.25 (m, 3H), 5.40 - 5.10 (m, 2H), 4.70 - 4.60 (m, 1H), 4.26 - 4.17 (m, 1H), 4.09 - 4.00 (m, 1H), 3.86 - 3.78 (m, 1H), 3.77 - 3.66 (m, 4H), 3.65 - 3.58 (m, 8H), 3.55 - 3.44 (m, 1H), 3.02 - 2.79 (m, 2H), 2.61 - 2.51 (m, 3H), 2.51 - 2.44 (m, 1H), 2.44 - 2.34 (m, 1H), 2.20 (dd, J = 19.5, 11.7 Hz, 2H), 1.91 - 1.80 (m, 2H), 1.74 - 1.61 (m, 1H), 1.48 (d, J = 6.5 Hz, 3H).

### Example 48

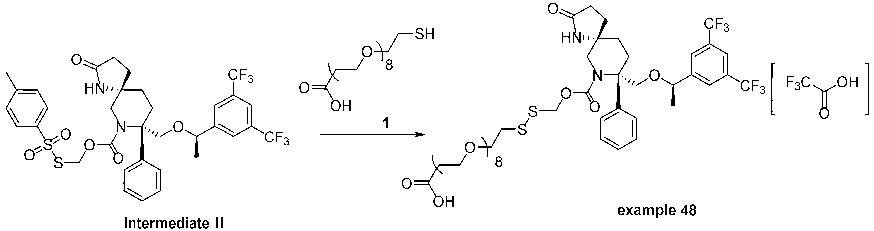

Intermediate II (100 mg, 0.107 mmol, 1 eq) was dissolved in anhydrous dichloromethane (5 mL) in a 50 mL three-necked flask. Under nitrogen protection at -40 °C, triethylamine (21.67 mg, 0.214 mmol, 2 eq) and Compound 1 (98.54 mg, 0.161 mmol, 1.5 eq) were added. The reaction mixture was stirred at this temperature for 30 min, then quenched with water (50 mL) and extracted with dichloromethane (50 mL) three times. The combined organic layers were washed with water and brine, dried over anhydrous sodium sulfate, filtered, concentrated, and purified by preparative HPLC (Gemini-C18, 150 × 21.2 mm, 5 µm; ACN-H₂O (0.1% TFA), gradient 50-70) to afford the compound of example 48 (25 mg) in 17.63% yield. LC-MS [M+H]⁺: 1046.8. ¹H NMR (400 MHz, DMSO-*d₆*) δ 12.13 (s, 1H), 8.13 (s, 1H), 8.01 (s, 1H), 7.96 (s, 2H), 7.35 - 7.30 (m, 2H), 7.25 - 7.20 (m, 3H), 5.21-5.10 (m, 2H), 4.66 (q, J = 6.2 Hz, 1H), 4.05 (dd, J = 19.2, 8.9 Hz, 2H), 3.59 (dd, J = 12.3, 6.0 Hz, 5H), 3.49 - 3.47 (m, 10H), 3.36 (s, 20H), 2.89 (s, 2H), 2.43 (t, J = 6.3 Hz, 2H), 2.28 - 2.14 (m, 2H), 2.04-1.91 (m, 2H), 1.71-1.64 (m, 2H), 1.44-1.39 (m, 4H).

### Example 49

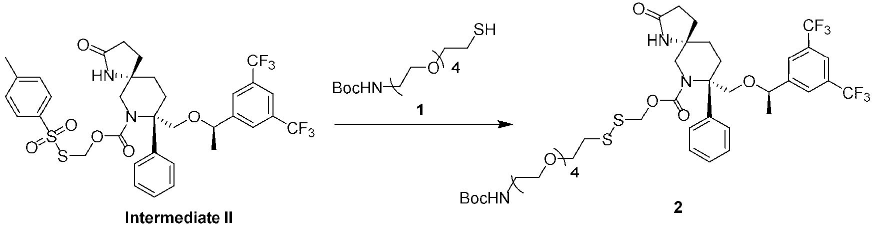

Intermediate II (200.0 mg, 0.27 mmol) was dissolved in anhydrous dichloromethane (10 mL) in a 50 mL three-necked flask under nitrogen protection. Triethylamine (81.3 mg, 0.80 mmol) and Compound 1 (113.6 mg, 0.32 mmol) were added, and the reaction mixture was stirred at -40 °C for 10 min. The reaction mixture was then poured into ice water (20 mL), and the resulting solution was extracted with dichloromethane (20 mL) three times. The combined organic layers were washed once with water, dried over anhydrous sodium sulfate, filtered, and concentrated to afford Compound 2 (200 mg) in 71.2% yield, which was used directly in the next step without further purification. LC-MS [M+H]⁺: 941.5.

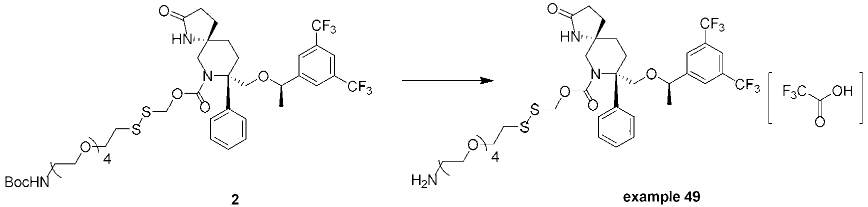

Compound 2 (200.0 mg, 0.2 mmol) was dissolved in a mixture of dichloromethane (4 mL) and trifluoroacetic acid (1 mL) in a 25 mL single-necked flask under nitrogen protection. The reaction mixture was stirred at room temperature for 0.5 h, then concentrated to dryness. The residue was purified by preparative HPLC (solution A: 0.1% TFA; solution B: acetonitrile) to afford the compound of example 49 (30.0 mg) in 16.0% yield. LC-MS [M+H]⁺: 841.6. ¹H NMR (400 MHz, DMSO-*d₆*) δ 8.15 (s, 1H), 8.02 (s, 1H), 7.96 (s, 2H), 7.75 (s, 2H), 7.35 - 7.29 (m, 2H), 7.25 - 7.21 (m, 3H), 5.21 (s, 1H), 5.12 (d, J = 10.6 Hz, 2H), 4.66 (q, J = 6.4 Hz, 2H), 4.08-4.05 (m, 2H), 3.60 - 3.54 (m, 8H), 3.52-3.50 (m, 8H), 2.97-2.95 (m, 2H), 2.90 (s, 2H), 2.33 - 2.11 (m, 4H), 2.01-1.98 (m, 1H), 1.92-1.86 (m, 1H), 1.74 - 1.66 (m, 2H), 1.40 (d, J = 6.3 Hz, 3H).

### Example 50

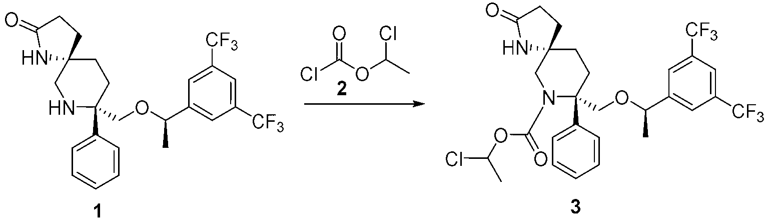

Compound 1 (1.1 g, 2.19 mmol) was dissolved in dichloromethane (15 mL), and the solution was cooled to -30 °C. Under nitrogen protection, diisopropylethylamine (2.26 g, 17.52 mmol) and trimethylchlorosilane (618 mg, 5.69 mmol) were added, and the reaction mixture was stirred at room temperature for 2 h. Then, under nitrogen protection, Compound 2 (385 mg, 2.69 mmol) was added dropwise at -5 °C, and the solution was stirred at -5 °C for 2 h. The reaction was quenched with water (200 mL), and the resulting mixture was extracted with dichloromethane (200 mL) three times. The combined organic layers were washed successively with water and brine, dried over anhydrous sodium sulfate, filtered, and concentrated. The residue was purified by silica gel column chromatography (petroleum ether/ethyl acetate = 4/1) to afford Compound 3 (700 mg) in 52.51% yield. LC-MS: 606.9 [M+H]⁺.

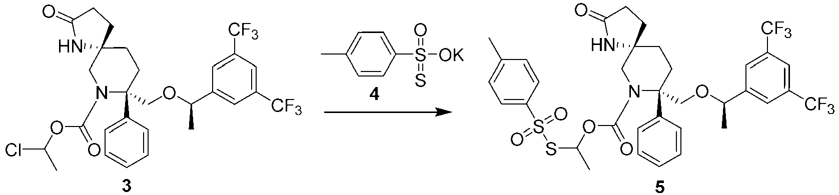

Compound 4 (167 mg, 0.74 mmol) was added to a solution of Compound 3 (300 mg, 0.49 mmol) in anhydrous acetonitrile (5 mL). The resulting solution was stirred at 100 °C for 3 h. The solvent was then removed under reduced pressure to afford Compound 5 (300 mg) in 80.04% yield. LC-MS: 759.2 [M+H]⁺.

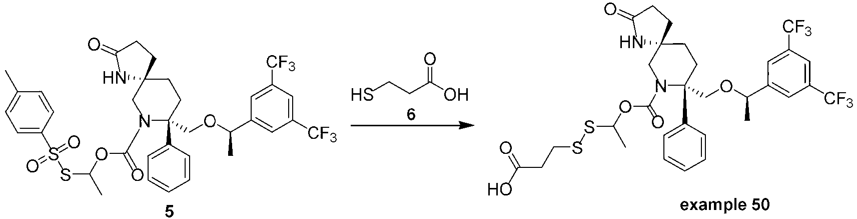

Compound 5 (100 mg, 0.131 mmol) was dissolved in anhydrous dichloromethane (5 mL) in a 50 mL three-necked flask, and the solution was cooled to 0 °C under nitrogen protection. Triethylamine (39.89 mg, 0.393 mmol) and 3-mercaptopropionic acid (41.84 mg, 0.393 mmol) were added, and the reaction mixture was stirred at this temperature for 30 min. The reaction mixture was concentrated to dryness, and the residue was purified by preparative HPLC (Gemini-C18, 150 × 21.2 mm, 5 µm; ACN-H₂O, 0.1% FA) to afford the compound of example 50 (5 mg) in 4.8% yield. LC-MS:731.1[M+Na]⁺ . ¹H NMR (400 MHz, DMSO) δ 8.12 (s, 1H), 8.03 - 7.92 (m, 3H), 7.35 - 7.30 (m, 2H), 7.27-7.21 (m, 3H), 5.69 (s, 1H), 4.66-4.64 (m, 1H), 4.12 (d, J = 8.6 Hz, 1H), 4.01 (d, J = 9.0 Hz, 1H), 3.57 - 3.33 (m, 2H), 2.90 - 2.69 (m, 2H), 2.61 - 2.52 (m, 2H), 2.44 (s, 1H), 2.31 - 2.11 (m, 2H), 2.08-1.97 (m, 2H), 1.75 - 1.59 (m, 2H), 1.46-1.39 (m, 7H).

### Test Example 1: Plasma Stability Assay

### 1.1 Test plasma

Human plasma (Vendor: AoNeng Biotech).

### 1.2 Preparation of compound solutions

A certain amount of the compound of the examples was weighed and dissolved in DMSO to prepare a 10 mM stock solution. An aliquot of the 10 mM stock solution was diluted with DMSO to obtain a 1600 µM intermediate solution. An aliquot of the 1600 µM intermediate solution was further diluted with 45% methanol to prepare a 16 µM working solution.

### 1.3 Experimental procedure

1) Plasma was pre-incubated in a water bath at 37 °C for 5 min.
2) 4 µL of the 16 µM working solution of the compound of the examples was added to 60 µL of plasma to give a final compound concentration of 1 µM, and the mixture was gently mixed.
3) Samples were incubated at each time point (0, 15, 30, 60, and 120 min). After incubation, 192 µL of acetonitrile containing an internal standard was added, followed by shaking on a shaker for 1 min. The mixture was centrifuged at 3700 rpm at 4 °C for 20 min, and the supernatant was collected for LC-MS analysis.

### 2. Results

The conversion of the compounds of the present invention in human plasma is shown in Table 1.

**Table 1**

| Examples | Compound of formula (I) | 17 | 25 | 38 | 39 | 42 |
|---|---|---|---|---|---|---|
| Time point (min) | Parent drug% | | | | | |
| 0 | 2.99 | 8.76 | 0.32 | 3.78 | 2.08 | 6.09 |
| 15 | 2.87 | 13.98 | 9.85 | 50.99 | 22.89 | 52.71 |
| 30 | 3.22 | 17.55 | 19.48 | 54.49 | 35.31 | 60.94 |
| 60 | 3.13 | 31.00 | 30.30 | 49.71 | 45.97 | 53.88 |
| 120 | 3.78 | 40.63 | 45.76 | 61.38 | 55.21 | 66.59 |

| | | | | | | |
|---|---|---|---|---|---|---|
| Note: In the table, the parent drug refers to Lorapiprant. Conclusion: The compounds of formula (I) are basically not converted to the active metabolite Lorapiprant in plasma. Examples 17, 25, 38, 39, and 42 are converted to the active metabolite Lorapiprant in plasma by approximately half or more. | | | | | | |

### Test Example 2: Liver S9 Stability Assay

### 1.1 Liver S9

Human pooled liver S9 (Vendor: Xenotech), thawed in a 37°C water bath before use.

### 1.2 Compound Solution Preparation

Weigh a certain amount of the example compound, dissolve in DMSO to prepare a 10 mM stock solution, then dilute an appropriate volume of the stock solution with DMSO to obtain a 200 µM working solution.

### 1.3 Sample Incubation

Take 1.5 µL of the 200 µM working solution of the example compound and add it to 238.5 µL of liver S9 working solution, pre-incubate in a 37°C water bath for 5 min; then add 60 µL of NADPH working solution to initiate the reaction and mix by gentle inversion. At the 0 min time point, transfer the above mixture into quenching solution and mix; at the 5, 15, 30, and 60 min time points, transfer the mixture into quenching solution and DMSO and mix. Vortex vigorously for 1 min, then centrifuge at 3700 rpm, 4°C for 15 min. Collect the supernatant for LC-MS analysis.

### 2. Results

The conversion of the example compounds of the invention in human liver S9 is shown in Table 2.

**Table 2**

| Examples | Compound of formula (I) | 2 | 25 | 30 | 32 | 33 | 36 | 37 |
|---|---|---|---|---|---|---|---|---|
| Time point (min) | Parent drug% | | | | | | | |
| 0 | 37.2 | 6.5 | 25.2 | 18.1 | 11.2 | 13.2 | 11.8 | 23.9 |
| 5 | 36.4 | 11.7 | 30.2 | 23.0 | 16.8 | 22.5 | 27.9 | 31.8 |
| 15 | 43.8 | 20.4 | 47.4 | 28.1 | 25.5 | 41.9 | 36.7 | 34.2 |
| 30 | 40.3 | 24.7 | 61.9 | 34.3 | 31.2 | 50.0 | 41.4 | 43.2 |
| 60 | 34.7 | 37.0 | 62.9 | 32.5 | 35.4 | 57.3 | 45.1 | 38.0 |
| | | | | | | | | |

| Examples | 38 | 39 | 42 | 44 | 45 | 46 | 47 | |
|---|---|---|---|---|---|---|---|---|
| Time point (min) | Parent drug% | | | | | | | |
| 0 | 23.1 | 24.3 | 34.8 | 42.6 | 60.0 | 8.8 | 14.4 | |
| 5 | 32.7 | 40.7 | 40.6 | 48.3 | 59.5 | 26.0 | 23.8 | |
| 15 | 37.8 | 57.2 | 48.5 | 51.5 | 63.7 | 35.2 | 42.5 | |
| 30 | 36.6 | 58.3 | 51.9 | 55.2 | 63.7 | 36.7 | 45.8 | |
| 60 | 36.4 | 59.2 | 44.0 | 55.4 | 64.7 | 36.5 | 41.5 | |

| | | | | | | | | |
|---|---|---|---|---|---|---|---|---|
| Note: In the table, the parent drug refers to Lorapiprant. Conclusion: After 30 min incubation in human liver S9, Examples 2, 30, 32, 37, 38, 39, 42, 44, 45, and 46 show a relatively high conversion to the active metabolite Lorapiprant, indicating a fast conversion rate. Among them, Examples 25, 33, 36, 38, 39, 42, 44, 45, and 47 show conversion rates and extents comparable to or better than those of the compounds of formula (I). | | | | | | | | |

### Test Example 3: Thermodynamic Solubility

### 1.1 Reagent Preparation

100 mM phosphate buffer solution: Dissolve 7.94 g of K₂HPO₄.3H₂O and 2.07 g of KH₂PO₄ in 500 mL ultrapure water. Adjust the solution pH to 7.4 with 1 N NaOH, then filter and set aside.

### 1.2 Test Method

Accurately weigh an appropriate amount of the example compound and add it in small portions to 100 mM phosphate buffer solution to prepare a final concentration of 20 mg/mL. After shaking at room temperature for 16 hours, let stand for 30 min, then filter the supernatant and dilute with DMSO. Add the diluted sample solution to quenching solution, mix thoroughly, and analyze by LC/MS. Data are shown in Table 3.

### 2. Results

**Table 3**

| Examples | Solubility (pH=7.4) |
|---|---|
| 33 | 13.8 mg/mL |
| 38 | 17.7 mg/mL |
| 39 | 6.54 mg/mL |
| 42 | 13.2 mg/mL |
| 47 | 10.9 mg/mL |

### Test Example 4: In Vitro Human Hemolysis Assay

### 1.1 Test Samples

Examples 38, 39, 42.

### 1.2 Test Whole Blood

Fresh human whole blood.

### 1.3 Preparation of Cell Suspension

Collect the whole blood and store it in a 250 mL Erlenmeyer flask. Shake at 500 rpm for 10 minutes to remove fibrinogen, producing defibrinated blood. Wash with physiological saline (blood volume: saline volume = 1:4). Centrifuge the blood/saline mixture at 2500 rpm for 15 minutes at 4°C, discard the supernatant. Wash the sedimented red blood cells several times with physiological saline (blood volume: saline volume = 1:4) and centrifuge at 2500 rpm for 15 minutes until the supernatant is no longer red. Prepare a red blood cell suspension using physiological saline at a ratio of 1:49 (cell volume: saline volume) and store at 4°C until use.

### 1.4 Preparation of Working Solutions

Prepare compounds of examples 38, 39, 42 in DMSO at concentrations of 200, 80, and 20 µM, respectively, for later use.

### 1.5 Experimental Procedure

1) Add the working solution of the compounds of examples 38, 39, 42 to the red blood cell suspension and mix gently.
2) Incubate the mixture in a CO₂ incubator at 37±0.5°C for 45 minutes. Observe for aggregation every 15 minutes.
3) Centrifuge the resulting solution at 4000 rpm for 15 minutes at 4°C. Transfer 100 µL of the supernatant and measure absorbance at 540 nm to calculate the hemolysis rate (%).

### 2. Results

Compounds of examples 38, 39, 42 showed no hemolytic activity even at concentrations as high as 200 µM.

### Test Example 5: In Vivo Pharmacokinetic Study in Rats

Rats were used as test subjects. LC/MS/MS was employed to measure the drug concentrations in whole blood at various time points after intravenous administration compound of the Formula (I) and compounds of examples 38, 39, 42. This study evaluated the pharmacokinetic behavior of the compounds in rats and assessed their pharmacokinetic characteristics.

### 1.1 Drug Preparation

Weighed amounts of the compound of the Formula (I) and compounds of examples 38, 39, 42 were each dissolved in 20 mmol/L sodium dihydrogen phosphate to prepare pH 4.0 solutions for use.

### 1.2 Administration

Intravenous push injection was performed over approximately 5 minutes, with a dose of 2 mg/kg, a concentration of 0.4 mg/mL, and a volume of 5 mL/kg.

### 1.3 Procedure

Blood samples were collected via jugular vein puncture before administration and at 0.083 h, 0.25 h, 0.5 h, 1 h, 2 h, 4 h, 6 h, 8 h, 10 h, 24 h, and 48 h after administration. Sodium heparin was used as an anticoagulant. Immediately after collection, whole blood proteins were precipitated using methanol:acetonitrile (1:1), and the supernatant was transferred for LC/MS/MS analysis.

### 1.4 Pharmacokinetic Parameter Results

The pharmacokinetic parameters of the compounds are summarized in Table 4.

**Table 4**

| | Formula (I) | | 38 | | 39 | | 42 | |
|---|---|---|---|---|---|---|---|---|
| | (ng/mL)^{a} | (ng/mL)^{b} | (ng/mL)^{a} | (ng/mL)^{b} | (ng/mL)^{a} | (ng/mL)^{b} | (ng/mL)^{a} | (ng/mL)^{b} |
| AUCₗₐₛₜ (ng/mL*h) | 82.6 | 2308 | 10.7 | 2118 | 19.9 | 2136 | 9.42 | 3474 |
| Cₘₐₓ (ng/mL) | 328 | 338 | 61.0 | 397 | 116 | 263 | 61.2 | 441 |
| T_{1/2} (h) | 0.439 | 6.74 | 0.160 | 5.67 | 0.139 | 6.06 | 0.0776 | 6.96 |
| Tₘₐₓ (h) | 0.0833 | 0.139 | 0.0833 | 0.0833 | 0.0833 | 0.250 | 0.0833 | 0.0833 |
| MRT_{inf}(h) | 0.350 | 9.01 | 0.150 | 8.22 | 0.126 | 8.58 | 0.0909 | 10.1 |

| | | | | | | | | |
|---|---|---|---|---|---|---|---|---|
| Note: ^{a}Pharmacokinetic parameters of the compounds from the examples in rats; ^{b}Pharmacokinetic parameters of lorlatinib formed from the example compounds in rats. Conclusion: After injection, compounds of examples 38, 39, 42 were rapidly converted to the active metabolite lorlatinib in rats. The AUClast, Cmax, and Tmax data indicate that compounds of examples 38, 39, 42 exhibited lorlatinib pharmacokinetic properties comparable to the compound of Formula (I). The T1/2 data show that example 42 is converted to lorlatinib more rapidly after administration, and the AUClast and Cmax data indicate that the systemic exposure of lorlatinib after conversion is higher, demonstrating superior pharmacokinetic properties compared to the Formula (I) compound. | | | | | | | | |

### Test Example 6: Pharmacokinetic Study of Compounds in Crab-Eating Monkeys

Crab-eating monkeys were used as test subjects. LC/MS/MS was employed to measure drug concentrations in whole blood at various time points after intravenous administration of the Formula (I) compound and compound of example 42. This study evaluated the pharmacokinetic behavior of the compounds in crab-eating monkeys and assessed their pharmacokinetic characteristics.

### 1.1 Drug Preparation

Weighed amounts of the compound of Formula (I) and compound of example 42 were each dissolved in 5% DMSO/20% PEG400/H₂O to prepare solutions for use.

### 1.2 Administration

Intravenous infusion was performed over 30 minutes, with a dose of 2 mg/kg, a concentration of 0.4 mg/mL, and a volume of 5 mL/kg.

### 1.3 Procedure

Blood samples were collected from the forelimb vein before administration and at 0.083 h, 0.25 h, 0.5 h, 1 h, 2 h, 4 h, 8 h, 10 h, 24 h, 36 h, 48 h, and 72 h after the start of administration. Sodium heparin was used as an anticoagulant. Immediately after collection, whole blood proteins were precipitated using methanol:acetonitrile (1:1), and the supernatant was transferred for LC/MS/MS analysis.

### 1.4 Pharmacokinetic Parameter Results

The pharmacokinetic parameters of the compounds are summarized in Table 5.

**Table 5**

| | Formula (I) | | 42 | |
|---|---|---|---|---|
| | (ng/mL)^{a} | (ng/mL)^{b} | (ng/mL)^{a} | (ng/mL)^{b} |
| AUCₗₐₛₜ (ng/mL*h) | 688 | 3555 | 528 | 4420 |
| Cₘₐₓ (ng/mL) | 2110 | 188 | 1617 | 237 |
| T_{1/2} (h) | 0.151 | 10.6 | 0.101 | 8.56 |
| Tₘₐₓ (h) | 0.500 | 1.17 | 0.5 | 1.67 |

| | | | | |
|---|---|---|---|---|
| Note: ^{a}Pharmacokinetic parameters of the compounds from the examples in crab-eating monkeys; ^{b}Pharmacokinetic parameters of lorlatinib formed from the example compounds in crab-eating monkeys. Conclusion: After intravenous infusion, the Formula (I) and example 42 were rapidly converted to the active metabolite lorlatinib in crab-eating monkeys. Plasma metabolite identification showed that lorlatinib is the major metabolite of example 42, with approximately 98% of Implementation Example 42 being converted to the active metabolite lorlatinib. The T1/2 data indicate that example 42 is converted to lorlatinib more rapidly after administration, and the AUClast and Cmax data show that the systemic exposure of lorlatinib derived from example 42 is significantly higher than that from the Formula (I) compound, demonstrating superior pharmacokinetic properties compared to the Formula (I) compound. | | | | |

## Claims

1. A compound having a structure represented by formula (II), formula (III), or formula (IV), an isomer thereof, or a pharmaceutically acceptable salt or acid thereof, or a deuterated derivative thereof: 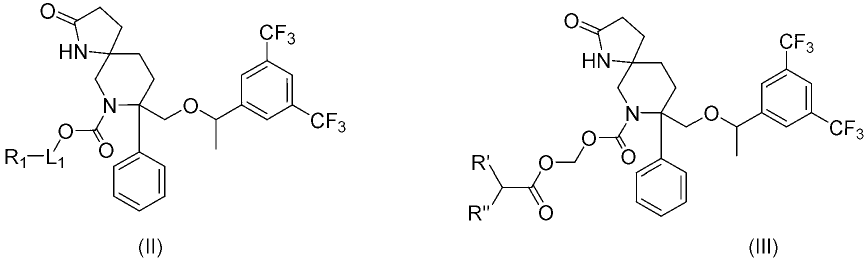 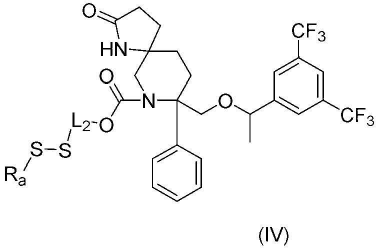 wherein:
in formula (II),
L₁ is selected from -[C(R₄)(R₅)]ₘ-(A)_{q}-, -[C(R₄)(R₅)]ₘ-(A)ₚ-C(O)-[C(R₄)(R₅)]ₙ-(A)_{q}-, -[C(R₄)(R₅)]ₘ-C(O)-(A)ₚ-[C(R₄)(R₅)]ₙ-(A)_{q}-, -[C(R₄)(R₅)]ₘ-(A)ₚ-B-(A)_{q}-, - [C(R₄)(R₅)]ₘ-C(O)-(A)ₚ-B-(A)_{q}-, -[C(R₄)(R₅)]ₘ-(A)ₚ-C(O)-B-(A)_{q}-, -[C(R₄)(R₅)]ₘ₋(A)ₚ-C(S)-B-(A)_{q}-, -[C(R₄)(R₅)]ₘ-C(S)-(A)ₚ-B-(A)_{q}-, -[C(R₄)(R₅)]ₘ-B-(A)ₚ-, - [C(R₄)(R₅)]ₘ-B-C(O)-(A)ₚ-, -[C(R₄)(R₅)]ₘ-B-(A)ₚ-C(O)-(A)_{q}-, -[C(R₄)(R₅)]ₘ-B-(A)ₚ-C(S)-(A)_{q}-, -[C(R₄)(R₅)]ₘ-B-C(S)-(A)ₚ-, -[C(R₄)(R₅)]ₘ- (A)ₚ-S(O)ₜ-B-, - [C(R₄)(R₅)]ₘ-S(O)ₜ-(A)ₚ-B-, -[C(R₄)(R₅)]ₘ-S(O)=N-[C(R₄)(R₅)]ₙ-, and -[C(R₄)(R₅)]ₘ-N=S(O)-[C(R₄)(R₅)]ₙ-;
A is selected from O, S, and N(R₆);
B is selected from the group consisting of cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, or alkynyl, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of alkyl, haloalkyl, cycloalkyl, alkoxy, hydroxyalkyl, alkenyl, alkynyl, nitro, cyano, hydroxy, SH, and halogen;
R₁ is selected from 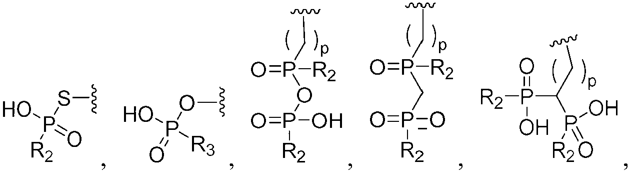 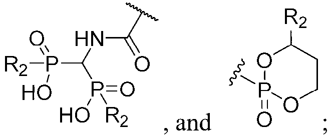
R₂ is selected from the group consisting of hydrogen, hydroxy, SH, alkyl, haloalkyl, alkoxy, -[C(R₄)(R₅)]ₘ-N(R₇)₃⁺, cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, alkynyl, or cyano, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₃ is selected from the group consisting of hydrogen, SH, alkyl, haloalkyl, alkoxy, -[C(R₄)(R₅)]ₘ-N(R₇)₃⁺, cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, alkenyl, or alkynyl, wherein the cycloalkyl, heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
when L₁ is -[C(R₄)(R₅)]ₘ-(A)ₚ-B-(A)_{q}-, -[C(R₄)(R₅)]ₘ-S(O)=N-[C(R₄)(R₅)]ₙ-, or - [C(R₄)(R₅)]ₘ-N=S(O)-[C(R₄)(R₅)]ₙ-, R₁ can further be selected from 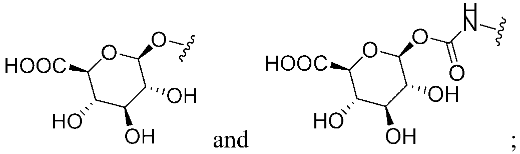
when L₁ is-[C(R₄)(R₅)]ₘ-(A)_{q}-, -[C(R₄)(R₅)]ₘ-S(O)=N-[C(R₄)(R₅)]ₙ-, or - [C(R₄)(R₅)]ₘ-N=S(O)-[C(R₄)(R₅)]ₙ-, R₁ can further be 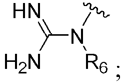
R₄ and R₅ are independently selected from the group consisting of hydrogen, hydroxy, SH, alkyl, haloalkyl, alkoxy, cycloalkyl, heterocyclyl, aryl, heteroaryl, alkenyl, alkynyl, cyano, or halogen, wherein the cycloalkyl, heterocyclyl, aryl, and heteroaryl are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₆ is selected from the group consisting of hydrogen, C₁-C₆ alkyl, haloalkyl, C₁-C₈ cycloalkyl, C₁-C₈ heterocyclyl, aryl, and heteroaryl, wherein the cycloalkyl, heterocyclyl, aryl, and heteroaryl are optionally substituted with one or more substituents selected from the group consisting of H, NH₂, OH, SH, alkyl, haloalkyl, cycloalkyl, alkoxy, hydroxyalkyl, alkenyl, alkynyl, nitro, cyano, and halogen;
R₇ is selected from the group consisting of C₁-C₆ alkyl, haloalkyl, alkenyl, alkynyl, and cyano;
m is an integer from 0 to 6; n is an integer from 0 to 6; p is 0, 1, or 2; q is 0, 1, or 2; t is 1 or 2;
in formula (III),
R' is selected from the group consisting of H, 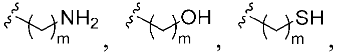 alkoxy, alkenyl, alkynyl, nitro, cyano, halogen, acetyl, sulfonyl, 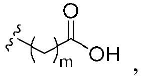 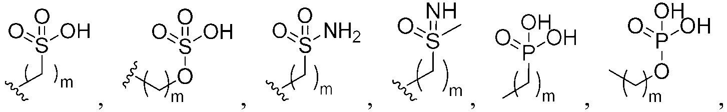 C₁-C₆ alkyl, haloalkyl, C₁-C₈ cycloalkyl, C₁-C₈ heterocyclyl, aryl, heteroaryl and 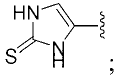
R" is selected from the group consisting of NH₂ and groups selected from the substituted or unsubstituted following: 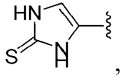 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, -[C(R₄)(R₅)]m-N(R‴)₃⁺, C₂-C₇ cyclic quaternary ammonium salts (e.g., 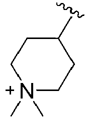 ), 5-12 membered heterospirocyclic quaternary ammonium salts containing N, O, or S (e.g., 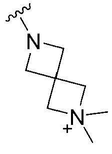 ), and 5-12 membered heterobridged quaternary ammonium salts containing N, O, or S (e.g., 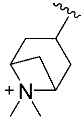 ), wherein substituents are selected from the group consisting of H, OH, SH, NH₂, C₁-C₆ alkyl, haloalkyl, alkenyl, alkynyl, nitro, cyano, and halogen; R₄ and R₅ are as previously defined;
m is an integer from 0 to 6;
or R' and R" together with the carbon atom to which they are attached form a substituted or unsubstituted group selected from the following: 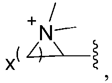 groups containing a 5-12 membered spirocyclic, 5-12 membered heterobridged cyclic, or 6-12 membered fused cyclic system containing N, O, or S, wherein substituents are selected from the group consisting of H, OH, SH, NH₂, C₁-C₆ alkyl, haloalkyl, alkenyl, alkynyl, nitro, cyano, and halogen; x is an integer from 1 to 6;
R‴ is selected from the group consisting of C₁-C₆ alkyl, haloalkyl, alkenyl, alkynyl, and cyano;
in formula (IV),
L₂ is selected from the group consisting of -[C(R_{b})(R_{c})]ₓ-, -[C(R_{b})(R_{c})]ₓ-Z-[C(R_{d})(Rₑ)]ₕ-, -[C(R_{b})(R_{c})]ₓ-A-C(O)-[C(R_{d})(Rₑ)]ₕ-, -[C(R_{b})(R_{c})]ₓ-A-C(S)-[C(R_{d})(Rₑ)]ₕ-, -[C(R_{b})(R_{c})]ₓ-A-S(O)₂-[C(R_{d})(Rₑ)]ₕ-, -[C(R_{b})(R_{c})]ₓ-C(O)-A-[C(R_{d})(Rₑ)]ₕ - -[C(R_{b})(R_{c})]ₓ-C(S)-A-[C(R_{d})(Rₑ)]ₕ-, -[C(R_{b})(R_{c})]ₓ-S(O)₂-A-[C(R_{d})(Rₑ)]ₕ-, -[C(R_{b})(R_{c})]ₓ-ethenyl-[C(R_{d})(Rₑ)]ₕ-, -[C(R_{b})(R_{c})]ₓ-ethynyl-[C(R_{d})(Rₑ)]ₕ-, -[C(R_{b})(R_{c})]ₓ-S(O)(NRₖ)- [C(R_{d})(Rₑ)]ₕ-, -[C(R_{b})(R_{c})]ₓ-S(O)=N-[C(R_{d})(Rₑ)]ₕ-, and -[C(R_{b})(R_{c})]ₓ -S(O)ₜ -[C(R_{d})(Rₑ)]ₕ-;
Rₐ is selected from the group consisting of -[C(R_{f})(R_{g})]ₙ-Rⱼ, 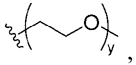 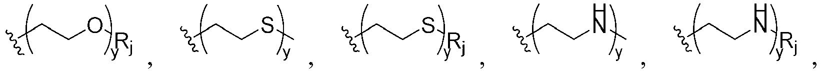 -Y-[C(R_{f})(R_{g})]ₙ-Rⱼ, -[C(R_{f})(R_{g})]ₙ-N(Rₚ)₂⁺- [C(Rₕ)(Rᵢ)]ᵢ-Rⱼ, -[C(R_{f})(R_{g})]ₙ-A-C(O)-[C(Rₕ)(Rᵢ)]ᵢ-Rⱼ, -[C(R_{f})(R_{g})]ₙ-A-C(S)-[C(Rₕ)(Rᵢ)]ᵢ-Rⱼ, -[C(R_{f})(R_{g})]ₙ-ethenyl-[C(Rₕ)(Rᵢ)]ᵢ-Rⱼ, -[C(R_{f})(R_{g})]ₙ-ethynyl-[C(Rₕ)(Rᵢ)]ᵢ-Rⱼ, -[C(R_{f})(R_{g})]ₙ-S(O)(NRₖ)-[C(Rₕ)(Rᵢ)]ᵢ-Rⱼ, -[C(R_{f})(R_{g})]ₙ-S(O)=N-[C(Rₕ)(Rᵢ)]ᵢ-Rⱼ, -[C(R_{f})(R_{g})]ₙ-S(O)ₜ-[C(Rₕ)(Rᵢ)]ᵢ-Rⱼ, -[C(R_{f})(R_{g})]ₙ-C(O)-A-[C(Rₕ)(Rᵢ)]ᵢ -Rⱼ, -[C(R_{f})(R_{g})]ₙ-C(S)-A-[C(Rₕ)(Rᵢ)]ᵢ -Rⱼ, -[C(R_{f})(R_{g})]ₙ-ethenyl-[C(Rₕ)(Rᵢ)]ᵢ -Rⱼ, -[C(R_{f})(R_{g})]ₙ-ethynyl-[C(Rₕ)(Rᵢ)]ᵢ-Rⱼ, -[C(R_{f})(R_{g})]ₙ-S(O)(NRₖ)- [C(Rₕ)(Rᵢ)]ᵢ-Rⱼ, [C(R_{f})(R_{g})]ₙ-N=S(O)-[C(Rₕ)(Rᵢ)]ᵢ -Rⱼ, -[C(R_{f})(R_{g})]ₙ -S(O)ₜ -[C(Rₕ)(Rᵢ)]ᵢ -Rⱼ, and -[C(R_{f})(R_{g})]ₙ -N(Rₚ)₂⁺-[C(Rₕ)(Rᵢ)]ᵢ -Rⱼ;
R_{b}, R_{c}, R_{d}, Rₑ, R_{f}, R_{g}, Rₕ and Rᵢ are each independently selected from the group consisting of hydrogen, alkyl, haloalkyl, alkoxy, hydroxy, cycloalkyl, heterocyclyl, aryl, heteroaryl, alkenyl, alkynyl, cyano, or halogen, wherein the cycloalkyl, heterocyclyl, aryl, and heteroaryl are optionally substituted with one or more substituents selected from the group consisting of alkyl, haloalkyl, cycloalkyl, alkoxy, hydroxyalkyl, alkenyl, alkynyl, nitro, cyano, halogen, acetyl, sulfonyl, 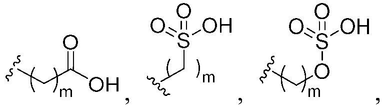 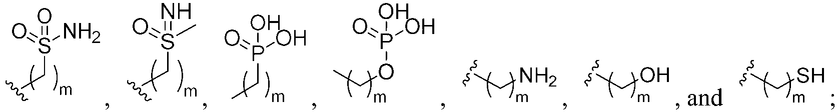
or R_{b} and R_{c}, R_{d} and Rₑ, R_{f} and R_{g}, Rₕ and Rᵢ together with the carbon atom(s) to which they are attached, respectively form a substituted or unsubstituted group selected from the following: cycloalkyl, heterocycloalkyl, 5-12 membered spirocyclic groups containing N, O, or S, 5-12 membered heterobridged cyclic groups containing N, O, or S, and 6-12 membered fused cyclic groups containing N, O, or S, wherein substituents are selected from the group consisting of H, OH, SH, NH₂, C₁-C₆ alkyl, haloalkyl, alkenyl, alkynyl, nitro, cyano, and halogen;
Rⱼ is selected from the group consisting of H, NH₂, OH, SH, alkoxy, hydroxyalkyl, haloalkyl, alkenyl, alkynyl, nitro, cyano, halogen, C₁-C₆ alkyl, C₁-C₈ cycloalkyl, C₁-C₈ heterocyclyl, aryl, heteroaryl, 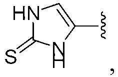 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, 6-12 membered fused heterocyclic groups containing N, O, or S, -N(Rₚ)₃⁺, C₂-C₇ cyclic quaternary ammonium salts (e.g., 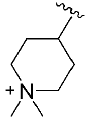 ), 5-12 membered heterospirocyclic quaternary ammonium salts containing N, O, or S (e.g., 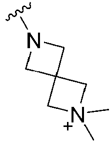 ), and 5-12 membered heterobridged quaternary ammonium salts containing N, O, or S (e.g., 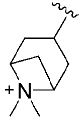 ), 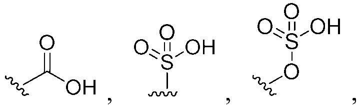 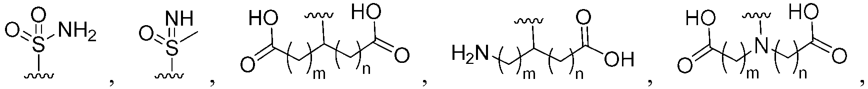 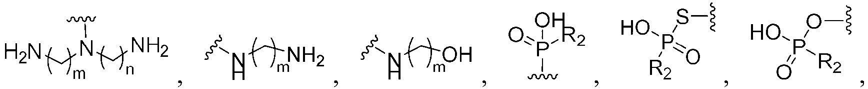 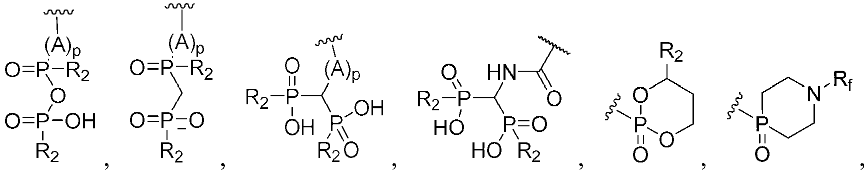 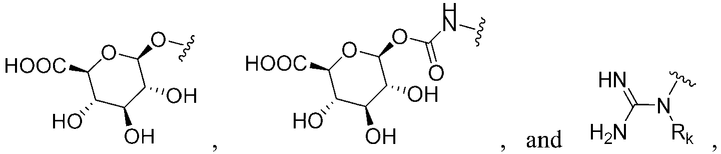 wherein the cycloalkyl, heterocyclyl, aryl, and heteroaryl are optionally substituted with one or more substituents selected from the group consisting of alkyl, cycloalkyl, alkoxy, hydroxyalkyl, alkenyl, alkynyl, nitro, cyano, hydroxy, amino, halogen, acetyl, sulfonyl, 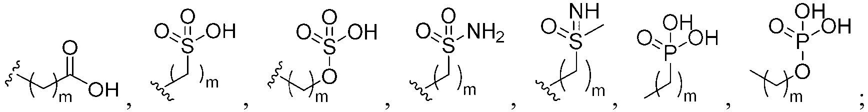 R₂ is as previously defined;
Rₖ is selected from the group consisting of hydrogen, C₁-C₆ alkyl, haloalkyl, C₁-C₈ cycloalkyl, C₁-C₈ heterocyclyl, aryl, and heteroaryl, wherein the C₁-C₈ cycloalkyl, C₁-C₈ heterocyclyl, aryl, and heteroaryl are optionally substituted with one or more substituents selected from the group consisting of alkyl, haloalkyl, cycloalkyl, alkoxy, hydroxyalkyl, alkenyl, alkynyl, nitro, cyano, hydroxy, amino, SH, and halogen;
Rₚ is selected from the group consisting of C₁-C₆ alkyl, haloalkyl, alkenyl, alkynyl, and cyano;
A is selected from O, S, or N(Rₖ);
Y is selected from substituted or unsubstituted C₁-C₈ cycloalkyl, C₁-C₈ heterocyclyl, aryl, heteroaryl, 5-12 membered spirocyclic groups, 5-12 membered heterospirocyclic groups containing N, O, or S, 5-12 membered bridged cyclic groups, 5-12 membered heterobridged cyclic groups containing N, O, or S, 6-12 membered fused cyclic groups, and 6-12 membered fused heterocyclic groups containing N, O, or S, wherein substituents are selected from H, NH₂, OH, SH, C₁-C₆ alkyl, haloalkyl, alkenyl, alkynyl, nitro, cyano, and halogen;
Z is selected from cycloalkyl, heterocyclyl, aryl, and heteroaryl, wherein the cycloalkyl, heterocyclyl, aryl, and heteroaryl are optionally substituted with one substituent selected from H, NH₂, OH, SH, alkyl, haloalkyl, cycloalkyl, alkoxy, hydroxyalkyl, alkenyl, alkynyl, nitro, cyano, and halogen;
h is an integer from 0 to 10; i is an integer from 0 to 10; m is an integer from 0 to 6; n is an integer from 0 to 6; x is an integer from 1 to 6; y is an integer from 0 to 10.

2. The compound, isomer, or pharmaceutically acceptable salt, acid, or deuterated derivative thereof according to claim 1, wherein in formula (II): L₁ is selected from - [C(R₄)(R₅)]ₘ-(A)_{q}- or -[C(R₄)(R₅)]ₘ-(A)ₚ-C(O)-[C(R₄)(R₅)]ₙ-(A)_{q}-; A is selected from O or S;
R₁ is selected from 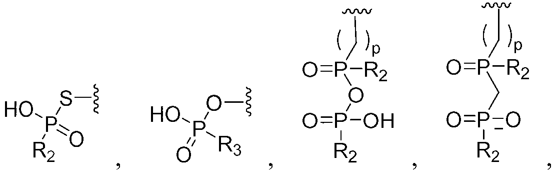 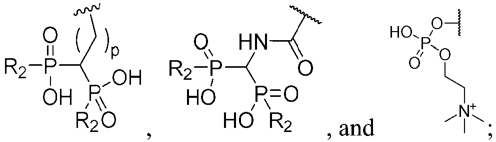
R₂ is selected from hydrogen, hydroxy, SH, alkyl, C₁-C₆ alkyl, and C₁-C₆ alkoxy;
R₃ is selected from hydrogen, SH, alkyl, C₁-C₆ alkyl, and C₁-C₆ alkoxy;
m is an integer from 0 to 6; n is an integer from 0 to 6; p is 0, 1, or 2; q is 0, 1, or 2; t is 1 or 2.

3. The compound, isomer, or pharmaceutically acceptable salt, acid, or deuterated derivative thereof according to claim 1, wherein in formula (II): R₁ is selected from 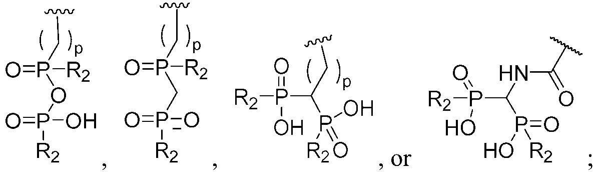 R₂ is selected from hydrogen, hydroxy, SH, alkyl, C₁-C₆ alkyl, and C₁-C₆ alkoxy; p is 0, 1, or 2.

4. The compound, isomer, or pharmaceutically acceptable salt, acid, or deuterated derivative thereof according to claim 1, wherein in formula (III):
R' is selected from H, C₁-C₆ alkyl, 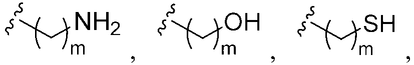 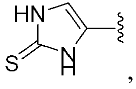 C₁-C₆ alkoxy, nitro, cyano, or halogen;
R" is selected from groups substituted or unsubstituted as follows: 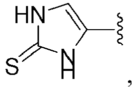 5-12 membered heterospirocyclic groups containing N, O, or S, -[C(R₄)(R₅)]ₘ-N(R‴)₃⁺; the substituents are selected from H, OH, SH, NH₂, C₁-C₄ alkyl, C₁-C₄ haloalkyl, nitro, cyano, or halogen; m is 0, 1, 2, 3, 4, 5, or 6; R₄ and R₅ are independently selected from hydrogen, hydroxy, SH, alkyl, or C₁-C₄ alkyl;
alternatively, R' and R" together with the carbon atom to which they are attached form a substituted or unsubstituted group selected from 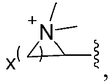 5-12 membered spirocyclic groups having N, O, or S, 5-12 membered heterobridged cyclic groups containing N, O, or S, or 6-12 membered fused cyclic groups containing N, O, or S; the substituents are selected from H, OH, SH, NH₂, C₁-C₄ alkyl, C₁-C₄ haloalkyl, nitro, cyano, or halogen; x is 1, 2, 3, 4, 5, or 6;
R‴ is selected from C₁-C₆ alkyl, C₁-C₆ haloalkyl, alkenyl, alkynyl, or cyano;
preferably,
R'is selected from H, C₁-C₆ alkyl, 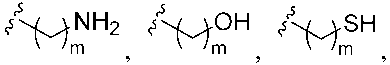 C₁-C₄ alkoxy, nitro, cyano, or halogen;
R" is selected from NH₂ and groups substituted or unsubstituted as follows: - [C(R₄)(R₅)]ₘ-N(R‴)₃⁺; the substituents are selected from H, OH, SH, NH₂, C₁-C₄ alkyl, C₁-C₄ haloalkyl, nitro, cyano, or halogen; m is 0, 1, 2, 3, 4, 5, or 6; R₄ and R₅ are independently selected from hydrogen, hydroxy, SH, alkyl, or C₁-C₄ alkyl;
R‴ is selected from C₁-C₄ alkyl, C₁-C₄ haloalkyl, alkenyl, alkynyl, or cyano; preferably methyl, ethyl, propyl, or isopropyl.

5. The compound, isomer, or pharmaceutically acceptable salt, acid, or deuterated derivative thereof according to claim 1, wherein in formula (IV), L₂ is selected from-[C(R_{b})(R_{c})]ₓ-, -[C(R_{b})(R_{c})]ₓ-Z-, -[C(R_{b})(R_{c})]ₓ-O-C(O)-[C(R_{d})(Rₑ)]ₕ-, -[C(R_{b})(R_{c})]ₓ-S-C(O)-[C(R_{d})(Rₑ)]ₕ -, -[C(R_{b})(R_{c})] ₓ - O -C(O)-[C(R_{d})(Rₑ)]ₕ -, -[C(R_{b})(R_{c})] ₓ - O -C(S)-[C(R_{d})(Rₑ)]ₕ-; R_{b} is H or methyl; R_{c} is H or methyl; Z is a C₅-C₁₀ aryl or a 5-6 membered heteroaryl, preferably phenyl, naphthyl, or thiophenyl; R_{d} is methyl or ethyl; Rₑ is H, methyl, or ethyl; x is 1, 2, 3, 4, 5, or 6; h is an integer from 0 to 10;
preferably, L₂ is selected from -CH₂-, -CH(CH₃)- or -C(CH₃)₂-; more preferably, L₂ is -CH₂-;
preferably, L₂ is selected from -CH₂-O-C(O)-(CH₂)ₕ-, -CH₂-S-C(O)-(CH₂)ₕ-, - CH₂-O-C(O)-[C(R_{d})(Rₑ)] ₕ-, -CH₂-O-C(S)-[C(R_{d})(Rₑ)] ₕ-; R_{d} is methyl; Rₑ is H or methyl; h is 0 or an integer up to 10;
more preferably, h is an integer from 0 to 6;
most preferably, h is 0, 1, 2, 3, or 4.

6. The compound, isomer, or pharmaceutically acceptable salt, acid, or deuterated derivative thereof according to claim 1, wherein in formula (IV), Rₐ is selected from substituted or unsubstituted groups as follows: C₁-C₁₀ alkyl, 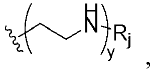 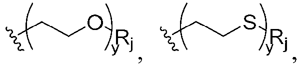 -(CH₂)ₕCHRₓR_{y}, -(CH₂)ₕNRₘRₙ, -(CH₂)ₕN(Rₚ)2⁺-Rⱼ; the substituents are selected from NH₂, -OH, -COOH, 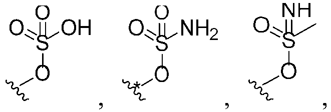 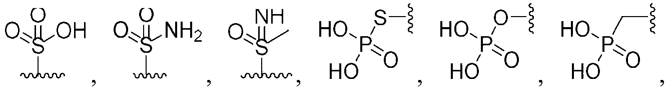 C₁-C₄ alkyl, C₁-C₄ alkoxy, or C₁-C₄ carboxyalkyl;
h or y is an integer from 0 to 10;
Rₓ is selected from H, -COOH, or C₁-C₄ alkyl;
R_{y} is selected from H, amino, or C₁-C₄ alkyl;
Rₘ and Rₙ are independently selected from H, amino, -COOH, or C₁-C₄ alkyl; or Rₘ and Rₙ together with the nitrogen atom to which they are attached form a 5-11 membered heterocyclic group containing 1-3 heteroatoms selected from N, O, or S;
Rⱼ is selected from H or C₁-C₆ alkyl;
Rₚ is selected from H or C₁-C₆ alkyl;
preferably, in formula (IV), Rₐ is selected from substituted or unsubstituted groups as follows: C₁-C₆ alkyl, 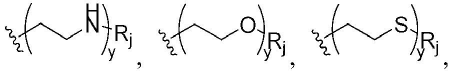 -(CH₂)ₕCHRₓR_{y}, -(CH₂) ₕNRₘRₙ, -(CH₂)ₕN(Rₚ)₂⁺-Rⱼ; the substituents are selected from -NH₂, -OH, - COOH, 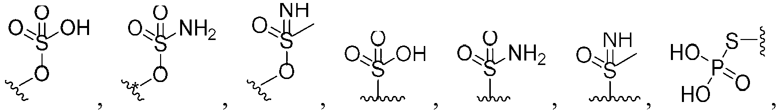 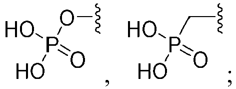
y is 1, 2, 3, 4, 5, or 6;
h is 0, 1, 2, 3, or 4;
Rₓ is selected from H, -COOH, methyl, ethyl, propyl, or isopropyl;
R_{y} is selected from H, amino, methyl, ethyl, propyl, or isopropyl;
Rₘ and Rₙ are independently selected from H, amino, -COOH, methyl, ethyl, propyl, or isopropyl; or Rm and Rn together with the nitrogen atom to which they are attached form a 5-11 membered heterocyclic group containing 1-3 heteroatoms selected from N, O, or S;
Rⱼ is selected from H, methyl, ethyl, propyl, or isopropyl;
Rₚ is selected from H, methyl, ethyl, propyl, or isopropyl.

7. A compound, isomer, or pharmaceutically acceptable salt, acid, or deuterated derivative thereof having the following structure:

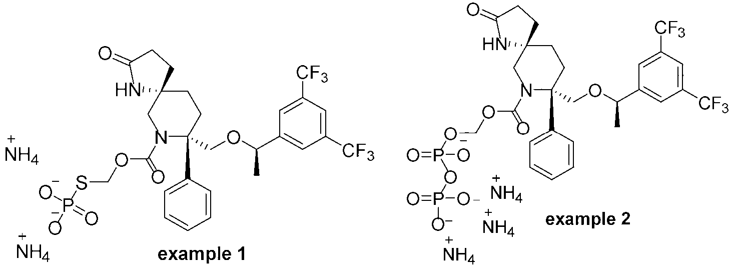

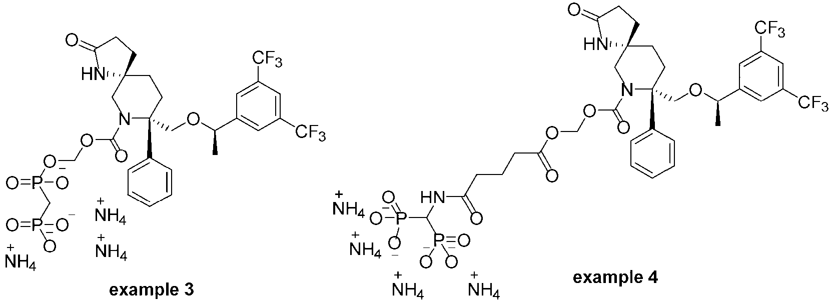

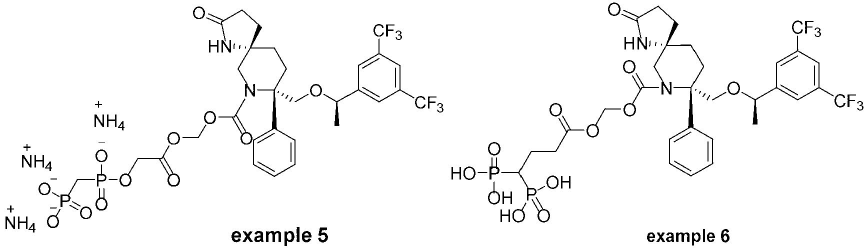

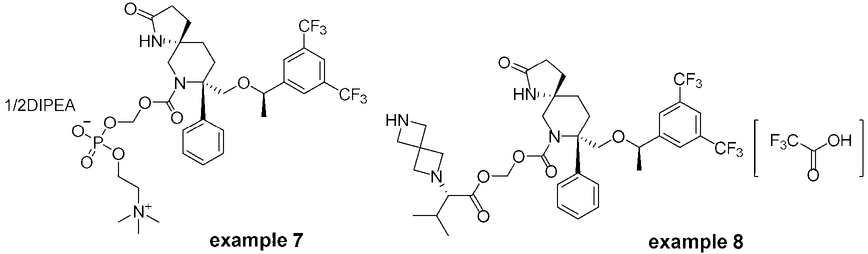

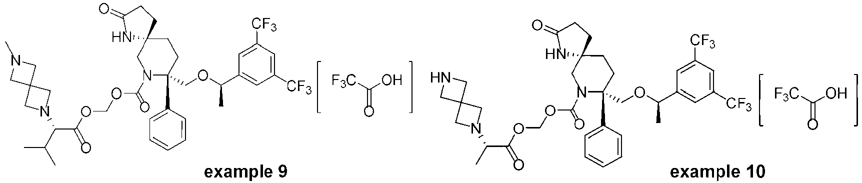

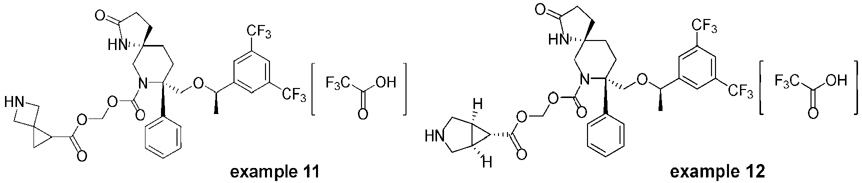

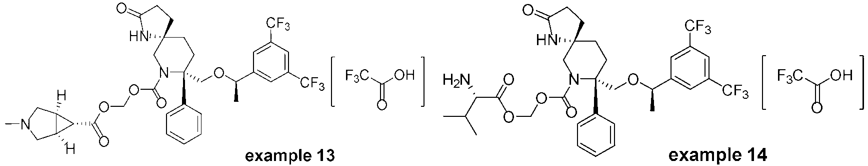

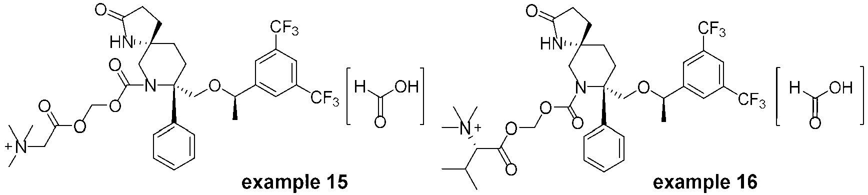

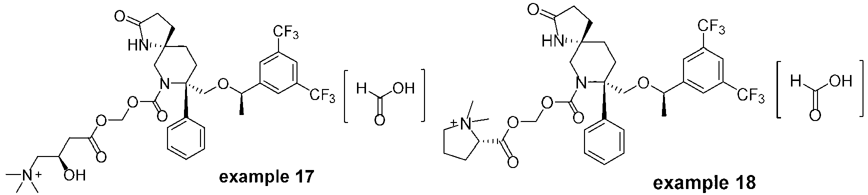

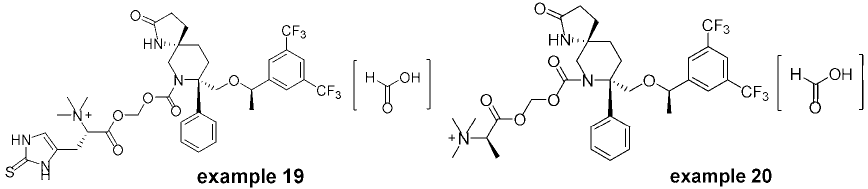

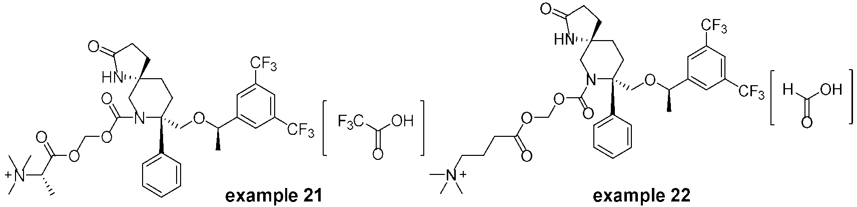

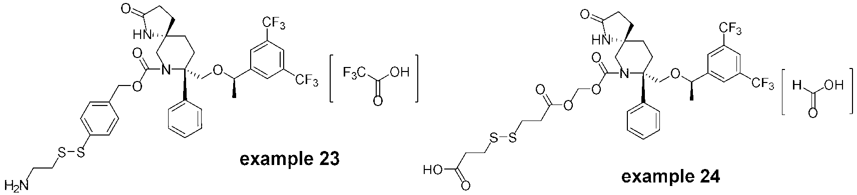

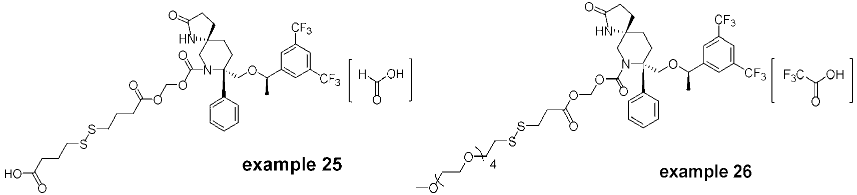

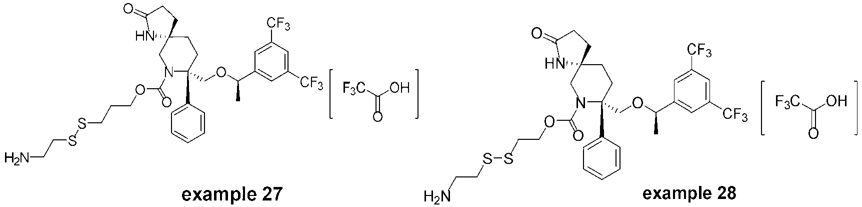

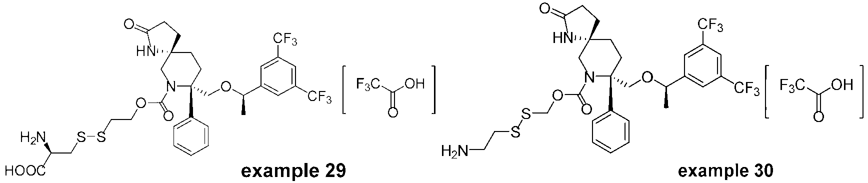

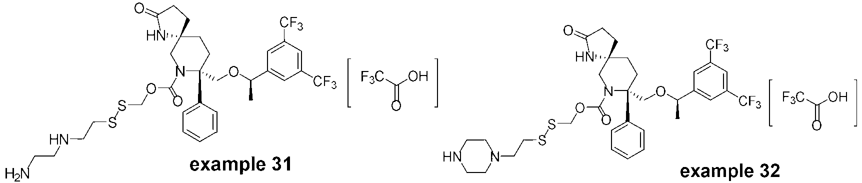

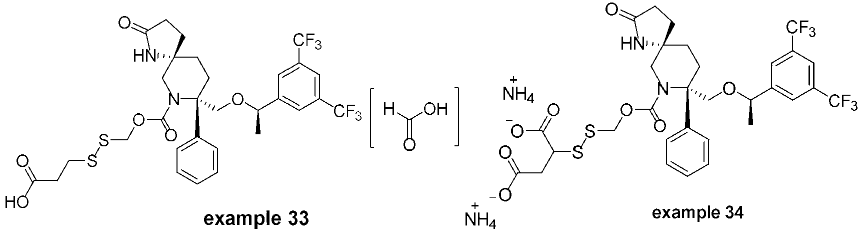

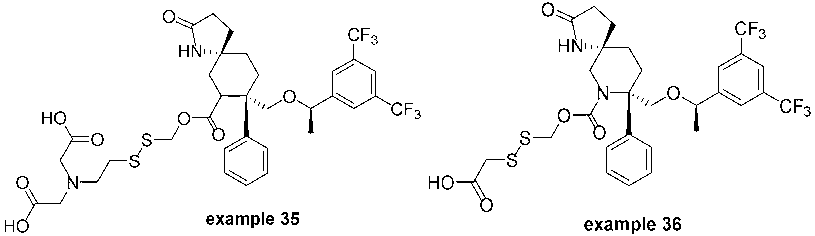

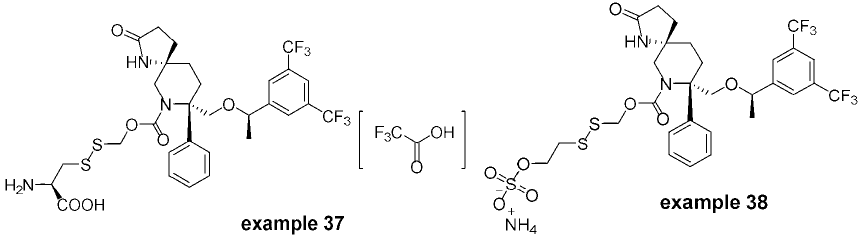

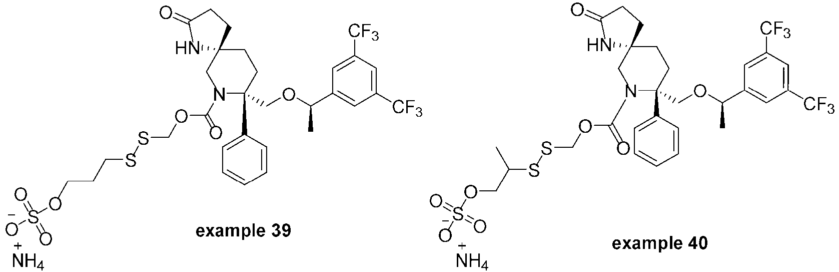

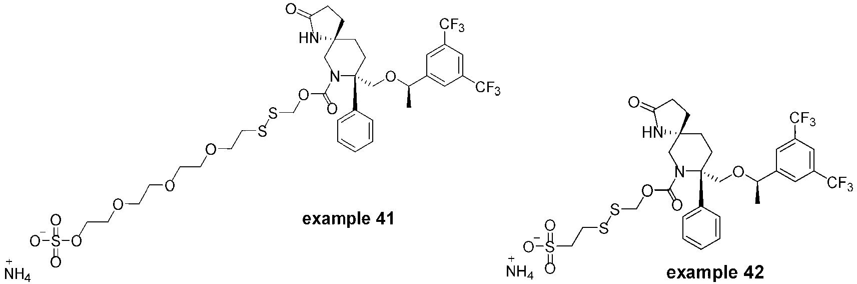

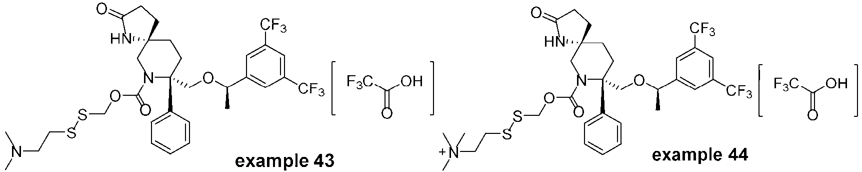

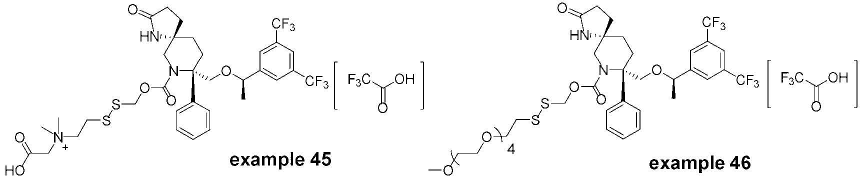

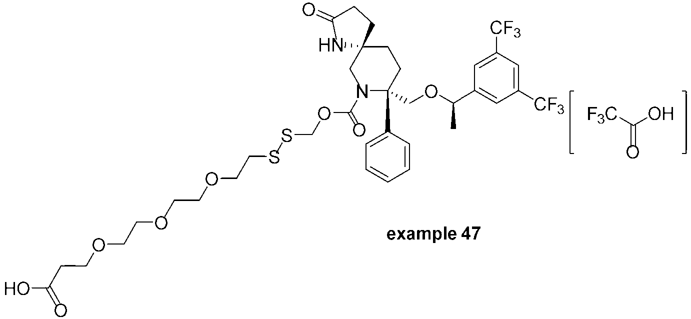

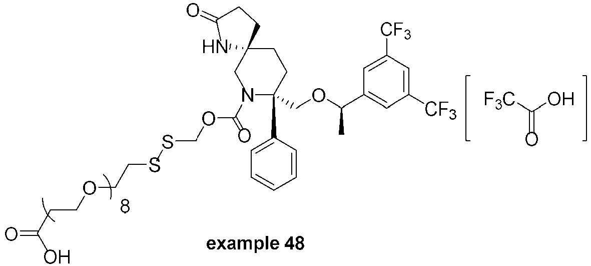

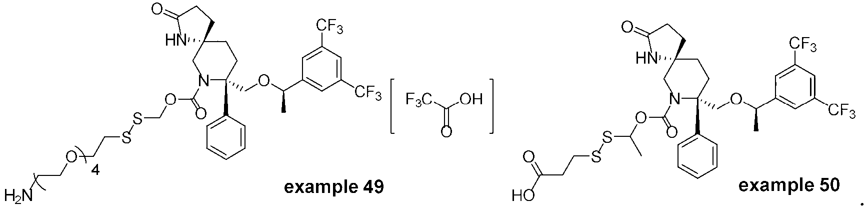

8. A composition comprising the compound, isomer, or pharmaceutically acceptable salt, acid, or deuterated derivative thereof according to any one of claims 1-7, and a pharmaceutically acceptable carrier.

9. Use of the compound, isomer, or pharmaceutically acceptable salt, acid, or deuterated derivative thereof according to any one of claims 1-7 in the preparation of a neurokinin-1 receptor antagonist.

10. Use of the compound, isomer, or pharmaceutically acceptable salt, acid, or deuterated derivative thereof according to any one of claims 1-7 in the preparation of a medicament for treating vomiting, nausea, asthma, anxiety, depression, cough, or migraine.
